(19) Europäisches Patentamt
European Patent Office
Office européen des brevets

(11) EP 3 581 570 B1

(12) **EUROPEAN PATENT SPECIFICATION**

(45) Date of publication and mention of the grant of the patent:
**21.04.2021 Bulletin 2021/16**

(21) Application number: **19178086.5**

(22) Date of filing: **04.06.2019**

(51) Int Cl.:
***C07D 401/14*** *(2006.01)* ***C09K 11/06*** *(2006.01)*
***H01L 51/00*** *(2006.01)* ***H01L 51/50*** *(2006.01)*
***H05B 33/10*** *(2006.01)*

(54) **CARBAZOLE-PYRIDINE-TRIFLUOROMETHYLPHENYL DERIVATIVES AND RELATED COMPOUNDS FOR USE AS TADF LUMINESCENT EMITTERS IN OLEDS**

CARBAZOLE-PYRIDINE-TRIFLUOROMETHYLPHENYL DERIVATE UND ÄHNLICHE VERBINDUNGEN ZUR VERWENDUNG ALS TADF LUMINESZENZEMITTER IN OLEDS

DÉRIVÉS CARBAZOLE-PYRIDINE-TRIFLUOROMÉTHYLPHÉNYL ET COMPOSÉS SIMILAIRES À ÊTRE UTILISÉS COMME ÉMETTEURS LUMINESCENTS TADF DANS D'OLEDS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.06.2018 DE 102018114468**

(43) Date of publication of application:
**18.12.2019 Bulletin 2019/51**

(73) Proprietor: **CYNORA GMBH**
**76646 Bruchsal (DE)**

(72) Inventor: **ARJONA ESTEBAN, Alhama**
**76131 Karlsruhe (DE)**

(74) Representative: **Hoppe, Georg Johannes**
**Darani Anwaltskanzlei**
**Beuckestrasse 20**
**14163 Berlin (DE)**

(56) References cited:
**EP-A1- 3 287 451 WO-A1-2017/005699**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 581 570 B1

## Description

The invention relates to organic molecules and their use in organic light-emitting diodes (OLEDs) and in other optoelectronic devices.

EP 3287451 A1 and WO 2017/005699 A1 both disclose organic molecules, in particular for use in organic optoelectronic devices.

## Description

An object of the present invention is to provide molecules which are suitable for use in optoelectronic devices.

This object is achieved by the invention which provides a new class of organic molecules.

The organic molecules of the invention are preferably purely organic molecules, i.e. they do not contain any metal ions, which is in contrast to metal complexes known for use in optoelectronic devices. Therefore, according to the present invention, it is preferred that the organic molecules are free of metal atoms or metal ions. The pure organic molecules may, however, include metalloids, in particular, B and Si or Sn, Se, and Ge.

According to the present invention, the organic molecules exhibit emission maxima in the blue, sky-blue or green spectral range. The organic molecules exhibit in particular emission maxima between 420 nm and 520 nm, preferably between 440 nm and 495 nm, more preferably between 450 nm and 470 nm. The photoluminescence quantum yields of the organic molecules according to the invention are, in particular, 20 % or more. The molecules according to the invention exhibit in particular thermally activated delayed fluorescence (TADF). The use of the molecules according to the invention in an optoelectronic device, for example an organic light-emitting diode (OLED), leads to higher efficiencies of the device. Corresponding OLEDs have a higher stability than OLEDs with known emitter materials and comparable color.

The organic light-emitting molecules of the invention comprise or consist of a first chemical moiety comprising or consisting of a structure of Formula I,

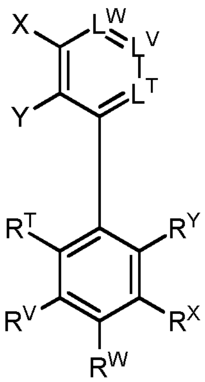

Formula I

and

- two second chemical moieties, each independently from another comprising or consisting of a structure of Formula II,

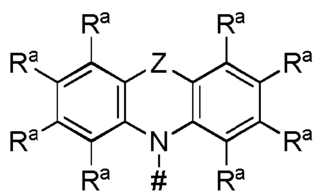

Formula II

wherein the first chemical moiety is linked to each of the two second chemical moieties via a single bond.

$L^T$ is N or C-$R^1$.
$L^V$ is N or C-$R^1$.

$L^W$ is N or C-W.
X is the binding site of a single bond linking the first chemical moiety to one of the two second chemical moieties or is $R^2$.
Y is the binding site of a single bond linking the first chemical moiety to one of the two second chemical moieties or is $R^2$.
W is the binding site of a single bond linking the first chemical moiety to one of the two second chemical moieties or is $R^2$.
$R^T$ is selected from the group consisting of $CF_3$ and $R^I$,
$R^V$ is selected from the group consisting of $CF_3$ and $R^I$,
$R^W$ is the binding site of a single bond linking the first chemical moiety to one of the two second chemical moieties or is selected from the group consisting of $CF_3$ and $R^I$.
$R^X$ is the binding site of a single bond linking the first chemical moiety to one of the two second chemical moieties or is $R^I$,
$R^Y$ is the binding site of a single bond linking the first chemical moiety to one of the two second chemical moieties or is $R^I$.
# represents the binding site of a single bond linking the second chemical moieties to the first chemical moiety;
Z is at each occurrence independently from another selected from the group consisting of a direct bond, $CR^3R^4$, $C=CR^3R^4$, C=O, $C=NR^3$, $NR^3$, O, $SiR^3R^4$, S, S(O) and $S(O)_2$;
$R^1$ is at each occurrence independently from another selected from the group consisting of hydrogen,
deuterium,
$C_1$-$C_5$-alkyl,
wherein one or more hydrogen atoms are optionally substituted by deuterium;
C2-C8-alkenyl,

wherein one or more hydrogen atoms are optionally substituted by deuterium; C2-C8-alkynyl,
wherein one or more hydrogen atoms are optionally substituted by deuterium; and $C_6$-$C_{18}$-aryl,
which is optionally substituted with one or more substituents $R^6$.

$R^2$ is at each occurrence independently from another selected from the group consisting of hydrogen,
deuterium,
$C_1$-$C_5$-alkyl,
wherein one or more hydrogen atoms are optionally substituted by deuterium;
C2-C8-alkenyl,

wherein one or more hydrogen atoms are optionally substituted by deuterium; C2-C8-alkynyl,
wherein one or more hydrogen atoms are optionally substituted by deuterium; and $C_6$-$C_{18}$-aryl,
which is optionally substituted with one or more substituents $R^6$.

$R^I$ is at each occurrence independently from another selected from the group consisting of hydrogen,
deuterium,
$C_1$-$C_5$-alkyl,
wherein one or more hydrogen atoms are optionally substituted by deuterium;
$C_2$-$C_8$-alkenyl,

wherein one or more hydrogen atoms are optionally substituted by deuterium; C2-C8-alkynyl,
wherein one or more hydrogen atoms are optionally substituted by deuterium; and $C_6$-$C_{18}$-aryl,
which is optionally substituted with one or more substituents $R^6$.

$R^a$, $R^3$ and $R^4$ is at each occurrence independently from another selected from the group consisting of hydrogen, deuterium, $N(R^5)_2$, $OR^5$, $Si(R^5)_3$, $B(OR^5)_2$, $OSO_2R^5$, $CF_3$, CN, F, Br, I, $C_1$-$C_{40}$-alkyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-alkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-thioalkoxy,

which is optionally substituted with one or more substituents $R^5$ and wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkenyl,

which is optionally substituted with one or more substituents $R^5$ and wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkynyl,

which is optionally substituted with one or more substituents $R^5$ and wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_6$-$C_{60}$-aryl,
which is optionally substituted with one or more substituents $R^5$; and
$C_3$-$C_{57}$-heteroaryl,
which is optionally substituted with one or more substituents $R^5$.
$R^5$ is at each occurrence independently from another selected from the group consisting of hydrogen, deuterium, $N(R^6)_2$, $OR^6$, $Si(R^6)_3$, $B(OR^6)_2$, $OSO_2R^6$, $CF_3$, CN, F, Br, I,
$C_1$-$C_{40}$-alkyl,

which is optionally substituted with one or more substituents $R^6$ and wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_1$-$C_{40}$-alkoxy,

which is optionally substituted with one or more substituents $R^6$ and wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_1$-$C_{40}$-thioalkoxy,

which is optionally substituted with one or more substituents $R^6$ and wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_2$-$C_{40}$-alkenyl,

which is optionally substituted with one or more substituents $R^6$ and wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_2$-$C_{40}$-alkynyl,

which is optionally substituted with one or more substituents $R^6$ and wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_6$-$C_{60}$-aryl,
which is optionally substituted with one or more substituents $R^6$; and
$C_3$-$C_{57}$-heteroaryl,
which is optionally substituted with one or more substituents $R^6$.

$R^6$ is at each occurrence independently from another selected from the group consisting of hydrogen, deuterium, OPh (Ph = phenyl), $CF_3$, CN, F,
$C_1$-$C_5$-alkyl,
wherein optionally one or more hydrogen atoms are independently from each other substituted by deuterium, CN, $CF_3$, or F;
$C_1$-$C_5$-alkoxy,
wherein optionally one or more hydrogen atoms are independently from each other substituted by deuterium, CN, $CF_3$, or F;
$C_1$-$C_5$-thioalkoxy,
wherein optionally one or more hydrogen atoms are independently from each other substituted by deuterium, CN, $CF_3$, or F;
C2-C5-alkenyl,
wherein optionally one or more hydrogen atoms are independently from each other substituted by deuterium, CN, $CF_3$, or F;
C2-C5-alkynyl,
wherein optionally one or more hydrogen atoms are independently from each other substituted by deuterium, CN, $CF_3$, or F;
$C_6$-$C_{18}$-aryl,

which is optionally substituted with one or more $C_1$-$C_5$-alkyl substituents; $C_3$-$C_{17}$-heteroaryl,
which is optionally substituted with one or more $C_1$-$C_5$-alkyl substituents; $N(C_6$-$C_{18}$-aryl$)_2$,

$N(C_3$-$C_{17}$-heteroaryl$)_2$; and
$N(C_3$-$C_{17}$-heteroaryl$)(C_6$-$C_{18}$-aryl$)$.

The substituents $R^a$, $R^3$, $R^4$ or $R^5$ independently from each other can optionally form a mono-or polycyclic, aliphatic, aromatic and/or benzo-fused ring system with one or more substituents $R^a$, $R^3$, $R^4$ or $R^5$.

According to the invention exactly one ring atom selected from the group consisting of $L^T$, $L^V$ and $L^W$ is N; exactly one substituent selected from the group consisting of $R^T$, $R^V$ and $R^W$ is $CF_3$; exactly one substituent selected from the group consisting of W, Y and X represents the binding site of a single bond linking the first chemical moiety and one of the two second chemical moieties and exactly one substituent selected from the group consisting of $R^W$, $R^Y$ and $R^X$ represents the binding site of a single bond linking the first chemical moiety and one of the two second chemical moieties.

In one embodiment of the invention, first chemical moiety comprises or consists of a structure of Formula Ia:

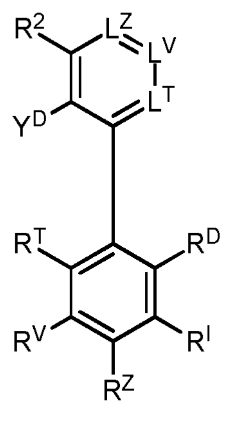

Formula Ia

wherein $R^1$, $R^2$, $R^T$, $R^V$, $R^I$, $L^T$ and $L^V$ are defined as above;
$Y^D$ is the binding site of a single bond linking the first chemical moiety to one of the two second chemical moieties;
$R^D$ is the binding site of a single bond linking the first chemical moiety to one of the two second chemical moieties;
$L^Z$ is N or C-$R^2$;
$R^Z$ is $CF_3$ or $R^I$;

According to this embodiment of the invention, exactly one substituent selected from the group consisting of $R^T$, $R^V$ and

$R^Z$ is $CF_3$ and exactly one ring atom selected from the group consisting of $L^T$, $L^V$ and $L^Z$ is N;

In one embodiment, $R^1$, $R^2$ and $R^I$ is at each occurrence independently from another selected from the group consisting of hydrogen (H), methyl, mesityl, tolyl and phenyl. The term tolyl comprises 2-tolyl, 3-tolyl and 4-tolyl.

In one embodiment, $R^1$, $R^2$ and $R^I$ is at each occurrence independently from another selected from the group consisting of hydrogen (H), methyl, and phenyl.

In one embodiment, $R^1$, $R^2$ and $R^I$ is hydrogen (H).

In one embodiment, $R^V$ is $CF_3$.

In one embodiment, $R^W$ is $CF_3$.

In one embodiment, $R^Z$ is $CF_3$.

In one embodiment, $L^V$ is N.

In one embodiment, $L^W$ is N.

In one embodiment, $L^Z$ is N.

In one embodiment, $R^V$ is $CF_3$ and $L^V$ is N.

In one embodiment, $R^V$ is $CF_3$ and $L^W$ is N.

In one embodiment, $R^V$ is $CF_3$ and $L^Z$ is N.

In one embodiment, $R^W$ is $CF_3$ and $L^W$ is N.

In one embodiment, $R^W$ is $CF_3$ and $L^Z$ is N.

In one embodiment, $R^W$ is $CF_3$ and $L^V$ is N.

In one embodiment, $R^Z$ is $CF_3$ and $L^W$ is N.

In one embodiment, $R^Z$ is $CF_3$ and $L^Z$ is N.

In one embodiment, $R^Z$ is $CF_3$ and $L^V$ is N.

In a further embodiment of the invention, the two second chemical moieties each at each occurrence independently from another comprise or consist of a structure of Formula IIa:

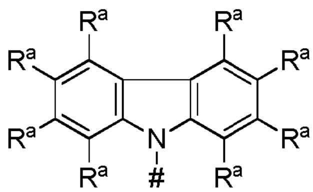

Formula IIa

wherein # and $R^a$ are defined as above.

In a further embodiment of the invention, $R^a$ is at each occurrence independently from another selected from the group consisting of H,

Me,

$^iPr$,

$^tBu$,

CN,

$CF_3$,

Ph, which is optionally substituted with one or more substituents independently from each other selected from the group consisting of Me, $^iPr$, $^tBu$, CN, $CF_3$, and Ph,

pyridinyl, which is optionally substituted with one or more substituents independently from each other selected from the group consisting of Me, $^iPr$, $^tBu$, CN, $CF_3$, and Ph,

pyrimidinyl, which is optionally substituted with one or more substituents independently from each other selected from the group consisting of Me, $^iPr$, $^tBu$, CN, $CF_3$, and Ph,

carbazolyl, which is optionally substituted with one or more substituents independently from each other selected from the group consisting of Me, $^iPr$, $^tBu$, CN, $CF_3$, and Ph,

triazinyl, which is optionally substituted with one or more substituents independently from each other selected from the group consisting of Me, $^iPr$, $^tBu$, CN, $CF_3$, and Ph,

and $N(Ph)_2$.

In a further embodiment of the invention, $R^a$ is at each occurrence independently from another selected from the group consisting of H,

Me,

$^iPr$,

$^tBu$,

CN,

$CF_3$,
Ph, which is optionally substituted with one or more substituents independently from each other selected from the group consisting of Me, $^{i}$Pr, $^{t}$Bu, CN, $CF_3$, and Ph,
pyridinyl, which is optionally substituted with one or more substituents independently from each other selected from the group consisting of Me, $^{i}$Pr, $^{t}$Bu, CN, $CF_3$, and Ph,
pyrimidinyl, which is optionally substituted with one or more substituents independently from each other selected from the group consisting of Me, $^{i}$Pr, $^{t}$Bu, CN, $CF_3$, and Ph, and
triazinyl, which is optionally substituted with one or more substituents independently from each other selected from the group consisting of Me, $^{i}$Pr, $^{t}$Bu, CN, $CF_3$, and Ph.

In a further embodiment of the invention, $R^a$ is at each occurrence independently from another selected from the group consisting of H,
Me,
$^{t}$Bu,
Ph, which is optionally substituted with one or more substituents independently from each other selected from the group consisting of Me, $^{i}$Pr, $^{t}$Bu, CN, $CF_3$, and Ph,
triazinyl, which is optionally substituted with one or more substituents independently from each other selected from the group consisting of Me, $^{i}$Pr, $^{t}$Bu, CN, $CF_3$, and Ph.

In a further embodiment of the invention, $R^a$ is H at each occurrence

In a further embodiment of the invention, the two second chemical moieties each at each occurrence independently from another comprise or consist of a structure of Formula IIb, a structure of Formula IIb-2, a structure of Formula IIb-3 or a structure of Formula IIb-4:

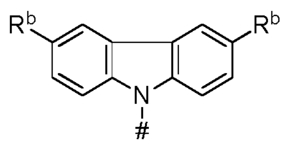
Formula IIb

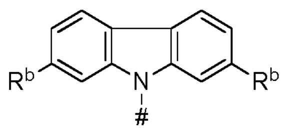
Formula IIb-2

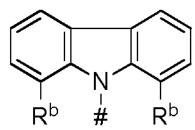
Formula IIb-3

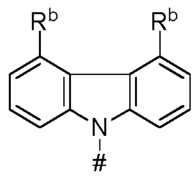
Formula IIb-4

wherein

$R^b$ is at each occurrence independently from another selected from the group consisting of deuterium,
$N(R^5)_2$,
$OR^5$,
$Si(R^5)_3$,
$B(OR^5)_2$,
$OSO_2R^5$,
$CF_3$,
CN,
F,
Br,
I,
$C_1$-$C_{40}$-alkyl,

which is optionally substituted with one or more substituents $R^5$ and
wherein one or more non-adjacent $CH_2$-groups are optionally substituted by $R^5C{=}CR^5$, C≡C, $Si(R^5)_2$, $Ge(R^5)_2$, $Sn(R^5)_2$, C=O, C=S, C=Se, $C{=}NR^5$, $P(=O)(R^5)$, SO, $SO_2$, $NR^5$, O, S or $CONR^5$;

$C_1$-$C_{40}$-alkoxy,

which is optionally substituted with one or more substituents $R^5$ and
wherein one or more non-adjacent $CH_2$-groups are optionally substituted by $R^5C{=}CR^5$, C≡C, $Si(R^5)_2$, $Ge(R^5)_2$, $Sn(R^5)_2$, C=O, C=S, C=Se, $C{=}NR^5$, $P(=O)(R^5)$, SO, $SO_2$, $NR^5$, O, S or $CONR^5$;

$C_1$-$C_{40}$-thioalkoxy,

which is optionally substituted with one or more substituents $R^5$ and wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkenyl,

which is optionally substituted with one or more substituents $R^5$ and wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkynyl,

which is optionally substituted with one or more substituents $R^5$ and wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_6$-$C_{60}$-aryl,
which is optionally substituted with one or more substituents $R^5$; and
$C_3$-$C_{57}$-heteroaryl,
which is optionally substituted with one or more substituents $R^5$;
apart from that the aforementioned definitions apply.

In one additional embodiment of the invention, the two second chemical moieties each at each occurrence independently from another comprise or consist of a structure of Formula IIc, a structure of Formula IIc-2, a structure of Formula IIc-3 or a structure of Formula IIc-4:

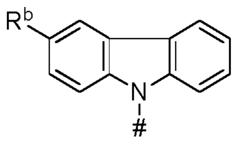
Formula IIc

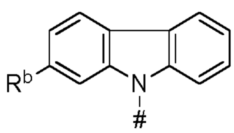
Formula IIc-2

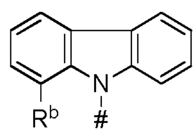
Formula IIc-3

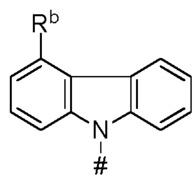
Formula IIc-4

wherein the aforementioned definitions apply.

In a further embodiment of the invention, $R^b$ is at each occurrence independently from another selected from the group consisting of
Me,
$^i$Pr,
$^t$Bu,
CN,
$CF_3$,
Ph, which is optionally substituted with one or more substituents independently from each other selected from the group consisting of Me, $^i$Pr, $^t$Bu, CN, $CF_3$, and Ph,
pyridinyl, which is optionally substituted with one or more substituents independently from each other selected from the group consisting of Me, $^i$Pr, $^t$Bu, CN, $CF_3$, and Ph,
carbazolyl, which is optionally substituted with one or more substituents independently from each other selected from the group consisting of Me, $^i$Pr, $^t$Bu, CN, $CF_3$, and Ph,
triazinyl, which is optionally substituted with one or more substituents independently from each other selected from the group consisting of Me, $^i$Pr, $^t$Bu, CN, $CF_3$, and Ph,
and $N(Ph)_2$.

In a further embodiment of the invention, $R^b$ is at each occurrence independently from another selected from the group consisting of
Me,
$^i$Pr,
$^t$Bu,
CN,

$CF_3$,
Ph, which is optionally substituted with one or more substituents independently from each other selected from the group consisting of Me, $^{i}Pr$, $^{t}Bu$, CN, $CF_3$, and Ph,
pyridinyl, which is optionally substituted with one or more substituents independently from each other selected from the group consisting of Me, $^{i}Pr$, $^{t}Bu$, CN, $CF_3$, and Ph,
pyrimidinyl, which is optionally substituted with one or more substituents independently from each other selected from the group consisting of Me, $^{i}Pr$, $^{t}Bu$, CN, $CF_3$, and Ph, and
triazinyl, which is optionally substituted with one or more substituents independently from each other selected from the group consisting of Me, $^{i}Pr$, $^{t}Bu$, CN, $CF_3$, and Ph.

In a further embodiment of the invention, $R^b$ is at each occurrence independently from another selected from the group consisting of
Me,
$^{t}Bu$,
Ph, which is optionally substituted with one or more substituents independently from each other selected from the group consisting of Me, $^{i}Pr$, $^{t}Bu$, CN, $CF_3$, and Ph,
triazinyl, which is optionally substituted with one or more substituents independently from each other selected from the group consisting of Me, $^{i}Pr$, $^{t}Bu$, CN, $CF_3$, and Ph.

In the following, exemplary embodiments of the second chemical moiety are shown:

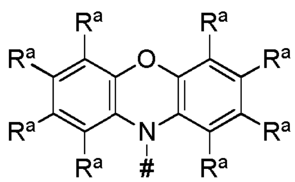
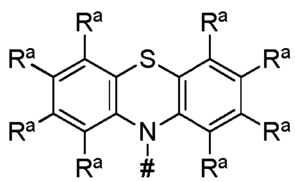
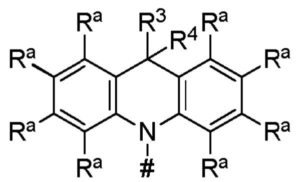
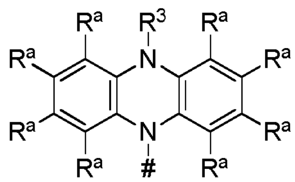
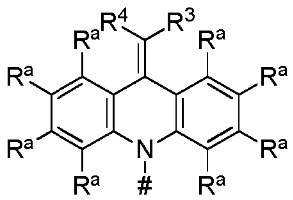
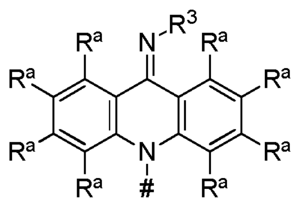
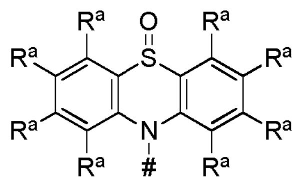
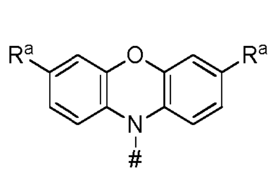
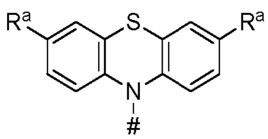
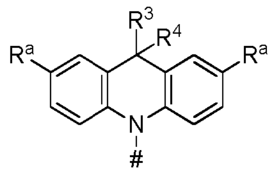
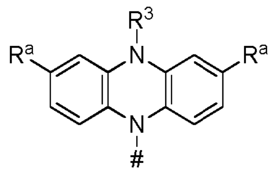
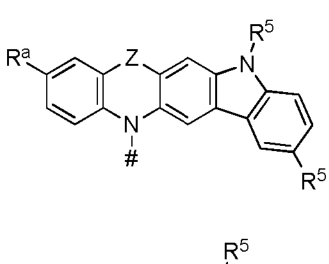
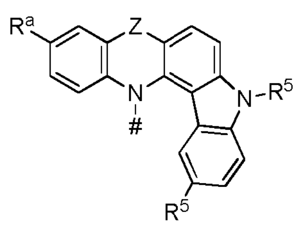
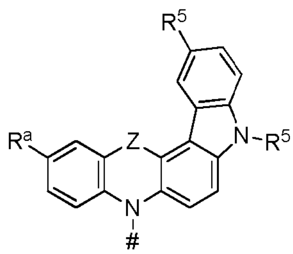
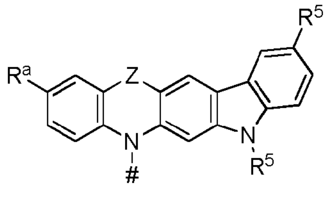
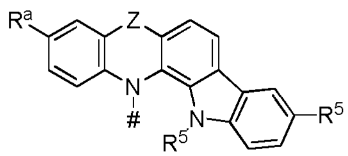
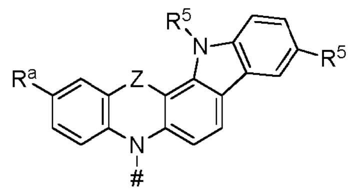

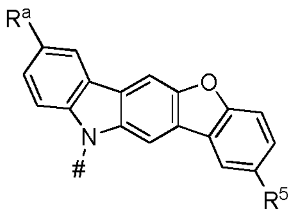
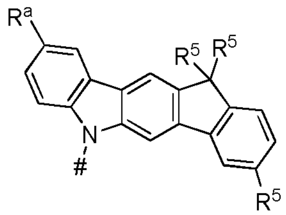
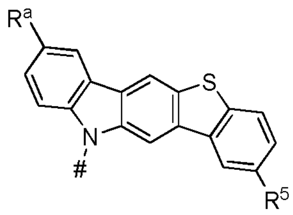

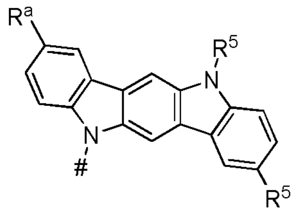

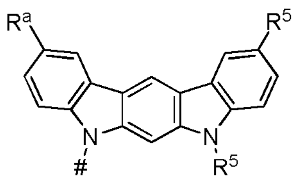
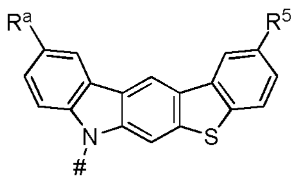
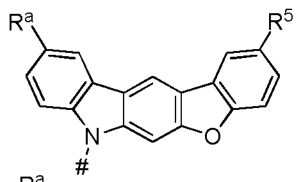

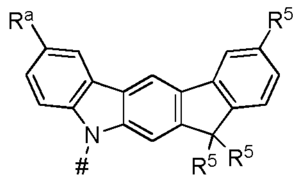
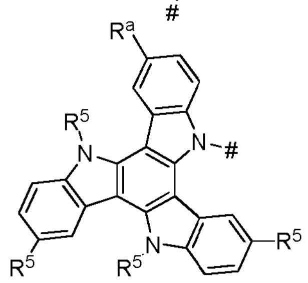

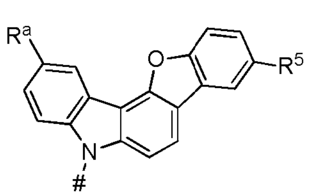
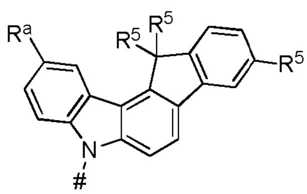
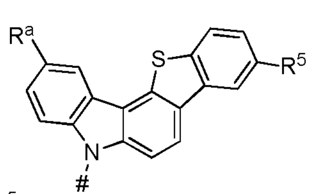

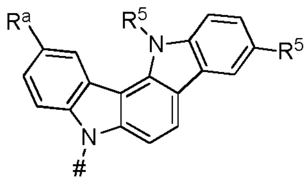
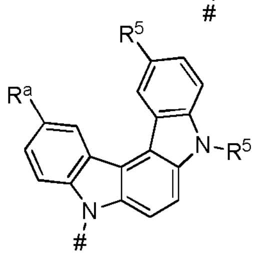

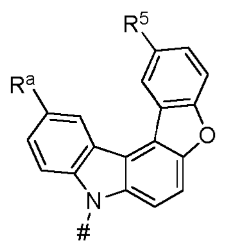
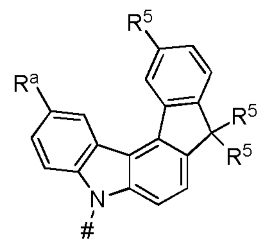
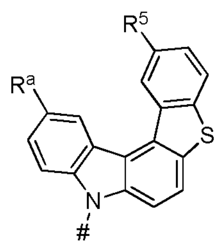

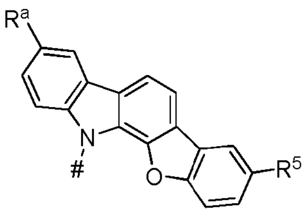
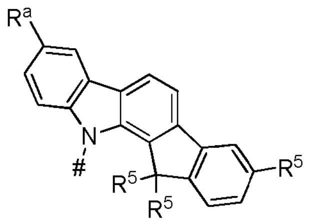
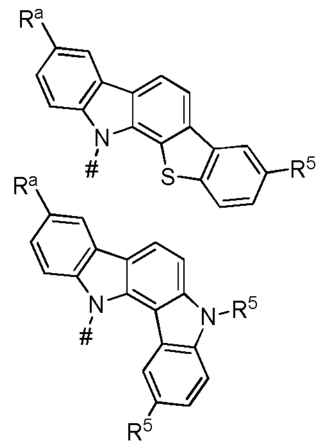
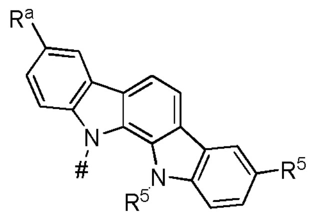

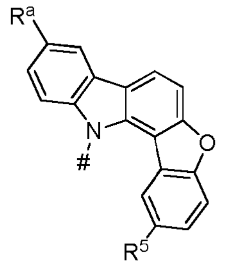
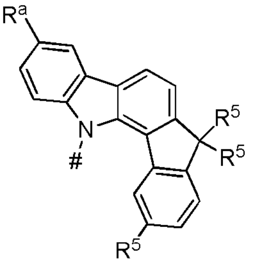
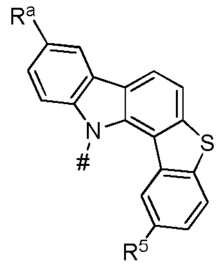

wherein for #, Z, $R^a$, $R^3$, $R^4$ and $R^5$ the aforementioned definitions apply.

In one embodiment, $R^a$ and $R^5$ is at each occurrence independently from another selected from the group consisting of hydrogen (H), methyl (Me), i-propyl ($CH(CH_3)_2$) ($^iPr$), t-butyl ($^tBu$), phenyl (Ph), CN, $CF_3$, and diphenylamine ($NPh_2$).

In one embodiment of the invention, the organic molecules comprise or consist of a structure of Formula III:

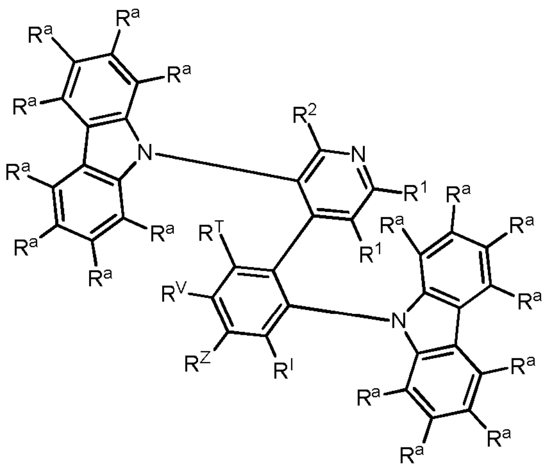

Formula III

wherein the aforementioned definitions apply,
and wherein exactly one substituent selected from the group consisting of $R^V$, $R^T$ and $R^Z$ is $CF_3$.

In a preferred embodiment of the invention, the organic molecules comprise or consist of a structure of Formula III and $R^V$ is $CF_3$.

In a further embodiment of the invention, the organic molecules comprise or consist of a structure selected from the group of Formula III-1 and Formula III-2:

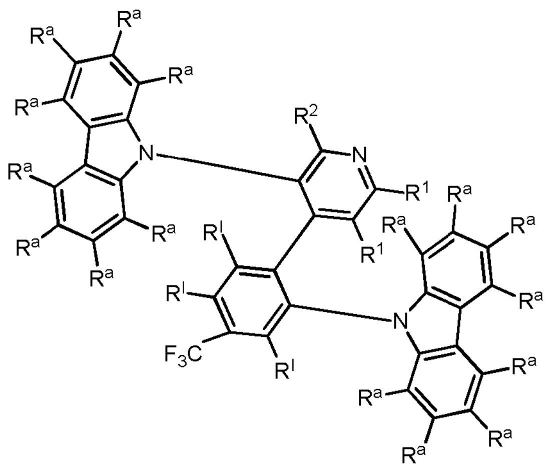

Formula III-1

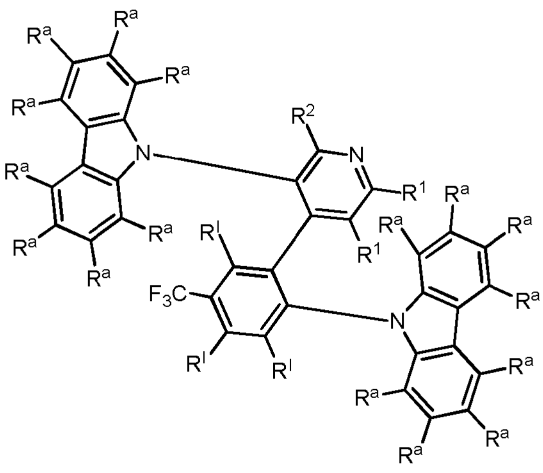

Formula III-2

wherein the aforementioned definitions apply.

In a further embodiment of the invention, the organic molecules comprise or consist of a structure selected from the group of Formula IIIa-1 and Formula IIIa-2:

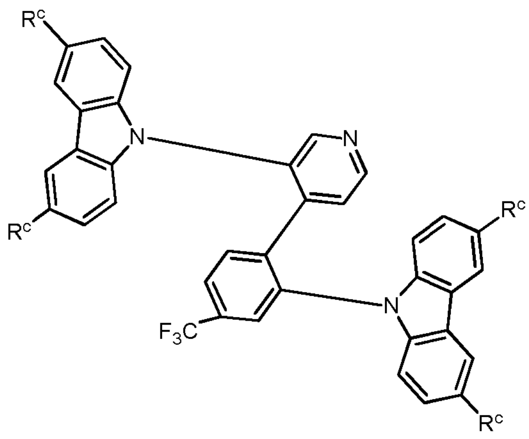

Formula IIIa-1

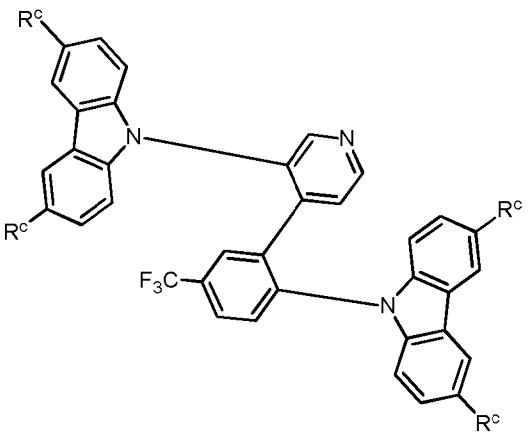

Formula IIIa-2

wherein

$R^c$ is at each occurrence independently from another selected from the group consisting of Me,
$^i$Pr,
$^t$Bu,
Ph, which is optionally substituted with one or more substituents independently from each other selected from the group consisting of Me, $^i$Pr, $^t$Bu, CN, $CF_3$, and Ph,
pyridinyl, which is optionally substituted with one or more substituents independently from each other selected from the group consisting of Me, $^i$Pr, $^t$Bu, CN, $CF_3$, and Ph,
pyrimidinyl, which is optionally substituted with one or more substituents independently from each other selected from the group consisting of Me, $^i$Pr, $^t$Bu, CN, $CF_3$, and Ph,
carbazolyl, which is optionally substituted with one or more substituents independently from each other selected from the group consisting of Me, $^i$Pr, $^t$Bu, CN, $CF_3$, and Ph,
triazinyl, which is optionally substituted with one or more substituents independently from each other selected from the group consisting of Me, $^i$Pr, $^t$Bu, CN, $CF_3$, and Ph,
and $N(Ph)_2$.

In a preferred embodiment of the invention, the organic molecules comprise or consist of a structure of Formula IIIa-1.

In a further embodiment of the invention, the organic molecules comprise or consist of a structure selected from the group of Formula IIIb-1 and Formula IIIb-2:

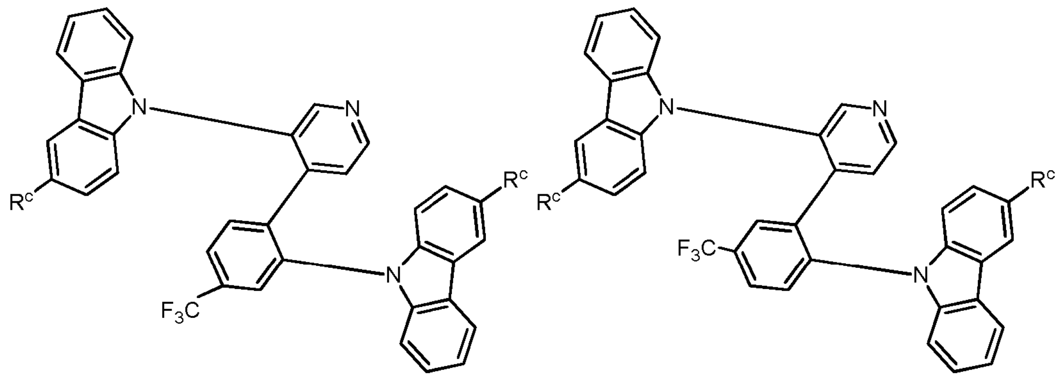

Formula IIIb-1 Formula IIIb-2

wherein the aforementioned definitions apply.

In a preferred embodiment of the invention, the organic molecules comprise or consist of a structure of Formula IIIb-1.

In one embodiment of the invention, the organic molecules comprise or consist of a structure of Formula IV:

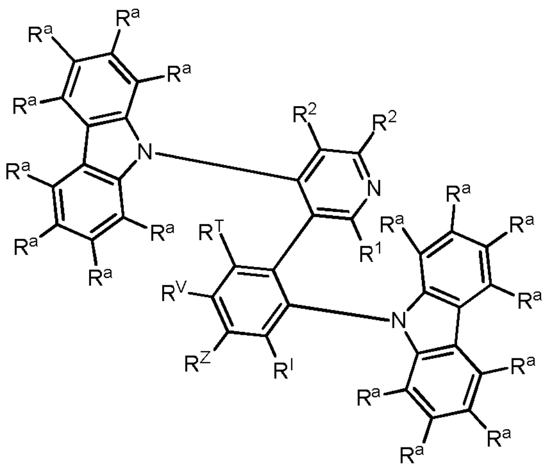

Formula IV

wherein the aforementioned definitions apply and wherein exactly one substituent selected from the group consisting of $R^T$, $R^V$ and $R^Z$ is $CF_3$.

In a preferred embodiment of the invention, the organic molecules comprise or consist of a structure of Formula IV and $R^V$ is $CF_3$.

In a further embodiment of the invention, the organic molecules comprise or consist of a structure selected from the group of Formula IV-1 and Formula IV-2:

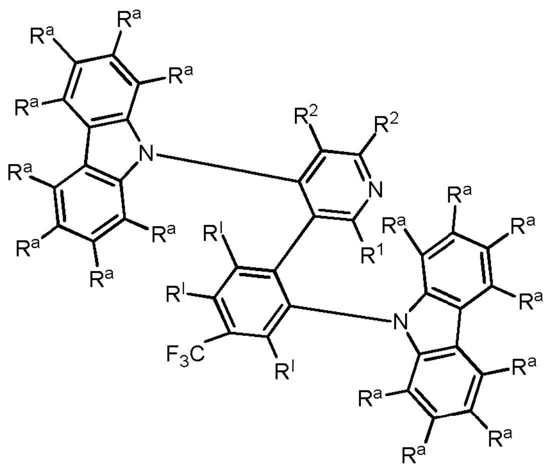

Formula IV-1

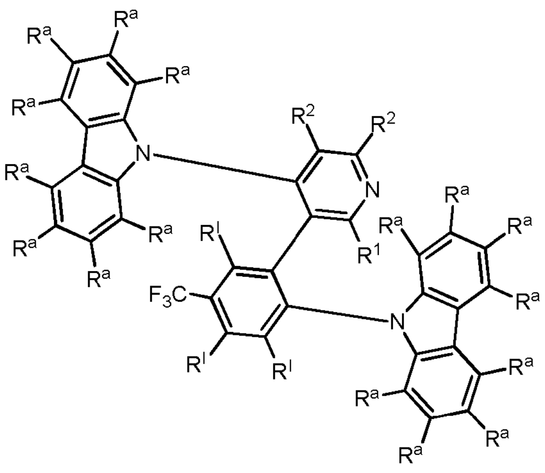

Formula IV-2

wherein the aforementioned definitions apply.

In a further embodiment of the invention, the organic molecules comprise or consist of a structure selected from the group of Formula IVa-1 and Formula IVa-2:

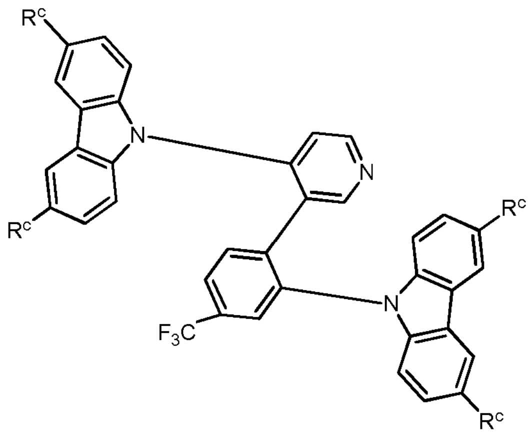

Formula IVa-1

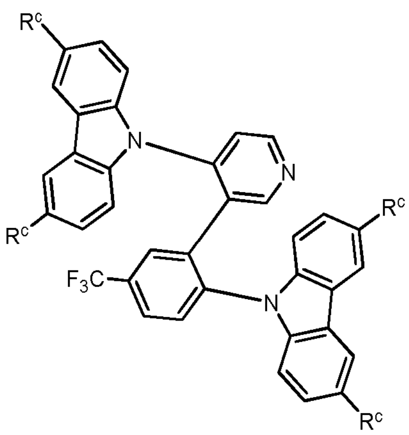

Formula IVa-2

wherein the aforementioned definitions apply.

In a preferred embodiment of the invention, the organic molecules comprise or consist of a structure of Formula IVa-1.

In a further embodiment of the invention, the organic molecules comprise or consist of a structure selected from the group of Formula IVb-1 and Formula IVb-2:

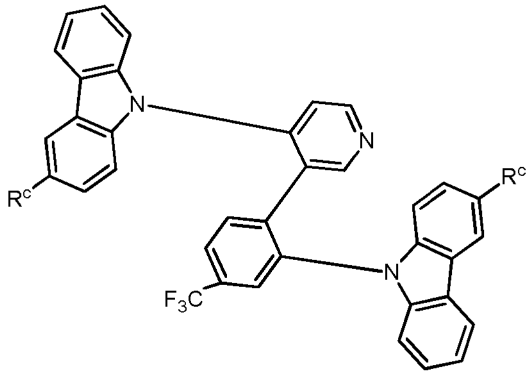

Formula IVb-1

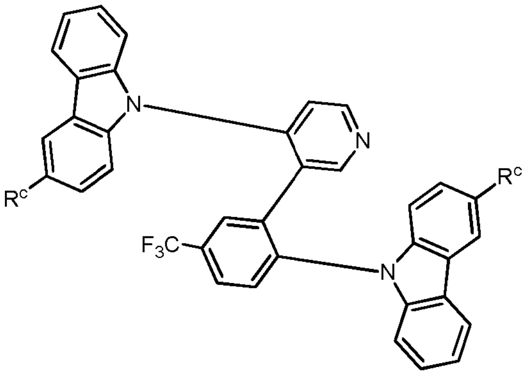

Formula IVb-2

wherein the aforementioned definitions apply.

In a preferred embodiment of the invention, the organic molecules comprise or consist of a structure of Formula IVb-1.

In one embodiment of the invention, the organic molecules comprise or consist of a structure of Formula V:

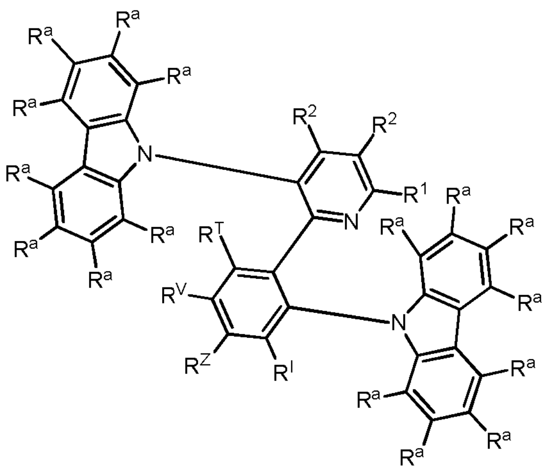

Formula V

wherein the aforementioned definitions apply and wherein exactly one substituent selected from the group consisting of $R^T$, $R^V$ and $R^Z$ is $CF_3$.

In a preferred embodiment of the invention, the organic molecules comprise or consist of a structure of Formula V and $R^V$ is $CF_3$.

In a further embodiment of the invention, the organic molecules comprise or consist of a structure selected from the group of Formula V-1 and Formula V-2:

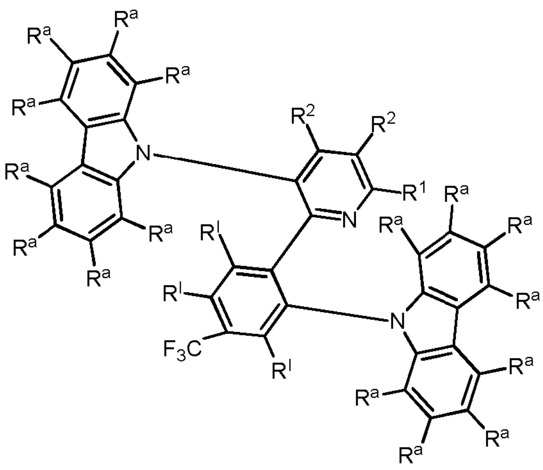

Formula V-1

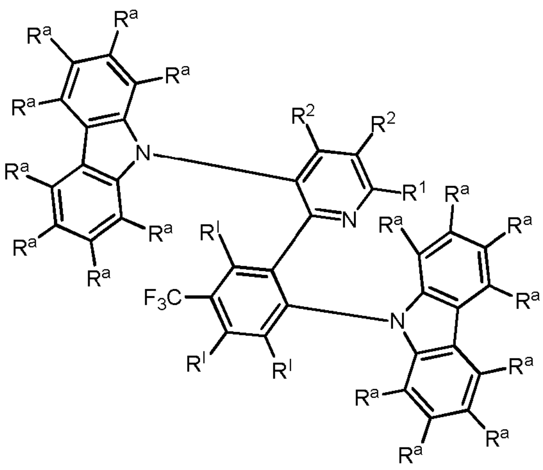

Formula V-2

wherein the aforementioned definitions apply.

In one embodiment of the invention, the organic molecules comprise or consist of a structure of Formula VI:

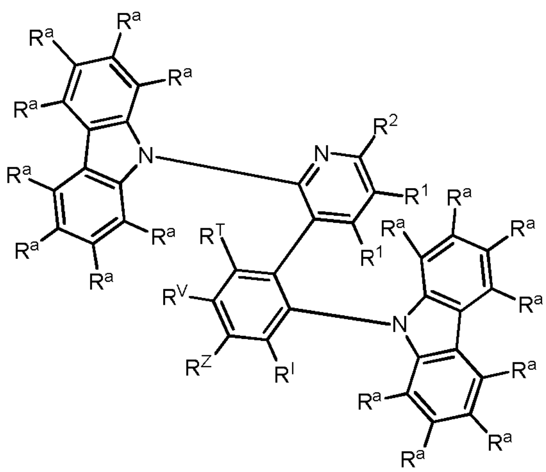

Formula VI

wherein the aforementioned definitions apply and wherein exactly one substituent selected from the group consisting of $R^T$, $R^V$ and $R^Z$ is $CF_3$.

In a preferred embodiment of the invention, the organic molecules comprise or consist of a structure of Formula VI and $R^V$ is $CF_3$.

In a further embodiment of the invention, the organic molecules comprise or consist of a structure selected from the group of Formula VI-1 and Formula VI-2:

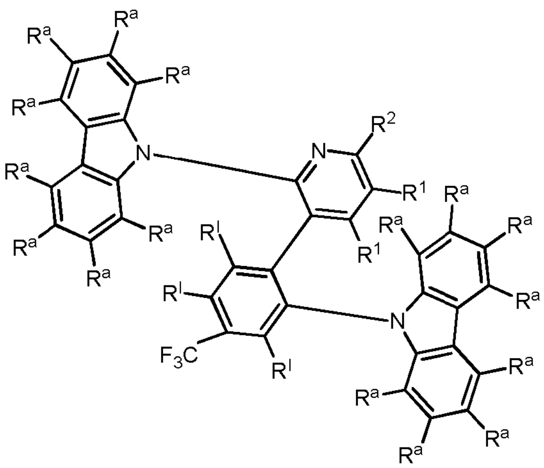

Formula VI-1

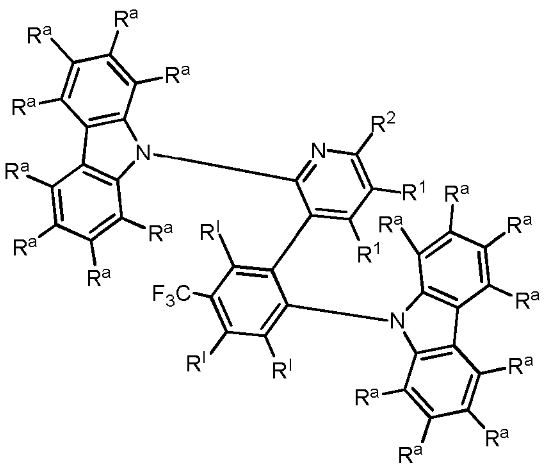

Formula VI-2

wherein the aforementioned definitions apply.

In one embodiment of the invention, the organic molecules comprise or consist of a structure of Formula VII:

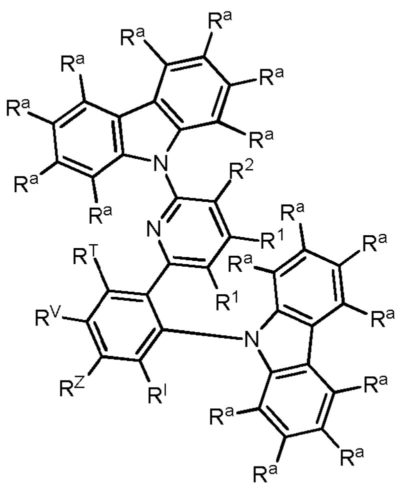

Formula VII

wherein the aforementioned definitions apply and wherein exactly one substituent selected from the group consisting of $R^T$, $R^V$ and $R^Z$ is $CF_3$.

In another embodiment of the invention, the organic molecules comprise or consist of a structure of Formula VII and $R^V$ is $CF_3$.

In one embodiment of the invention, the organic molecules comprise or consist of a structure of Formula VIII:

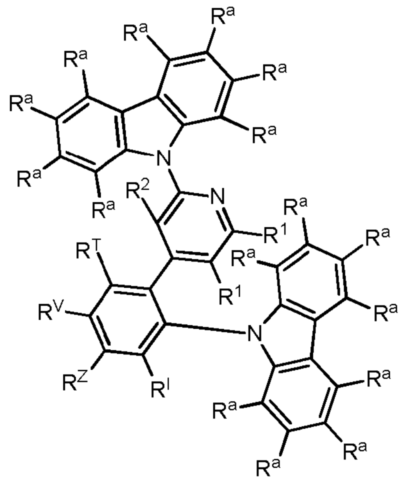

Formula VIII

wherein the aforementioned definitions apply and wherein exactly one substituent selected from the group consisting of $R^T$, $R^V$ and $R^Z$ is $CF_3$.

In another embodiment of the invention, the organic molecules comprise or consist of a structure of Formula VIII and $R^V$ is $CF_3$.

In one embodiment of the invention, the organic molecules comprise or consist of a structure of Formula IX:

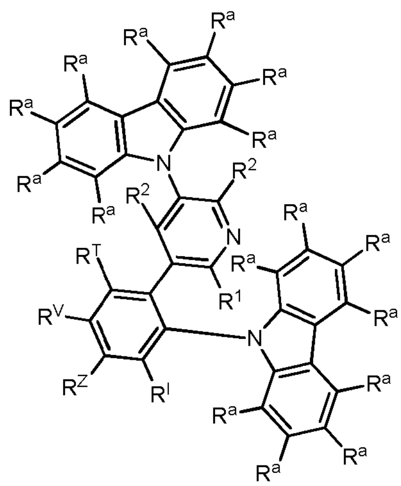

Formula IX

wherein the aforementioned definitions apply and wherein exactly one substituent selected from the group consisting of $R^T$, $R^V$ and $R^Z$ is $CF_3$.

In another embodiment of the invention, the organic molecules comprise or consist of a structure of Formula IX and $R^V$ is $CF_3$.

In one embodiment of the invention, the organic molecules comprise or consist of a structure of Formula X:

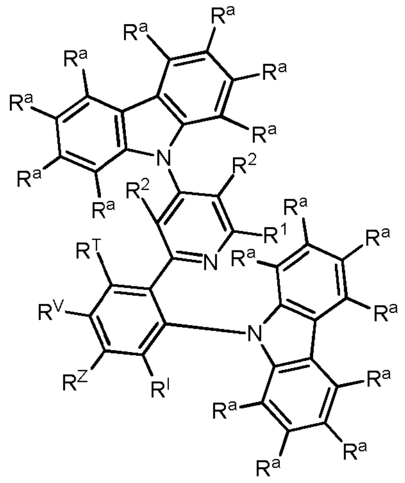

Formula X

wherein the aforementioned definitions apply and wherein exactly one substituent selected from the group consisting of $R^T$, $R^V$ and $R^Z$ is $CF_3$.

In another embodiment of the invention, the organic molecules comprise or consist of a structure of Formula X and $R^V$ is $CF_3$.

In one embodiment of the invention, the organic molecules comprise or consist of a structure of Formula XI:

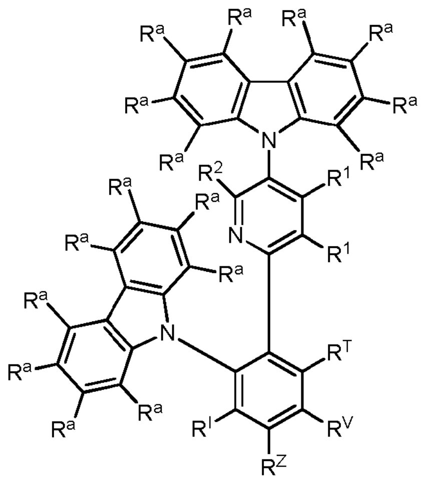

Formula XI

wherein the aforementioned definitions apply and wherein exactly one substituent selected from the group consisting of $R^T$, $R^V$ and $R^Z$ is $CF_3$.

In another embodiment of the invention, the organic molecules comprise or consist of a structure of Formula XI and $R^V$ is $CF_3$.

In one embodiment of the invention, the organic molecules comprise or consist of a structure of Formula XII:

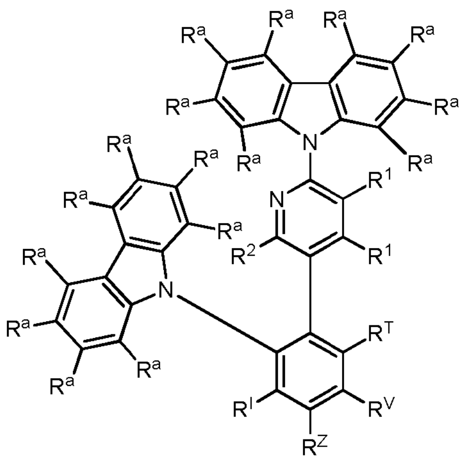

Formula XII

wherein the aforementioned definitions apply and wherein exactly one substituent selected from the group consisting of $R^T$, $R^V$ and $R^Z$ is $CF_3$.

In another embodiment of the invention, the organic molecules comprise or consist of a structure of Formula XII and $R^V$ is $CF_3$.

In one embodiment of the invention, the organic molecules comprise or consist of a structure of Formula XIII:

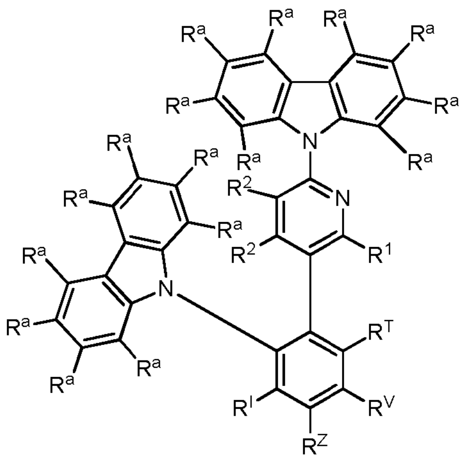

Formula XIII

wherein the aforementioned definitions apply and wherein exactly one substituent selected from the group consisting of $R^T$, $R^V$ and $R^Z$ is $CF_3$.

In another embodiment of the invention, the organic molecules comprise or consist of a structure of Formula XIII and $R^V$ is $CF_3$.

In one embodiment of the invention, the organic molecules comprise or consist of a structure of Formula XIV:

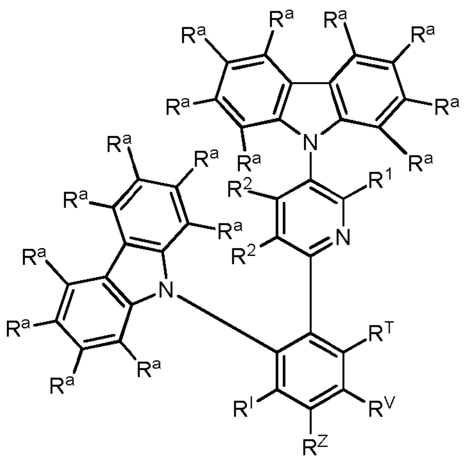

Formula XIV

wherein the aforementioned definitions apply and wherein exactly one substituent selected from the group consisting of $R^T$, $R^V$ and $R^Z$ is $CF_3$.

In another embodiment of the invention, the organic molecules comprise or consist of a structure of Formula XIV and $R^V$ is $CF_3$.

In one embodiment of the invention, the organic molecules comprise or consist of a structure of Formula XV:

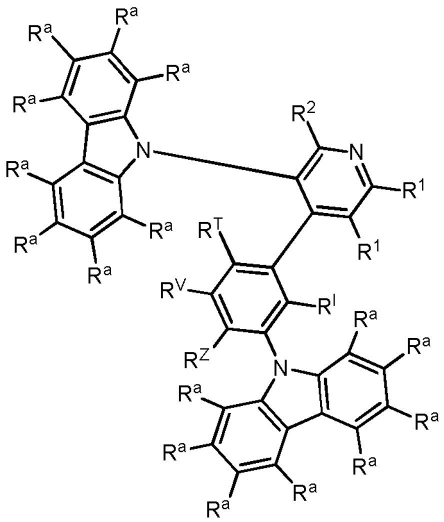

Formula XV

wherein the aforementioned definitions apply,
and wherein exactly one substituent selected from the group consisting of $R^V$, $R^T$ and $R^Z$ is $CF_3$.

In another embodiment of the invention, the organic molecules comprise or consist of a structure of Formula XV and $R^T$ is $CF_3$.

In one embodiment of the invention, the organic molecules comprise or consist of a structure of Formula XVI:

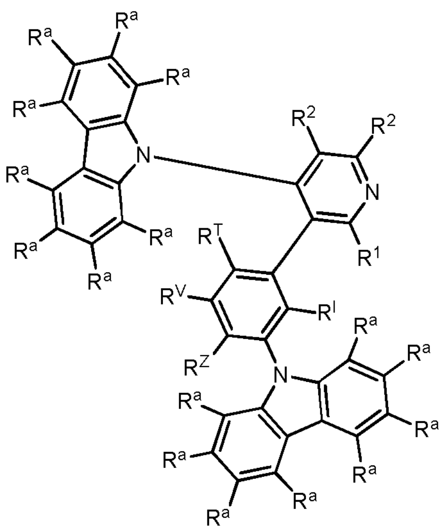

Formula XVI

wherein the aforementioned definitions apply,
and wherein exactly one substituent selected from the group consisting of $R^V$, $R^T$ and $R^Z$ is $CF_3$.

In another embodiment of the invention, the organic molecules comprise or consist of a structure of Formula XVI and $R^T$ is $CF_3$.

In one embodiment of the invention, the organic molecules comprise or consist of a structure of Formula XVII:

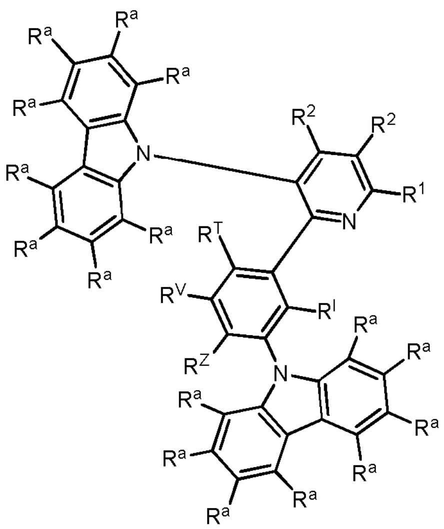

Formula XVII

wherein the aforementioned definitions apply,
and wherein exactly one substituent selected from the group consisting of $R^V$, $R^T$ and $R^Z$ is $CF_3$.

In another embodiment of the invention, the organic molecules comprise or consist of a structure of Formula XVII and $R^T$ is $CF_3$.

In one embodiment of the invention, the organic molecules comprise or consist of a structure of Formula XVIII:

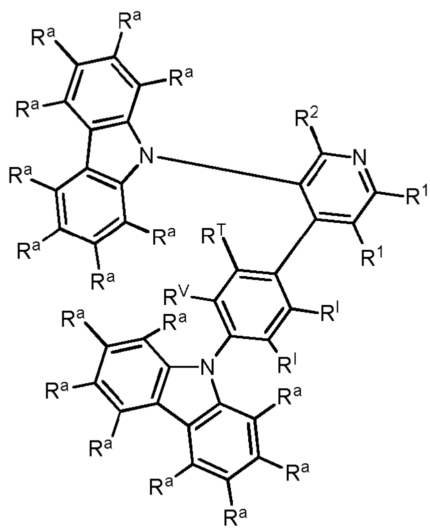

Formula XVIII

wherein the aforementioned definitions apply,
and wherein exactly one substituent selected from the group consisting of $R^V$ and $R^T$ is $CF_3$.

In another embodiment of the invention, the organic molecules comprise or consist of a structure of Formula XVIII and $R^T$ is $CF_3$.

In one embodiment of the invention, the organic molecules comprise or consist of a structure of Formula XIX:

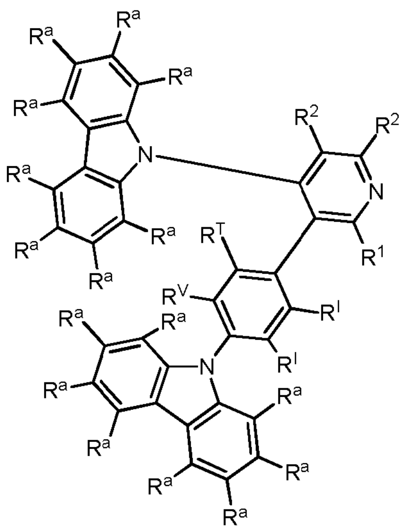

Formula XIX

wherein the aforementioned definitions apply,
and wherein exactly one substituent selected from the group consisting of $R^V$ and $R^T$ is $CF_3$.

In another embodiment of the invention, the organic molecules comprise or consist of a structure of Formula XIX and $R^T$ is $CF_3$.

In one embodiment of the invention, the organic molecules comprise or consist of a structure of Formula XX:

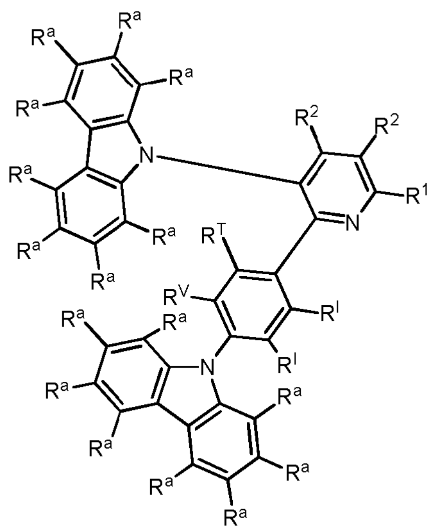

Formula XX

wherein the aforementioned definitions apply,
and wherein exactly one substituent selected from the group consisting of $R^V$ and $R^T$ is $CF_3$.

In another embodiment of the invention, the organic molecules comprise or consist of a structure of Formula XX and $R^T$ is $CF_3$.

In one embodiment of the invention, the organic molecules comprise or consist of a structure of Formula XXI:

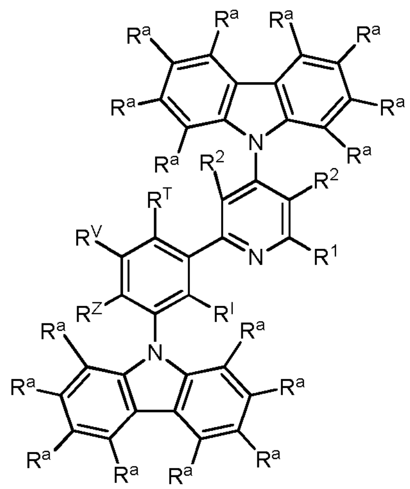

Formula XXI

wherein the aforementioned definitions apply,
and wherein exactly one substituent selected from the group consisting of $R^V$, $R^T$ and $R^Z$ is $CF_3$.

In another embodiment of the invention, the organic molecules comprise or consist of a structure of Formula XXI and $R^T$ is $CF_3$.

In one embodiment of the invention, the organic molecules comprise or consist of a structure of Formula XXII:

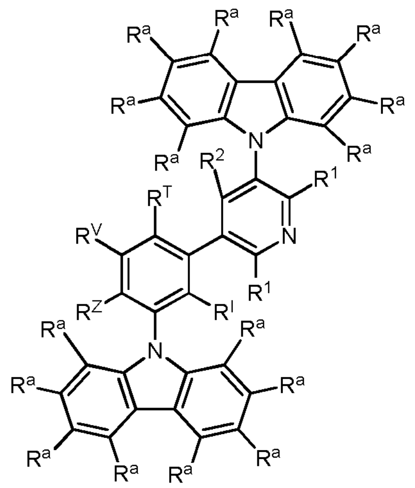

Formula XXII

wherein the aforementioned definitions apply,
and wherein exactly one substituent selected from the group consisting of $R^V$, $R^T$ and $R^Z$ is $CF_3$.

In another embodiment of the invention, the organic molecules comprise or consist of a structure of Formula XXII and $R^T$ is $CF_3$.

In one embodiment of the invention, the organic molecules comprise or consist of a structure of Formula XXIII:

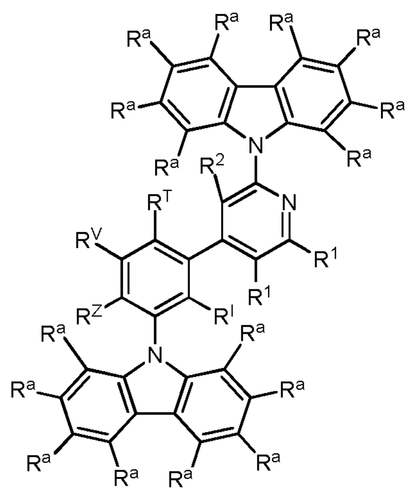

Formula XXIII

wherein the aforementioned definitions apply,
and wherein exactly one substituent selected from the group consisting of $R^V$, $R^T$ and $R^Z$ is $CF_3$.

In another embodiment of the invention, the organic molecules comprise or consist of a structure of Formula XXIII and $R^T$ is $CF_3$.

In another embodiment of the invention, the organic molecules comprise or consist of a structure of Formula XXIII and $R^V$ is $CF_3$.

In one embodiment of the invention, the organic molecules comprise or consist of a structure of Formula XXIV:

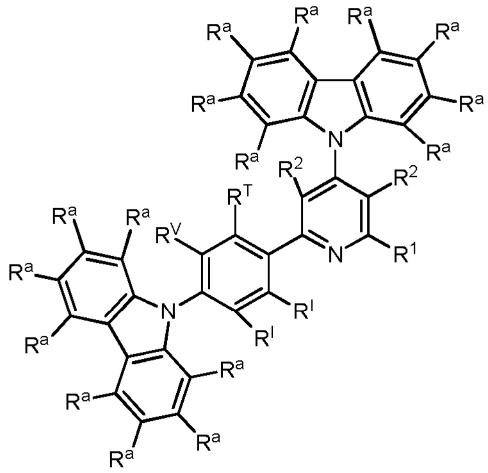

Formula XXIV

wherein the aforementioned definitions apply,
and wherein exactly one substituent selected from the group consisting of $R^V$ and $R^T$ is $CF_3$.

In another embodiment of the invention, the organic molecules comprise or consist of a structure of Formula XXIV and $R^T$ is $CF_3$.

In one embodiment of the invention, the organic molecules comprise or consist of a structure of Formula XXV:

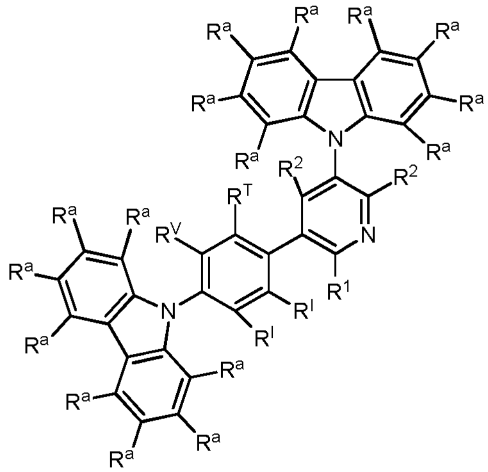

Formula XXV

wherein the aforementioned definitions apply,
and wherein exactly one substituent selected from the group consisting of $R^V$ and $R^T$ is $CF_3$.

In another embodiment of the invention, the organic molecules comprise or consist of a structure of Formula XXV and $R^T$ is $CF_3$.

In one embodiment of the invention, the organic molecules comprise or consist of a structure of Formula XXVI:

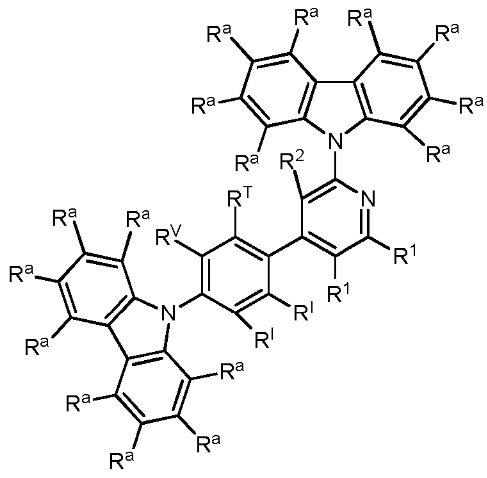

Formula XXVI

wherein the aforementioned definitions apply,
and wherein exactly one substituent selected from the group consisting of $R^V$ and $R^T$ is $CF_3$.

In another embodiment of the invention, the organic molecules comprise or consist of a structure of Formula XXVI and $R^T$ is $CF_3$.

In one embodiment of the invention, the organic molecules comprise or consist of a structure of Formula XXVII:

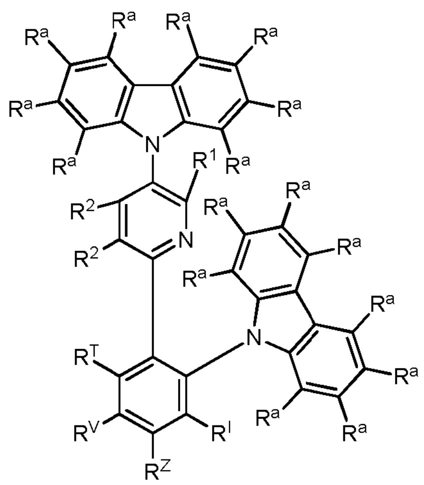

Formula XXVII

wherein the aforementioned definitions apply,
and wherein exactly one substituent selected from the group consisting of $R^V$ and $R^T$ is $CF_3$.

In another embodiment of the invention, the organic molecules comprise or consist of a structure of Formula XXVII and $R^V$ is $CF_3$.

In one embodiment of the invention, the organic molecules comprise or consist of a structure of Formula XXVIII:

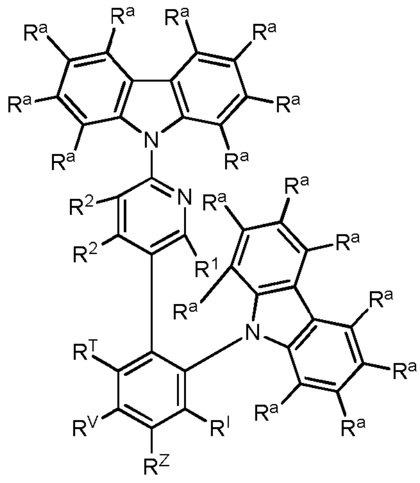

Formula XXVIII

wherein the aforementioned definitions apply,
and wherein exactly one substituent selected from the group consisting of $R^V$ and $R^T$ is $CF_3$.

In another embodiment of the invention, the organic molecules comprise or consist of a structure of Formula XXVIII and $R^V$ is $CF_3$.

In one embodiment of the invention, the organic molecules comprise or consist of a structure of Formula XXIX:

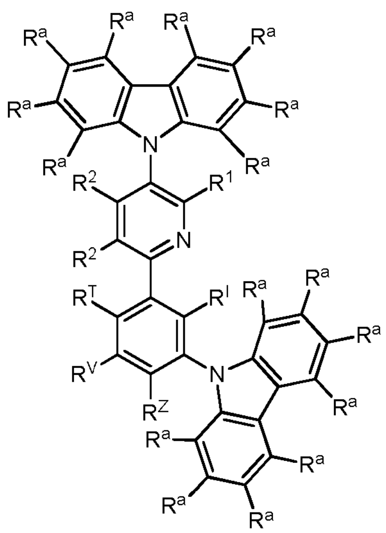

Formula XXIX

wherein the aforementioned definitions apply,
and wherein exactly one substituent selected from the group consisting of $R^V$ and $R^T$ is $CF_3$.

In another embodiment of the invention, the organic molecules comprise or consist of a structure of Formula XXIX and $R^T$ is $CF_3$.

In one embodiment of the invention, the organic molecules comprise or consist of a structure of Formula XXX:

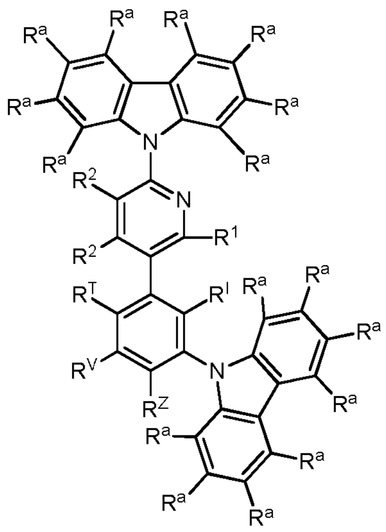

Formula XXX

wherein the aforementioned definitions apply,
and wherein exactly one substituent selected from the group consisting of $R^V$ and $R^T$ is $CF_3$.

In another embodiment of the invention, the organic molecules comprise or consist of a structure of Formula XXX and $R^T$ is $CF_3$.

In one embodiment of the invention, the organic molecules comprise or consist of a structure of Formula XXXI:

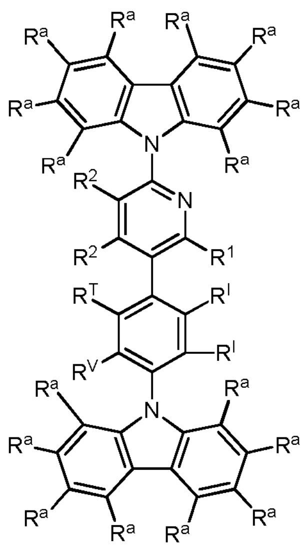

Formula XXXI

wherein the aforementioned definitions apply,
and wherein exactly one substituent selected from the group consisting of $R^V$ and $R^T$ is $CF_3$.

In another embodiment of the invention, the organic molecules comprise or consist of a structure of Formula XXXI and $R^T$ is $CF_3$.

In one embodiment of the invention, the organic molecules comprise or consist of a structure of Formula XXXII:

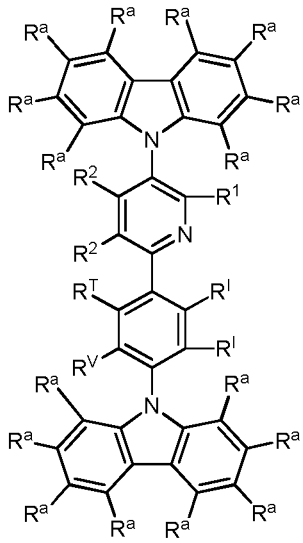

Formula XXXII

wherein the aforementioned definitions apply,
and wherein exactly one substituent selected from the group consisting of $R^V$ and $R^T$ is $CF_3$.

In another embodiment of the invention, the organic molecules comprise or consist of a structure of Formula XXXII and $R^T$ is $CF_3$.

In one embodiment of the invention $R^c$ is at each occurrence independently from another selected from the group consisting of
Me,
$^i$Pr,
$^t$Bu,
Ph, which is optionally substituted with one or more substituents independently from each other selected from the group consisting of Me, $^i$Pr, $^t$Bu, CN, $CF_3$ and Ph; and
triazinyl, which is optionally substituted with one or more substituents independently from each other selected from the group consisting of Me, $^i$Pr, $^t$Bu, CN, $CF_3$ and Ph.

As used throughout the present application, the terms "aryl" and "aromatic" may be understood in the broadest sense as any mono-, bi- or polycyclic aromatic moieties. Accordingly, an aryl group contains 6 to 60 aromatic ring atoms, and a heteroaryl group contains 5 to 60 aromatic ring atoms, of which at least one is a heteroatom. Notwithstanding, throughout the application the number of aromatic ring atoms may be given as subscripted number in the definition of certain substituents. In particular, the heteroaromatic ring includes one to three heteroatoms. Again, the terms "heteroaryl" and "heteroaromatic" may be understood in the broadest sense as any mono-, bi- or polycyclic hetero-aromatic moieties that include at least one heteroatom. The heteroatoms may at each occurrence be the same or different and be individually selected from the group consisting of N, O and S. Accordingly, the term "arylene" refers to a divalent substituent that bears two binding sites to other molecular structures and thereby serving as a linker structure. In case, a group in the exemplary embodiments is defined differently from the definitions given here, for example, the number of aromatic ring atoms or number of heteroatoms differs from the given definition, the definition in the exemplary embodiments is to be applied. According to the invention, a condensed (annulated) aromatic or heteroaromatic polycycle is built of two or more single aromatic or heteroaromatic cycles, which formed the polycycle via a condensation reaction.

In particular, as used throughout the present application the term aryl group or heteroaryl group comprises groups which can be bound via any position of the aromatic or heteroaromatic group, derived from benzene, naphthaline, anthracene, phenanthrene, pyrene, dihydropyrene, chrysene, perylene, fluoranthene, benzanthracene, benzphenanthrene, tetracene, pentacene, benzpyrene, furan, benzofuran, isobenzofuran, dibenzofuran, thiophene, benzothiophene, isobenzothiophene, dibenzothiophene; pyrrole, indole, isoindole, carbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthoimidazole, phenanthroimidazole, pyridoimidazole, pyrazinoimidazole, quinoxalinoimidazole, oxazole, benzoxazole, napthooxazole, anthroxazol, phenanthroxazol, isoxazole, 1,2-thiazole, 1,3-thiazole, benzothiazole, pyridazine, benzopyridazine, pyrimidine, benzopyrimidine, 1,3,5-triazine, quinoxaline, pyrazine, phenazine, naphthyridine, carboline, benzocarboline, phenanthroline, 1,2,3-triazole, 1,2,4-triazole, benzotriazole, 1,2,3-oxadiazole, 1,2,4-oxadiazole, 1,2,5-oxadiazole, 1,2,3,4-tetrazine, purine, pteridine, indolizine and benzothiadiazole or combinations of the abovementioned groups.

As used throughout the present application the term cyclic group may be understood in the broadest sense as any mono-, bi- or polycyclic moieties.

As used throughout the present application the term biphenyl as a substituent may be understood in the broadest sense as ortho-biphenyl, meta-biphenyl, or para-biphenyl, wherein ortho, meta and para is defined in regard to the binding site to another chemical moiety.

As used throughout the present application the term alkyl group may be understood in the broadest sense as any linear, branched, or cyclic alkyl substituent. In particular, the term alkyl comprises the substituents methyl (Me), ethyl (Et), n-propyl ($^{n}$Pr), i-propyl ($^{i}$Pr), cyclopropyl, n-butyl ($^{n}$Bu), i-butyl ($^{i}$Bu), s-butyl ($^{s}$Bu), t-butyl($^{t}$Bu),cyclobutyl, 2-methylbutyl, n-pentyl, s-pentyl, t-pentyl, 2-pentyl, neo-pentyl, cyclopentyl, n-hexyl, s-hexyl, t-hexyl, 2-hexyl, 3-hexyl, neo-hexyl, cyclohexyl, 1-methylcyclopentyl, 2-methylpentyl, n-heptyl, 2-heptyl, 3-heptyl, 4-heptyl, cycloheptyl, 1-methylcyclohexyl, n-octyl, 2-ethylhexyl, cyclooctyl, 1-bicyclo[2,2,2]octyl, 2-bicyclo[2,2,2]-octyl, 2-(2,6-dimethyl)octyl, 3-(3,7-dimethyl)octyl, adamantyl, 2,2,2-trifluorethyl, 1,1-dimethyl-n-hex-1-yl, 1,1-dimethyl-n-hept-1-yl, 1,1-dimethyl-n-oct-1-yl, 1,1-dimethyl-n-dec-1-yl, 1,1-dimethyl-n-dodec-1-yl, 1,1-dimethyl-n-tetradec-1-yl, 1,1-dimethyl-n-hexadec-1-yl, 1,1-dimethyl-n-octadec-1-yl, 1,1-diethyl-n-hex-1-yl, 1,1-diethyl-n-hept-1-yl, 1,1-diethyl-n-oct-1-yl, 1,1-diethyl-n-dec-1-yl, 1,1-diethyl-n-dodec-1-yl, 1,1-diethyl-n-tetradec-1-yl, 1,1-diethyln-n-hexadec-1-yl, 1,1-diethyl-n-octadec-1-yl, 1-(n-propyl)-cyclohex-1-yl, 1-(n-butyl)-cyclohex-1-yl, 1-(n-hexyl)-cyclohex-1-yl, 1-(n-octyl)-cyclohex-1-yl and 1-(n-decyl)-cyclohex-1-yl.

As used throughout the present application the term alkenyl comprises linear, branched, and cyclic alkenyl substituents. The term alkenyl group exemplarily comprises the substituents ethenyl, propenyl, butenyl, pentenyl, cyclopentenyl, hexenyl, cyclohexenyl, heptenyl, cycloheptenyl, octenyl, cyclooctenyl or cyclooctadienyl.

As used throughout the present application the term alkynyl comprises linear, branched, and cyclic alkynyl substituents. The term alkynyl group exemplarily comprises ethynyl, propynyl, butynyl, pentynyl, hexynyl, heptynyl or octynyl.

As used throughout the present application the term alkoxy comprises linear, branched, and cyclic alkoxy substituents. The term alkoxy group exemplarily comprises methoxy, ethoxy, n-propoxy, i-propoxy, n-butoxy, i-butoxy, s-butoxy, t-butoxy and 2-methylbutoxy.

As used throughout the present application the term thioalkoxy comprises linear, branched, and cyclic thioalkoxy substituents, in which the O of the exemplarily alkoxy groups is replaced by S.

As used throughout the present application, the terms "halogen" and "halo" may be understood in the broadest sense as being preferably fluorine, chlorine, bromine or iodine.

Whenever hydrogen (H) is mentioned herein, it could also be replaced by deuterium at each occurrence.

It is understood that when a molecular fragment is described as being a substituent or otherwise attached to another moiety, its name may be written as if it were a fragment (e.g. naphtyl, dibenzofuryl) or as if it were the whole molecule (e.g. naphthalene, dibenzofuran). As used herein, these different ways of designating a substituent or attached fragment are considered to be equivalent.

In one embodiment, the organic molecules according to the invention have an excited state lifetime of not more than 150 μs, of not more than 100 μs, in particular of not more than 50 μs, more preferably of not more than 10 μs or not more than 7 μs in a film of poly(methyl methacrylate) (PMMA) with 10 % by weight of organic molecule at room temperature.

In one embodiment of the invention, the organic molecules according to the invention represent thermally-activated delayed fluorescence (TADF) emitters, which exhibit a $\Delta E_{ST}$ value, which corresponds to the energy difference between the first excited singlet state (S1) and the first excited triplet state (T1), of less than 5000 $cm^{-1}$, preferably less than 3000 $cm^{-1}$, more preferably less than 1500 $cm^{-1}$, even more preferably less than 1000 $cm^{-1}$ or even less than 500 $cm^{-1}$.

In a further embodiment of the invention, the organic molecules according to the invention have an emission peak in the visible or nearest ultraviolet range, i.e., in the range of a wavelength of from 380 to 800 nm, with a full width at half maximum of less than 0.50 eV, preferably less than 0.48 eV, more preferably less than 0.45 eV, even more preferably less than 0.43 eV or even less than 0.40 eV in a film of poly(methyl methacrylate) (PMMA) with 10 % by weight of organic molecule at room temperature.

In a further embodiment of the invention, the organic molecules according to the invention have a "blue material index" (BMI), calculated by dividing the photoluminescence quantum yield (PLQY) in % by the CIEy color coordinate of the emitted light, of more than 150, in particular more than 200, preferably more than 250, more preferably of more than 300 or even more than 500.

Orbital and excited state energies can be determined either by means of experimental methods or by calculations employing quantum-chemical methods, in particular density functional theory calculations. The energy of the highest occupied molecular orbital $E^{HOMO}$ is determined by methods known to the person skilled in the art from cyclic voltammetry measurements with an accuracy of 0.1 eV. The energy of the lowest unoccupied molecular orbital $E^{LUMO}$ is calculated as $E^{HOMO} + E^{gap}$, wherein $E^{gap}$ is determined as follows: For host compounds, the onset of the emission spectrum of a film with 10 % by weight of host in poly(methyl methacrylate) (PMMA) is used as $E^{gap}$, unless stated otherwise. For emitter molecules, $E^{gap}$ is determined as the energy at which the excitation and emission spectra of a film with 10 % by

weight of emitter in PMMA cross.

The energy of the first excited triplet state T1 is determined from the onset of the emission spectrum at low temperature, typically at 77 K. For host compounds, where the first excited singlet state and the lowest triplet state are energetically separated by > 0.4 eV, the phosphorescence is usually visible in a steady-state spectrum in 2-Me-THF. The triplet energy can thus be determined as the onset of the phosphorescence spectrum. For TADF emitter molecules, the energy of the first excited triplet state T1 is determined from the onset of the delayed emission spectrum at 77 K, if not otherwise stated measured in a film of PMMA with 10 % by weight of emitter. Both for host and emitter compounds, the energy of the first excited singlet state S1 is determined from the onset of the emission spectrum, if not otherwise stated measured in a film of PMMA with 10 % by weight of host or emitter compound.

The onset of an emission spectrum is determined by computing the intersection of the tangent to the emission spectrum with the x-axis. The tangent to the emission spectrum is set at the high-energy side of the emission band and at the point at half maximum of the maximum intensity of the emission spectrum.

A further aspect of the invention relates to a process for preparing organic molecules (with an optional subsequent reaction) according to the invention, wherein a halo-fluorobenzotrifluoride, which is substituted with three $R^I$, is used as a reactant:

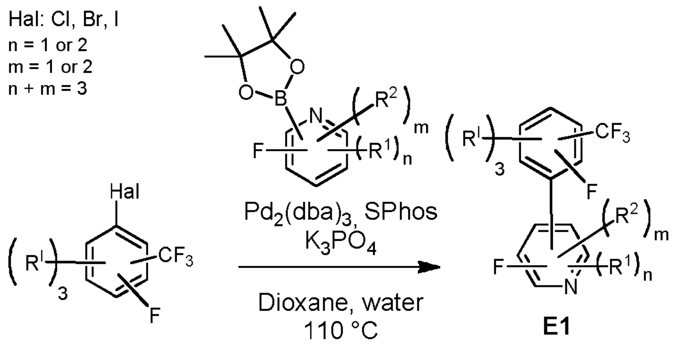


E1

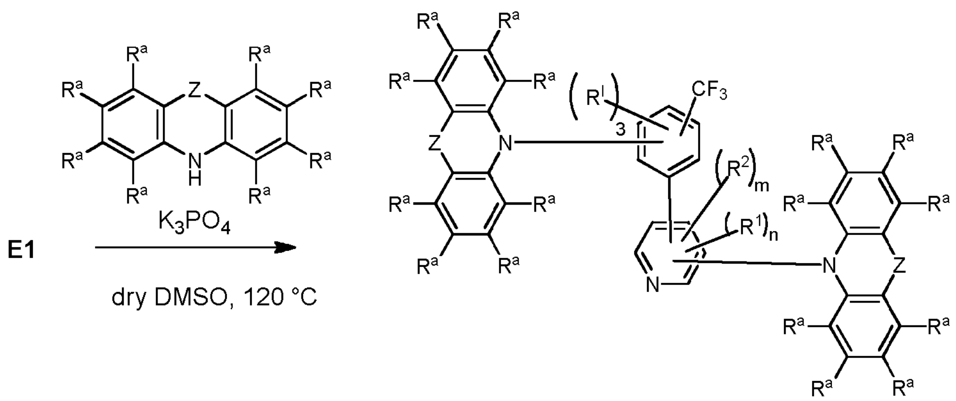


wherein Hal is selected from the group consisting Cl, Br and I and $R^I$ is, as described, at each occurrence independently from another selected from the group comprising

deuterium,

$C_1$-$C_5$-alkyl,

wherein one or more hydrogen atoms are optionally substituted by deuterium;

C2-C8-alkenyl,

wherein one or more hydrogen atoms are optionally substituted by deuterium;

C2-C8-alkynyl,

wherein one or more hydrogen atoms are optionally substituted by deuterium; and

$C_6$-$C_{18}$-aryl,

which is optionally substituted with one or more substituents $R^6$.

According to the invention, in the reaction for the synthesis of E1 a boronic acid or an equivalent boronic acid ester can be used instead of a boronic pinacol ester. Exemplary boronic acid esters or boronic acids are 3-fluoropyridine-2-boronic ester or acid, 4-fluoropyridine-2-boronic ester or acid, 5-fluoropyridine-2-boronic ester or acid, 4-fluoropyridine-3-boronic ester or acid, 5-fluoropyridine-3-boronic ester or acid, 2-fluoropyridine-5-boronic ester or acid, 3-fluoropyridine-4-boronic ester or acid, 2-fluoropyridine-4-boronic ester or acid, which are each optionally either one $R^1$ and two $R^2$ or two $R^1$ and one $R^2$ at the remaining positions.

Preferably, the halo-fluoro-benzotrifluoride is selected from chloro-fluorobenzotrifluoride and bromo-fluorobenzotrifluoride. Exemplary halo-fluoro-benzotrifluoride are 4-chloro-3-fluoro-benzotrifluoride, 4-chloro-2-fluoro-benzotrifluoride, 3-chloro-4-fluoro-benzotrifluoride, 3-chloro-5-fluoro-benzotrifluoride, 3-chloro-6-fluoro-benzotrifluoride, 3-chloro-2-fluoro-benzotrifluoride, 2-chloro-3-fluoro-benzotrifluoride, 2-chloro-4-fluoro-benzotrifluoride, 2-chloro-5-fluoro-benzotrifluoride, 2-chloro-6-fluoro-benzotrifluoride, 3-bromo-4-fluoro-benzotrifluoride, 3-bromo-5-fluoro-benzotrifluoride, 3-bromo-6-fluoro-benzotrifluoride, 3-bromo-2-fluoro-benzotrifluoride, 2-bromo-3-fluoro-benzotrifluoride, 2-bromo-4-fluoro-benzotrifluoride, 2-bromo-5-fluoro-benzotrifluoride and 2-bromo-6-fluoro-benzotrifluoride, which are each optionally substituted with three $R^I$ at the remaining positions.

An alternative synthesis route is described in the following synthesis scheme:

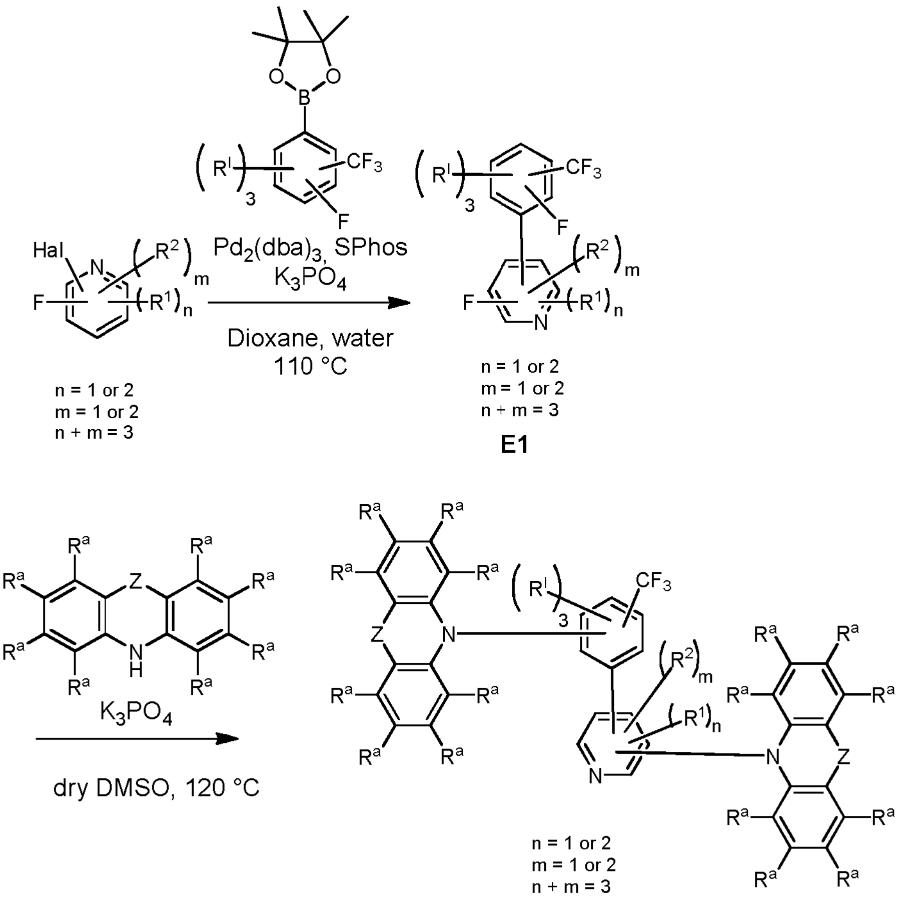


According to the invention, in the reaction for the synthesis of **E1** in the alternative synthesis route a boronic acid or an equivalent boronic acid ester can be used instead of a boronic pinacol ester. Exemplary boronic acid esters or boronic acids are 4-trifluoromethyl-2-fluorophenylboronic ester or acid, 5-trifluoromethyl-2-fluorophenylboronic ester or acid, 3-trifluoromethyl-2-fluorophenylboronic ester or acid, 6-trifluoromethyl-2-fluorophenylboronic ester or acid, 2- trifluoromethyl-3-fluorophenylboronic ester or acid, 4-trifluoromethyl-3-fluorophenylboronic ester or acid, 5-trifluoromethyl-3-fluorophenylboronic ester or acid, 6-trifluoromethyl-3-fluorophenylboronic ester or acid, 2-trifluoromethyl-4-fluorophenylboronic ester or acid and 3-trifluoromethyl-4-fluorophenylboronic ester or acid.

Preferably, the halo-fluoro-pyridine is selected from chloro-fluoropyridine and bromo-fluoropyridine. Exemplary halo-fluoro-pyridines are 4-chloro-3-fluoro-pyridine, 4-chloro-2-fluoro-pyridine, 3-chloro-4-fluoro-pyridine, 3-chloro-5-fluoro-pyridine, 3-chloro-6-fluoro-pyridine, 3-chloro-2-fluoro-pyridine, 2-chloro-3-fluoro-pyridine, 2-chloro-4-fluoro-pyridine, 2-chloro-5-fluoro-pyridine, 2-chloro-6-fluoro-pyridine, 3-bromo-4-fluoro-pyridine, 3-bromo-5-fluoro-pyridine, 3-bromo-6-fluoro-pyridine, 3-bromo-2-fluoro-pyridine, 2-bromo-3-fluoro-pyridine, 2-bromo-4-fluoro-pyridine, 2-bromo-5-fluoro-pyridine and 2-bromo-6-fluoro-pyridine, which are each substituted with either one $R^1$ and two $R^2$ or two $R^1$ and one $R^2$ at the remaining positions.

For the reaction of a nitrogen heterocycle in a nucleophilic aromatic substitution with an aryl halide, preferably an aryl fluoride, typical conditions include the use of a base, such as tribasic potassium phosphate or sodium hydride,

for example, in an aprotic polar solvent, such as dimethyl sulfoxide (DMSO) or N,N-dimethylformamide (DMF), for example.

An alternative synthesis route comprises the introduction of a nitrogen heterocycle via copper-or palladium-catalyzed coupling to an aryl halide or aryl pseudohalide, preferably an aryl bromide, an aryl iodide, aryl triflate or an aryl tosylate.

A further aspect of the invention relates to the use of an organic molecule according to the invention as a luminescent emitter or as an absorber, and/or as host material and/or as electron transport material, and/or as hole injection material, and/or as hole blocking material in an optoelectronic device.

The optoelectronic device may be understood in the broadest sense as any device based on organic materials that is suitable for emitting light in the visible or nearest ultraviolet (UV) range, i.e., in the range of a wavelength of from 380 to 800 nm. More preferably, the optoelectronic device may be able to emit light in the visible range, i.e., of from 400 nm to 800 nm.

In the context of such use, the optoelectronic device is more particularly selected from the group consisting of:

- organic light-emitting diodes (OLEDs),
- light-emitting electrochemical cells,
- OLED sensors, especially in gas and vapour sensors not hermetically externally shielded,
- organic diodes,
- organic solar cells,
- organic transistors,
- organic field-effect transistors,
- organic lasers, and
- down-conversion elements.

In a preferred embodiment in the context of such use, the optoelectronic device is a device selected from the group consisting of an organic light emitting diode (OLED), a light emitting electrochemical cell (LEC), and a light-emitting transistor.

In the case of the use, the fraction of the organic molecule according to the invention in the emission layer in an optoelectronic device, more particularly in OLEDs, is 1 % to 99 % by weight, more particularly 5 % to 80 % by weight. In an alternative embodiment, the proportion of the organic molecule in the emission layer is 100 % by weight.

In one embodiment, the light-emitting layer comprises not only the organic molecules according to the invention but also a host material whose triplet (T1) and singlet (S1) energy levels are energetically higher than the triplet (T1) and singlet (S1) energy levels of the organic molecule.

A further aspect of the invention relates to a composition comprising or consisting of:

(a) at least one organic molecule according to the invention, in particular in the form of an emitter and/or a host, and
(b) one or more emitter and/or host materials, which differ from the organic molecule according to the invention and
(c) optional one or more dyes and/or one or more solvents.

In one embodiment, the light-emitting layer comprises or essentially consists of a composition comprising or consisting of:

(a) at least one organic molecule according to the invention, in particular in the form of an emitter and/or a host, and
(b) one or more emitter and/or host materials, which differ from the organic molecule according to the invention and
(c) optional one or more dyes and/or one or more solvents.

Particularly preferably the light-emitting layer EML comprises or essentially consists of a composition comprising or consisting of:

(i) 1-50 % by weight, preferably 5-40 % by weight, in particular 10-30 % by weight, of one or more organic molecules according to the invention E;
(ii) 5-99 % by weight, preferably 30-94.9 % by weight, in particular 40-89% by weight, of at least one host compound H; and
(iii) optionally 0-94 % by weight, preferably 0.1-65 % by weight, in particular 1-50 % by weight, of at least one further host compound D with a structure differing from the structure of the molecules according to the invention; and
(iv) optionally 0-94 % by weight, preferably 0-65 % by weight, in particular 0-50 % by weight, of a solvent; and
(v) optionally 0-30 % by weight, in particular 0-20 % by weight, preferably 0-5 % by weight, of at least one further emitter molecule F with a structure differing from the structure of the molecules according to the invention.

Preferably, energy can be transferred from the host compound H to the one or more organic molecules according to the invention E, in particular transferred from the first excited triplet state T1(H) of the host compound H to the first excited triplet state T1(E) of the one or more organic molecules according to the invention E and/ or from the first excited singlet state S1(H) of the host compound H to the first excited singlet state S1(E) of the one or more organic molecules according to the invention E.

In a further embodiment, the light-emitting layer EML comprises or essentially consists of a composition comprising or consisting of:

(i) 1-50 % by weight, preferably 5-40 % by weight, in particular 10-30 % by weight, of one organic molecule according to the invention E;
(ii) 5-99 % by weight, preferably 30-94.9 % by weight, in particular 40-89% by weight, of one host compound H; and
(iii) optionally 0-94 % by weight, preferably 0.1-65 % by weight, in particular 1-50 % by weight, of at least one further host compound D with a structure differing from the structure of the molecules according to the invention; and
(iv) optionally 0-94 % by weight, preferably 0-65 % by weight, in particular 0-50 % by weight, of a solvent; and
(v) optionally 0-30 % by weight, in particular 0-20 % by weight, preferably 0-5 % by weight, of at least one further emitter molecule F with a structure differing from the structure of the molecules according to the invention.

In one embodiment, the host compound H has a highest occupied molecular orbital HOMO(H) having an energy $E^{HOMO}(H)$ in the range of from -5 to -6.5 eV and the at least one further host compound D has a highest occupied molecular orbital HOMO(D) having an energy $E^{HOMO}(D)$, wherein $E^{HOMO}(H) > E^{HOMO}(D)$.

In a further embodiment, the host compound H has a lowest unoccupied molecular orbital LUMO(H) having an energy $E^{LUMO}(H)$ and the at least one further host compound D has a lowest unoccupied molecular orbital LUMO(D) having an energy $E^{LUMO}(D)$, wherein $E^{LUMO}(H) > E^{LUMO}(D)$.

In one embodiment, the host compound H has a highest occupied molecular orbital HOMO(H) having an energy $E^{HOMO}(H)$ and a lowest unoccupied molecular orbital LUMO(H) having an energy $E^{LUMO}(H)$, and

the at least one further host compound D has a highest occupied molecular orbital HOMO(D) having an energy $E^{HOMO}(D)$ and a lowest unoccupied molecular orbital LUMO(D) having an energy $E^{LUMO}(D)$,
the organic molecule according to the invention E has a highest occupied molecular orbital HOMO(E) having an energy $E^{HOMO}(E)$ and a lowest unoccupied molecular orbital LUMO(E) having an energy $E^{LUMO}(E)$,

wherein
$E^{HOMO}(H) > E^{HOMO}(D)$ and the difference between the energy level of the highest occupied molecular orbital HOMO(E) of the organic molecule according to the invention E ($E^{HOMO}(E)$) and the energy level of the highest occupied molecular orbital HOMO(H) of the host compound H ($E^{HOMO}(H)$) is between -0.5 eV and 0.5 eV, more preferably between -0.3 eV and 0.3 eV, even more preferably between -0.2 eV and 0.2 eV or even between -0.1 eV and 0.1 eV; and $E^{LUMO}(H) > E^{LUMO}(D)$ and the difference between the energy level of the lowest unoccupied molecular orbital LUMO(E) of the organic molecule according to the invention E ($E^{LUMO}(E)$) and the lowest unoccupied molecular orbital LUMO(D) of the at least one further host compound D ($E^{LUMO}(D)$) is between -0.5 eV and 0.5 eV, more preferably between -0.3 eV and 0.3 eV, even more preferably between -0.2 eV and 0.2 eV or even between -0.1 eV and 0.1 eV.

In a further aspect, the invention relates to an optoelectronic device comprising an organic molecule or a composition of the type described here, more particularly in the form of a device selected from the group consisting of organic light-emitting diode (OLED), light-emitting electrochemical cell, OLED sensor, more particularly gas and vapour sensors not hermetically externally shielded, organic diode, organic solar cell, organic transistor, organic field-effect transistor, organic laser and down-conversion element.

In a preferred embodiment, the optoelectronic device is a device selected from the group consisting of an organic light emitting diode (OLED), a light emitting electrochemical cell (LEC), and a light-emitting transistor.

In one embodiment of the optoelectronic device of the invention, the organic molecule according to the invention E is used as emission material in a light-emitting layer EML.

In one embodiment of the optoelectronic device of the invention the light-emitting layer EML consists of the composition according to the invention described here.

Exemplarily, when the optoelectronic device is an OLED, it may exhibit the following layer structure:

1. substrate
2. anode layer A
3. hole injection layer, HIL
4. hole transport layer, HTL
5. electron blocking layer, EBL

6. emitting layer, EML
7. hole blocking layer, HBL
8. electron transport layer, ETL
9. electron injection layer, EIL
10. cathode layer,

wherein the OLED comprises each layer only optionally, different layers may be merged and the OLED may comprise more than one layer of each layer type defined above.

Furthermore, the optoelectronic device may optionally comprise one or more protective layers protecting the device from damaging exposure to harmful species in the environment including, exemplarily moisture, vapor and/or gases.

In one embodiment of the invention, the optoelectronic device is an OLED, which exhibits the following inverted layer structure:

1. substrate
2. cathode layer
3. electron injection layer, EIL
4. electron transport layer, ETL
5. hole blocking layer, HBL
6. emitting layer, B
7. electron blocking layer, EBL
8. hole transport layer, HTL
9. hole injection layer, HIL
10. anode layer A

Wherein the OLED with an inverted layer structure comprises each layer only optionally, different layers may be merged and the OLED may comprise more than one layer of each layer types defined above.

In one embodiment of the invention, the optoelectronic device is an OLED, which may exhibit stacked architecture. In this architecture, contrary to the typical arrangement, where the OLEDs are placed side by side, the individual units are stacked on top of each other. Blended light may be generated with OLEDs exhibiting a stacked architecture, in particular white light may be generated by stacking blue, green and red OLEDs. Furthermore, the OLED exhibiting a stacked architecture may optionally comprise a charge generation layer (CGL), which is typically located between two OLED subunits and typically consists of a n-doped and p-doped layer with the n-doped layer of one CGL being typically located closer to the anode layer.

In one embodiment of the invention, the optoelectronic device is an OLED, which comprises two or more emission layers between anode and cathode. In particular, this so-called tandem OLED comprises three emission layers, wherein one emission layer emits red light, one emission layer emits green light and one emission layer emits blue light, and optionally may comprise further layers such as charge generation layers, blocking or transporting layers between the individual emission layers. In a further embodiment, the emission layers are adjacently stacked. In a further embodiment, the tandem OLED comprises a charge generation layer between each two emission layers. In addition, adjacent emission layers or emission layers separated by a charge generation layer may be merged.

The substrate may be formed by any material or composition of materials. Most frequently, glass slides are used as substrates. Alternatively, thin metal layers (e.g., copper, gold, silver or aluminum films) or plastic films or slides may be used. This may allow a higher degree of flexibility. The anode layer A is mostly composed of materials allowing to obtain an (essentially) transparent film. As at least one of both electrodes should be (essentially) transparent in order to allow light emission from the OLED, either the anode layer A or the cathode layer C is transparent. Preferably, the anode layer A comprises a large content or even consists of transparent conductive oxides (TCOs). Such anode layer A may exemplarily comprise indium tin oxide, aluminum zinc oxide, fluorine doped tin oxide, indium zinc oxide, PbO, SnO, zirconium oxide, molybdenum oxide, vanadium oxide, wolfram oxide, graphite, doped Si, doped Ge, doped GaAs, doped polyaniline, doped polypyrrol and/or doped polythiophene.

Particularly preferably, the anode layer A (essentially) consists of indium tin oxide (ITO) (e.g., $(InO_3)0.9(SnO_2)0.1$). The roughness of the anode layer A caused by the transparent conductive oxides (TCOs) may be compensated by using a hole injection layer (HIL). Further, the HIL may facilitate the injection of quasi charge carriers (i.e., holes) in that the transport of the quasi charge carriers from the TCO to the hole transport layer (HTL) is facilitated. The hole injection layer (HIL) may comprise poly-3,4-ethylendioxy thiophene (PEDOT), polystyrene sulfonate (PSS), $MoO_2$, $V_2O_5$, CuPC or CuI, in particular a mixture of PEDOT and PSS. The hole injection layer (HIL) may also prevent the diffusion of metals from the anode layer A into the hole transport layer (HTL). The HIL may exemplarily comprise PEDOT:PSS (poly-3,4-ethylendioxy thiophene: polystyrene sulfonate), PEDOT (poly-3,4-ethylendioxy thiophene), mMTDATA (4,4',4''-tris[phenyl(m-tolyl)amino]triphenylamine), Spiro-TAD (2,2',7,7'-tetrakis(n,n-diphenylamino)-9,9'-spirobifluorene), DNTPD (N1,N1'-(biphenyl-4,4'-diyl)bis(N1-phenyl-N4,N4-di-m-tolylbenzene-1,4-diamine), NPB (N,N'-nis-(1-naphthalenyl)-N,N'-bisphenyl-(1,1'-biphenyl)-4,4'-diamine), NPNPB (N,N'-diphenyl-N,N'-di-[4-(N,N-diphenyl-amino)phenyl]benzidine), MeO-TPD (N,N,N',N'-tetrakis(4-methoxyphenyl)benzidine), HAT-CN (1,4,5,8,9,11-hexaazatriphenylen-hexacarbonitrile) and/orSpiro-NPD (N,N'-diphenyl-N,N'-bis-(1-naphthyl)-9,9'-spirobifluorene-2,7-diamine).

Adjacent to the anode layer A or hole injection layer (HIL) typically a hole transport layer (HTL) is located. Herein, any hole transport compound may be used. Exemplarily, electron-rich heteroaromatic compounds such as triarylamines and/or carbazoles may be used as hole transport compound. The HTL may decrease the energy barrier between the anode layer A and the light-emitting layer EML. The hole transport layer (HTL) may also be an electron blocking layer (EBL). Preferably, hole transport compounds bear comparably high energy levels of their triplet states T1. Exemplarily the hole transport layer (HTL) may comprise a star-shaped heterocycle such as tris(4-carbazoyl-9-ylphenyl)amine (TCTA), poly-TPD (poly(4-butylphenyl-diphenyl-amine)), [alpha]-NPD (poly(4-butylphenyl-diphenyl-amine)), TAPC (4,4'-cyclohexyliden-bis[N,N-bis(4-methylphenyl)benzenamine]), 2-TNATA (4,4',4''-tris[2-naphthyl(phenyl)amino]triphenylamine), Spiro-TAD, DNTPD, NPB, NPNPB, MeO-TPD, HAT-CN and/or TrisPcz (9,9'-diphenyl-6-(9-phenyl-9H-carbazol-3-yl)-9H,9'H-3,3'-bicarbazole). In addition, the HTL may comprise a p-doped layer, which may be composed of an inorganic or organic dopant in an organic hole-transporting matrix. Transition metal oxides such as vanadium oxide, molybdenum oxide or tungsten oxide may exemplarily be used as inorganic dopant. Tetrafluorotetracyanoquinodimethane ($F_4$-TCNQ), copper-pentafluorobenzoate (Cu(I)pFBz) or transition metal complexes may exemplarily be used as organic dopant.

The EBL may exemplarily comprise mCP (1,3-bis(carbazol-9-yl)benzene), TCTA, 2-TNATA, mCBP (3,3-di(9H-carbazol-9-yl)biphenyl), tris-Pcz, CzSi (9-(4-tert-Butylphenyl)-3,6-bis(triphenylsilyl)-9H-carbazole), and/or DCB (N,N'-dicarbazolyl-1,4-dimethylbenzene).

Adjacent to the hole transport layer (HTL), typically, the light-emitting layer EML is located. The light-emitting layer EML comprises at least one light emitting molecule. Particularly, the EML comprises at least one light emitting molecule according to the invention E. In one embodiment, the light-emitting layer comprises only the organic molecules according to the invention E. Typically, the EML additionally comprises one or more host materials H. Exemplarily, the host material H is selected from CBP (4,4'-Bis-(N-carbazolyl)-biphenyl), mCP, mCBP Sif87 (dibenzo[b,d]thiophen-2-yltriphenylsilane), CzSi, Sif88 (dibenzo[b,d]thiophen-2-yl)diphenylsilane), DPEPO (bis[2-(diphenylphosphino)phenyl] ether oxide), 9-[3-(dibenzofuran-2-yl)phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole, 9-[3,5-bis(2-dibenzothiophenyl)phenyl]-9H-carbazole, T2T (2,4,6-tris(biphenyl-3-yl)-1,3,5-triazine), T3T (2,4,6-tris(triphenyl-3-yl)-1,3,5-triazine) and/or TST (2,4,6-tris(9,9'-spirobifluorene-2-yl)-1,3,5-triazine). The host material H typically should be selected to exhibit first triplet (T1) and first singlet (S1) energy levels, which are energetically higher than the first triplet (T1) and first singlet (S1) energy levels of the organic molecule.

In one embodiment of the invention, the EML comprises a so-called mixed-host system with at least one hole-dominant host and one electron-dominant host. In a particular embodiment, the EML comprises exactly one light emitting molecule according to the invention E and a mixed-host system comprising T2T as electron-dominant host and a host selected from CBP, mCP, mCBP, 9-[3-(dibenzofuran-2-yl)phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole and 9-[3,5-bis(2-dibenzothiophenyl)phenyl]-9H-carbazole as hole-dominant host. In a further embodiment the EML comprises 50-80 % by weight, preferably 60-75 % by weight of a host selected from CBP, mCP, mCBP, 9-[3-(dibenzofuran-2-yl)phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole and 9-[3,5-bis(2-dibenzothiophenyl)phenyl]-9H-carbazole; 10-45 % by weight, preferably 15-30 % by weight of T2T and 5-40 % by weight, preferably 10-30 % by weight of light emitting molecule according to the invention.

Adjacent to the light-emitting layer EML an electron transport layer (ETL) may be located. Herein, any electron transporter may be used. Exemplarily, electron-poor compounds such as, e.g., benzimidazoles, pyridines, triazoles, oxadiazoles (e.g., 1,3,4-oxadiazole), phosphinoxides and sulfone, may be used. An electron transporter may also be a star-shaped heterocycle such as 1,3,5-tri(1-phenyl-1H-benzo[d]imidazol-2-yl)phenyl (TPBi). The ETL may comprise NBphen (2,9-bis(naphthalen-2-yl)-4,7-diphenyl-1,10-phenanthroline), $Alq_3$ (Aluminum-tris(8-hydroxyquinoline)), TSPO1 (diphenyl-4-triphenylsilylphenyl-phosphinoxide), BPyTP2 (2,7-di(2,2'-bipyridin-5-yl)triphenyle), Sif87 (dibenzo[b,d]thiophen-2-yltriphenylsilane), Sif88 (dibenzo[b,d]thiophen-2-yl)diphenylsilane), BmPyPhB (1,3-bis[3,5-di(pyridin-3-yl)phenyl]benzene) and/or BTB (4,4'-bis-[2-(4,6-diphenyl-1,3,5-triazinyl)]-1,1'-biphenyl). Optionally, the ETL may be doped with materials such as Liq. The electron transport layer (ETL) may also block holes or a holeblocking layer (HBL) is introduced.

The HBL may exemplarily comprise BCP (2,9-dimethyl-4,7-diphenyl-1,10-phenanthroline = Bathocuproine), BAlq (bis(8-hydroxy-2-methylquinoline)-(4-phenylphenoxy)aluminum), NBphen (2,9-bis(naphthalen-2-yl)-4,7-diphenyl-1,10-phenanthroline), $Alq_3$ (Aluminum-tris(8-hydroxyquinoline)), TSPO1 (diphenyl-4-triphenylsilylphenyl-phosphinoxide), T2T

(2,4,6-tris(biphenyl-3-yl)-1,3,5-triazine), T3T (2,4,6-tris(triphenyl-3-yl)-1,3,5-triazine), TST (2,4,6-tris(9,9'-spirobifluorene-2-yl)-1,3,5-triazine), and/or TCB/TCP (1,3,5-tris(N-carbazolyl)benzol/ 1,3,5-tris(carbazol)-9-yl) benzene).

Adjacent to the electron transport layer (ETL), a cathode layer C may be located. Exemplarily, the cathode layer C may comprise or may consist of a metal (e.g., Al, Au, Ag, Pt, Cu, Zn, Ni, Fe, Pb, LiF, Ca, Ba, Mg, In, W, or Pd) or a metal alloy. For practical reasons, the cathode layer may also consist of (essentially) intransparent metals such as Mg, Ca or Al. Alternatively or additionally, the cathode layer C may also comprise graphite and or carbon nanotubes (CNTs). Alternatively, the cathode layer C may also consist of nanoscalic silver wires.

An OLED may further, optionally, comprise a protection layer between the electron transport layer (ETL) and the cathode layer C (which may be designated as electron injection layer (EIL)). This layer may comprise lithium fluoride, cesium fluoride, silver, Liq (8-hydroxyquinolinolatolithium), $Li_2O$, $BaF_2$, MgO and/or NaF.

Optionally, also the electron transport layer (ETL) and/or a hole blocking layer (HBL) may comprise one or more host compounds H.

In order to modify the emission spectrum and/or the absorption spectrum of the light-emitting layer EML further, the light-emitting layer EML may further comprise one or more further emitter molecules F. Such an emitter molecule F may be any emitter molecule known in the art. Preferably such an emitter molecule F is a molecule with a structure differing from the structure of the molecules according to the invention E. The emitter molecule F may optionally be a TADF emitter. Alternatively, the emitter molecule F may optionally be a fluorescent and/or phosphorescent emitter molecule which is able to shift the emission spectrum and/or the absorption spectrum of the light-emitting layer EML. Exemplarily, the triplet and/or singlet excitons may be transferred from the emitter molecule according to the invention E to the emitter molecule F before relaxing to the ground state S0 by emitting light typically red-shifted in comparison to the light emitted by emitter molecule E. Optionally, the emitter molecule F may also provoke two-photon effects (i.e., the absorption of two photons of half the energy of the absorption maximum).

Optionally, an optoelectronic device (e.g., an OLED) may exemplarily be an essentially white optoelectronic device. Exemplarily such white optoelectronic device may comprise at least one (deep) blue emitter molecule and one or more emitter molecules emitting green and/or red light. Then, there may also optionally be energy transmittance between two or more molecules as described above.

As used herein, if not defined more specifically in the particular context, the designation of the colors of emitted and/or absorbed light is as follows:

| | |
|---|---|
| violet: | wavelength range of >380-420 nm; |
| deep blue: | wavelength range of >420-480 nm; |
| sky blue: | wavelength range of >480-500 nm; |
| green: | wavelength range of >500-560 nm; |
| yellow: | wavelength range of >560-580 nm; |
| orange: | wavelength range of >580-620 nm; |
| red: | wavelength range of >620-800 nm |

With respect to emitter molecules, such colors refer to the emission maximum. Therefore, exemplarily, a deep blue emitter has an emission maximum in the range of from >420 to 480 nm, a sky blue emitter has an emission maximum in the range of from >480 to 500 nm, a green emitter has an emission maximum in a range of from >500 to 560 nm, a red emitter has an emission maximum in a range of from >620 to 800 nm.

A deep blue emitter may preferably have an emission maximum of below 480 nm, more preferably below 470 nm, even more preferably below 465 nm or even below 460 nm. It will typically be above 420 nm, preferably above 430 nm, more preferably above 440 nm or even above 450 nm.

Accordingly, a further aspect of the present invention relates to an OLED, which exhibits an external quantum efficiency at 1000 cd/m$^2$ of more than 8 %, more preferably of more than 10 %, more preferably of more than 13 %, even more preferably of more than 15 % or even more than 20 % and/or exhibits an emission maximum between 420 nm and 500 nm, preferably between 430 nm and 490 nm, more preferably between 440 nm and 480 nm, even more preferably between 450 nm and 470 nm and/or exhibits a LT80 value at 500 cd/m$^2$ of more than 100 h, preferably more than 200 h, more preferably more than 400 h, even more preferably more than 750 h or even more than 1000 h. Accordingly, a further aspect of the present invention relates to an OLED, whose emission exhibits a CIEy color coordinate of less than 0.45, preferably less than 0.30, more preferably less than 0.20 or even more preferably less than 0.15 or even less than 0.10.

A further aspect of the present invention relates to an OLED, which emits light at a distinct color point. According to the present invention, the OLED emits light with a narrow emission band (small full width at half maximum (FWHM)). In one aspect, the OLED according to the invention emits light with a FWHM of the main emission peak of less than

0.50 eV, preferably less than 0.48 eV, more preferably less than 0.45 eV, even more preferably less than 0.43 eV or even less than 0.40 eV.

A further aspect of the present invention relates to an OLED, which emits light with CIEx and CIEy color coordinates close to the CIEx (= 0.131) and CIEy (= 0.046) color coordinates of the primary color blue (CIEx = 0.131 and CIEy = 0.046) as defined by ITU-R Recommendation BT.2020 (Rec. 2020) and thus is suited for the use in Ultra High Definition (UHD) displays, e.g. UHD-TVs. Accordingly, a further aspect of the present invention relates to an OLED, whose emission exhibits a CIEx color coordinate of between 0.02 and 0.30, preferably between 0.03 and 0.25, more preferably between 0.05 and 0.20 or even more preferably between 0.08 and 0.18 or even between 0.10 and 0.15 and/ or a a CIEy color coordinate of between 0.00 and 0.45, preferably between 0.01 and 0.30, more preferably between 0.02 and 0.20 or even more preferably between 0.03 and 0.15 or even between 0.04 and 0.10.

In a further aspect, the invention relates to a method for producing an optoelectronic component. In this case an organic molecule of the invention is used.

The optoelectronic device, in particular the OLED according to the present invention can be fabricated by any means of vapor deposition and/ or liquid processing. Accordingly, at least one layer is

- prepared by means of a sublimation process,
- prepared by means of an organic vapor phase deposition process,
- prepared by means of a carrier gas sublimation process,
- solution processed or printed.

The methods used to fabricate the optoelectronic device, in particular the OLED according to the present invention are known in the art. The different layers are individually and successively deposited on a suitable substrate by means of subsequent deposition processes. The individual layers may be deposited using the same or differing deposition methods.

Vapor deposition processes exemplarily comprise thermal (co)evaporation, chemical vapor deposition and physical vapor deposition. For active matrix OLED display, an AMOLED backplane is used as substrate. The individual layer may be processed from solutions or dispersions employing adequate solvents. Solution deposition process exemplarily comprise spin coating, dip coating and jet printing. Liquid processing may optionally be carried out in an inert atmosphere (e.g., in a nitrogen atmosphere) and the solvent may optionally be completely or partially removed by means known in the state of the art.

**Examples**

General synthesis scheme I

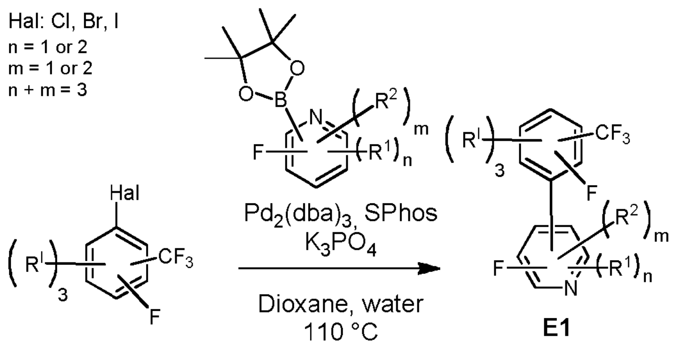


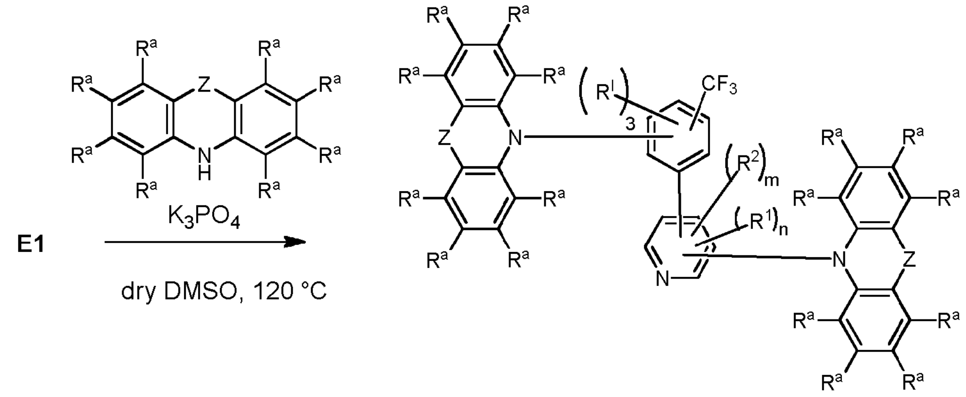


*General procedure for synthesis **AAV1:***

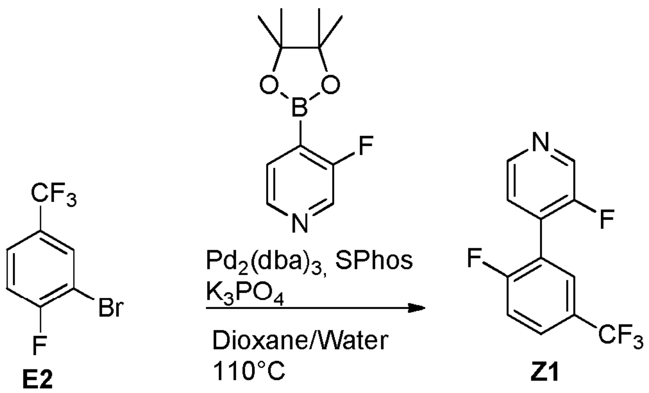


3-bromo-4-fluorobenzotrifluoride **E2** (1.0 equivalent), 3-fluoropyridine-4-boronic acid pinacol ester (1.1 equivalents), $Pd_2(dba)_3$ (0.04 equivalents), S-Phos (0.16 equivalents) and potassium phosphate (2.50 equivalents) are stirred under nitrogen atmosphere in a dioxane/water mixture (ratio of 10:1) at 110 °C for 20 h. Active carbon and Celite® (kieselguhr) are added to the reaction mixture and stirred at 90 °C for 10 min, then the mixture is hot filtered and the organic phase is concentrated under reduced pressure. The residue is purified by chromatography and the product **Z1** is obtained as a solid.

*General procedure for synthesis **AAV2:***

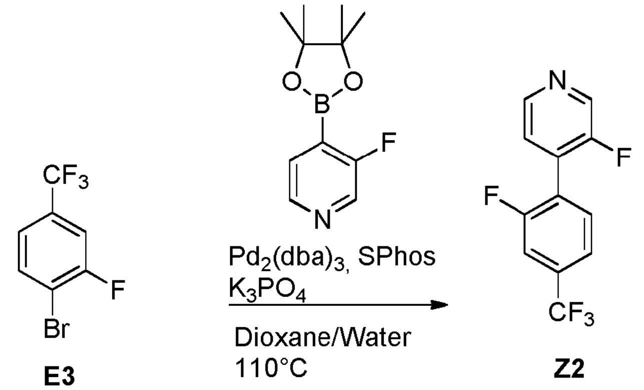


E3 Z2

The synthesis of **Z2** is carried out according to *AAV1,* wherein 4-bromo-3-fluorobenzotrifluoride **E3** reacts with 3-fluoropyridine-4-boronic acid pinacol ester.

*General procedure for synthesis* ***AAV3*****:**

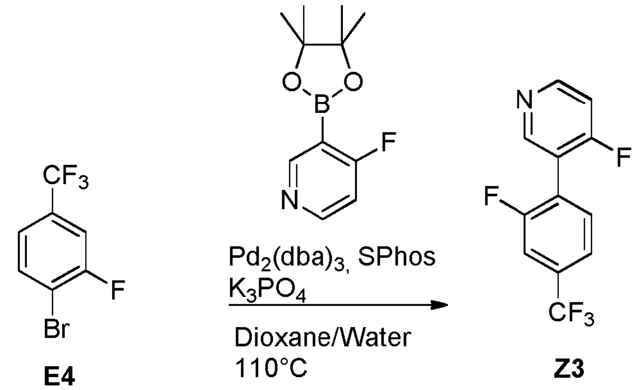


E4 Z3

The synthesis of **Z3** is carried out according to *AAV1,* wherein 4-bromo-3-fluorobenzotrifluoride **E4** reacts with 4-fluoropyridine-3-boronic acid pinacol ester.

*General procedure for synthesis* ***AAV4*****:**

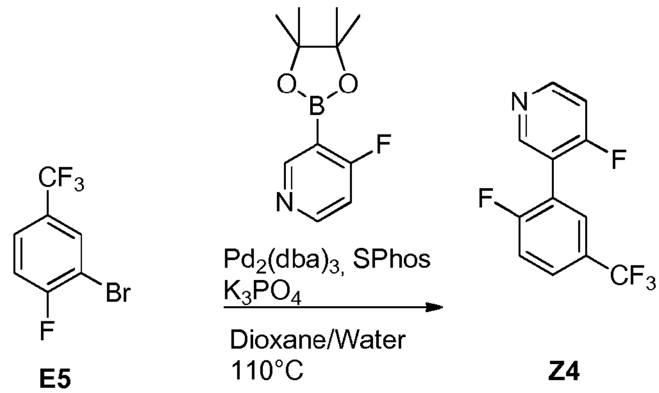


E5 Z4

The synthesis of **Z4** is carried out according to *AAV1,* wherein 3-bromo-3-fluorobenzotrifluoride **E5** reacts with 4-fluoropyridine-3-boronic acid pinacol ester.

*General procedure for synthesis **AAV5**:*

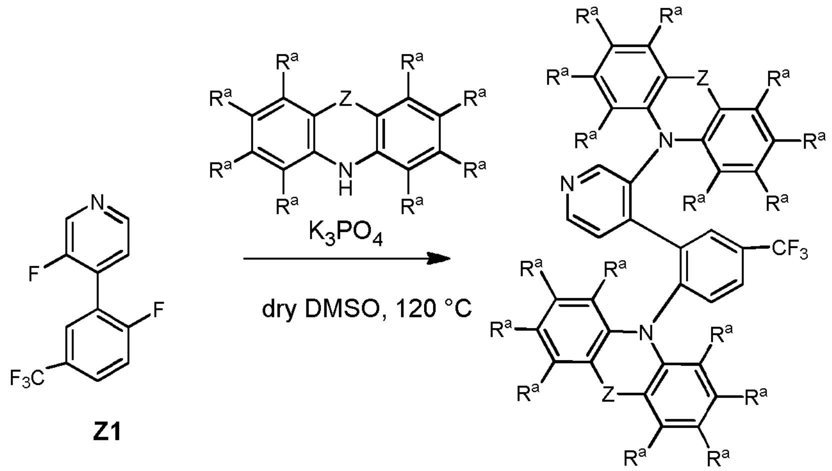


**Z1**

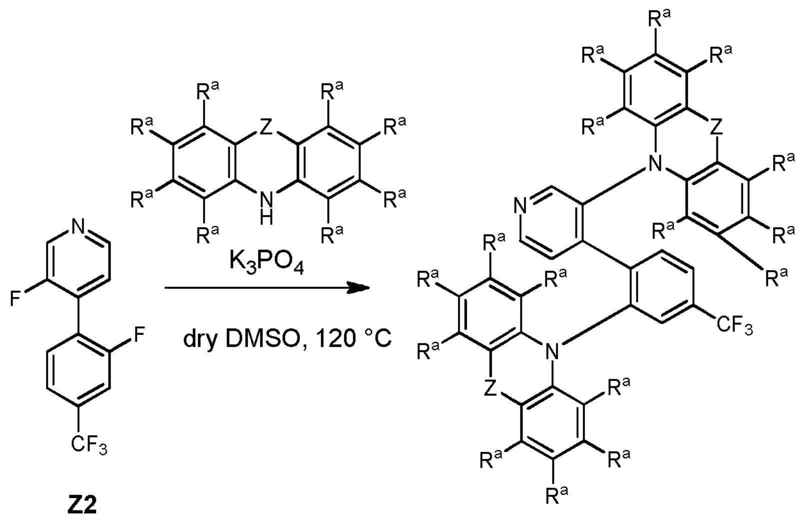


**Z2**

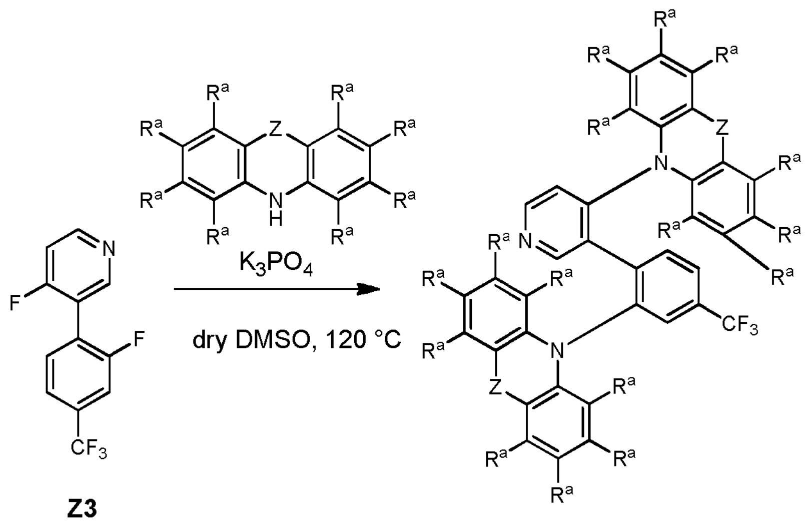


Z3

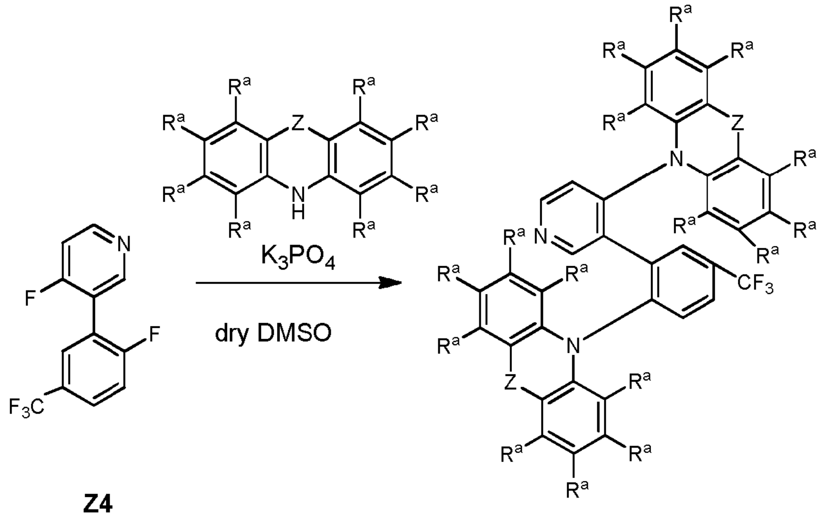


Z4

**Z1**, **Z2**, **Z3** or **Z4** (1 equivalent each), the corresponding donor molecule D-H (2.20 equivalents) and tribasic potassium phosphate (5.0 equivalents) are suspended under nitrogen atmosphere in dry DMSO and stirred at 110 °C for 20 h. The reaction mixture is poured into a saturated sodium chloride solution and the resulting precipitate is filtered. Subsequently the residue is dissolved in dichloromethane and washed with saturated sodium chloride solution, dried over $MgSO_4$ and the solvent evaporated under reduced pressure. The crude product is purified by recrystallization in ethanol or by chromatography.

In particular, the donor molecule D-H is a 3,6-substituted carbazole (e.g., 3,6-dimethylcarbazole, 3,6-diphenylcarbazole, 3,6-di-tert-butylcarbazole), a 2,7-substituted carbazole (e.g., 2,7-dimethylcarbazole, 2,7-diphenylcarbazole, 2,7-di-tert-butylcarbazole), a 1,8-substituted carbazole (e.g., 1,8-dimethylcarbazole, 1,8-diphenylcarbazole, 1,8-di-tert-butylcarbazole), a 1-substituted carbazole (e.g., 1-methylcarbazole, 1-phenylcarbazole, 1-tert-butylcarbazole), a 2-substituted carbazole (e.g., 2-methylcarbazole, 2-phenylcarbazole, 2-tert-butylcarbazole), or a 3-substituted carbazole (e.g., 3-methylcarbazole, 3-phenylcarbazole, 3-tert-butylcarbazole).

Exemplarily a halogen-substituted carbazole, particularly 3-bromocarbazole, can be used as D-H.

In a subsequent reaction a boronic acid ester functional group or boronic acid functional group may be exemplarily introduced at the position of the one or more halogen substituents, which was introduced via D-H, to yield the corresponding carbazol-3-ylboronic acid ester or carbazol-3-ylboronic acid, e.g., via the reaction with bis(pinacolato)diboron (CAS No. 73183-34-3). Subsequently, one or more substituents $R^a$ may be introduced in place of the boronic acid ester group or the boronic acid group via a coupling reaction with the corresponding halogenated reactant $R^a$-Hal, preferably $R^a$-Cl and $R^a$-Br.

Alternatively, one or more substituents $R^a$ may be introduced at the position of the one or more halogen substituents, which was introduced via D-H, via the reaction with a boronic acid of the substituent $R^a$ [$R^a$-$B(OH)_2$] or a corresponding boronic acid ester.

*Cyclic voltammetry*

Cyclic voltammograms are measured from solutions having concentration of $10^{-3}$ mol/L of the organic molecules in dichloromethane or a suitable solvent and a suitable supporting electrolyte (e.g. 0.1 mol/L of tetrabutylammonium hexafluorophosphate). The measurements are conducted at room temperature under nitrogen atmosphere with a three-electrode assembly (Working and counter electrodes: Pt wire, reference electrode: Pt wire) and calibrated using $FeCp_2/FeCp_2^+$ as internal standard. The HOMO data was corrected using ferrocene as internal standard against a saturated calomel electrode (SCE).

*Density functional theory calculation*

Molecular structures are optimized employing the BP86 functional and the resolution of identity approach (RI). Excitation energies are calculated using the (BP86) optimized structures employing Time-Dependent DFT (TD-DFT) methods. Orbital and excited state energies are calculated with the B3LYP functional. Def2-SVP basis sets (and a m4-grid for numerical integration are used. The Turbomole program package is used for all calculations.

*Photophysical measurements*

Sample pretreatment: Spin-coating
Apparatus: Spin150, SPS euro.
The sample concentration is 10 mg/ml, dissolved in a suitable solvent.
Program: 1) 3 s at 400 U/min; 20 s at 1000 U/min at 1000 Upm/s. 3) 10 s at 4000 U/min at 1000 Upm/s. After coating, the films are tried at 70 °C for 1 min.

Photoluminescence spectroscopy and Time-Correlated Single-Photon Counting (TCSPC)

Steady-state emission spectroscopy is measured by a Horiba Scientific, Modell FluoroMax-4 equipped with a 150 W Xenon-Arc lamp, excitation- and emissions monochromators and a Hamamatsu R928 photomultiplier and a time-correlated single-photon counting option. Emissions and excitation spectra are corrected using standard correction fits.

Excited state lifetimes are determined employing the same system using the TCSPC method with FM-2013 equipment and a Horiba Yvon TCSPC hub.
Excitation sources:

NanoLED 370 (wavelength: 371 nm, puls duration: 1,1 ns)
NanoLED 290 (wavelength: 294 nm, puls duration: <1 ns)
SpectraLED 310 (wavelength: 314 nm)
SpectraLED 355 (wavelength: 355 nm).

Data analysis (exponential fit) is done using the software suite DataStation and DAS6 analysis software. The fit is specified using the chi-squared-test.

Photoluminescence quantum yield measurements

For photoluminescence quantum yield (PLQY) measurements an *Absolute PL Quantum Yield Measurement C9920-03G* system (*Hamamatsu Photonics*) is used. Quantum yields and CIE coordinates are determined using the software U6039-05 version 3.6.0.
Emission maxima are given in nm, quantum yields $\phi$ in % and CIE coordinates as x,y values. PLQY is determined using the following protocol:

1) Quality assurance: Anthracene in ethanol (known concentration) is used as reference
2) Excitation wavelength: the absorption maximum of the organic molecule is determined and the molecule is excited using this wavelength
3) Measurement
Quantum yields are measured for sample of solutions or films under nitrogen atmosphere. The yield is calculated using the equation:

$$\Phi_{PL} = \frac{n_{photon}, emited}{n_{photon}, absorbed} = \frac{\int \frac{\lambda}{hc}\left[Int_{emitted}^{sample}(\lambda) - Int_{absorbed}^{sample}(\lambda)\right]d\lambda}{\int \frac{\lambda}{hc}\left[Int_{emitted}^{reference}(\lambda) - Int_{absorbed}^{reference}(\lambda)\right]d\lambda}$$

wherein $n_{photon}$ denotes the photon count and Int. the intensity.

HPLC-MS

HPLC-MS analysis is performed on an HPLC by Agilent (1100 series) with MS-detector (Thermo LTQ XL). Exemplary a typical HPLC method is as follows: a reverse phase column 4,6mm x 150mm, particle size 3,5 µm from Agilent *(ZORBAX Eclipse Plus 95Å C18,* 4.6 x *150 mm, 3.5 µm HPLC column)* is used in the HPLC. The HPLC-MS measurements are performed at room temperature (rt) following gradients

| Flow rate [ml/min] | time [min] | A[%] | B[%] | C[%] |
|---|---|---|---|---|
| 2.5 | 0 | 40 | 50 | 10 |
| 2.5 | 5 | 40 | 50 | 10 |
| 2.5 | 25 | 10 | 20 | 70 |
| 2.5 | 35 | 10 | 20 | 70 |
| 2.5 | 35.01 | 40 | 50 | 10 |
| 2.5 | 40.01 | 40 | 50 | 10 |
| 2.5 | 41.01 | 40 | 50 | 10 |

using the following solvent mixtures:

| | | |
|---|---|---|
| solvent A: | $H_2O$ (90%) | MeCN (10%) |
| solvent B: | $H_2O$ (10%) | MeCN (90%) |
| solvent C: | THF (50%) | MeCN (50%) |

An injection volume of 5 µL from a solution with a concentration of 0.5 mg/mL of the analyte is taken for the measurements.

Ionization of the probe is performed using an APCI (atmospheric pressure chemical ionization) source either in positive (APCI +) or negative (APCI -) ionization mode.

**Production and characterization of optoelectronic devices**

Optoelectronic devise, such as OLED devices, comprising organic molecules according to the invention can be produced via vacuum-deposition methods. If a layer contains more than one compound, the weight-percentage of one or more compounds is given in %. The total weight-percentage values amount to 100 %, thus if a value is not given, the fraction of this compound equals to the difference between the given values and 100 %.

The not fully optimized OLEDs are characterized using standard methods and measuring electroluminescence spectra, the external quantum efficiency (in %) in dependency on the intensity, calculated using the light detected by the photodiode, and the current. The OLED device lifetime is extracted from the change of the luminance during operation at constant current density. The LT50 value corresponds to the time, where the measured luminance decreased to 50 % of the initial luminance, analogously LT80 corresponds to the time point, at which the measured luminance decreased to 80 % of the initial luminance, LT 95 to the time point, at which the measured luminance decreased to 95 % of the initial luminance etc.

Accelerated lifetime measurements are performed (e.g. applying increased current densities). Exemplarily LT80 values at 500 cd/$m^2$ are determined using the following equation:

$$\mathrm{LT80}\left(500\frac{cd^2}{m^2}\right) = \mathrm{LT80}(L_0)\left(\frac{L_0}{500\frac{cd^2}{m^2}}\right)^{1.6}$$

wherein $L_0$ denotes the initial luminance at the applied current density.
The values correspond to the average of several pixels (typically two to eight), the standard deviation between these pixels is given.

**Example 1**

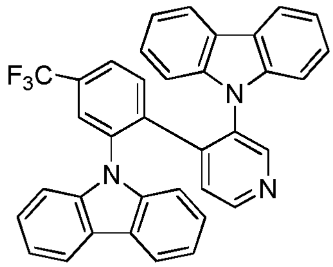

Example 1 was synthesized according to ***AAV2*** and ***AAV5*** (47 % yield).
MS (HPLC-MS):

| Molecular Formula | m/z calculated | m/z found |
|---|---|---|
| $C_{36}H_{22}F_3N_3$ | 553.59 | 553.24 |

Figure 1 depicts the emission spectrum of example 1 (10 % by weight in PMMA). The emission maximum ($\lambda_{max}$) is at 459 nm. The photoluminescence quantum yield (PLQY) is 61 %, the full width at half maximum (FWHM) is 0.43 eV and the emission lifetime is 16 µs. The resulting $CIE_x$ coordinate is determined at 0.16 and the $CIE_y$ coordinate at 0.17. The energy of the highest occupied molecular orbital $E^{HOMO}$ is determined at -5.9 eV.

**Example 2**

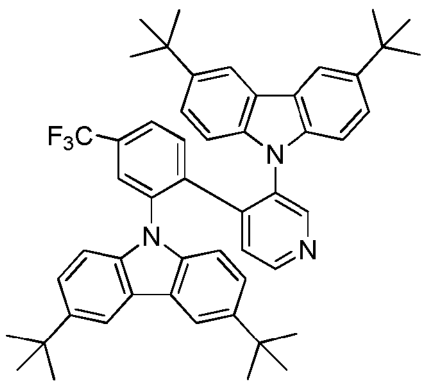

Example 2 was synthesized according to ***AAV2*** and ***AAV5*** (73 % yield).
MS (HPLC-MS):

| Molecular Formula | m/z calculated | m/z found |
|---|---|---|
| $C_{60}H_{38}N_4$ | 778.02 | 777.51 |

Figure 2 depicts the emission spectrum of example 2 (10 % by weight in PMMA). The emission maximum ($\lambda_{max}$) is at 472 nm. The photoluminescence quantum yield (PLQY) is 75 %, the full width at half maximum (FWHM) is 0.43 eV and the emission lifetime is 11 μs. The resulting $CIE_x$ coordinate is determined at 0.17 and the $CIE_y$ coordinate at 0.24. The energy of the highest occupied molecular orbital $E^{HOMO}$ is determined at -5.8 eV.

**Example 3**

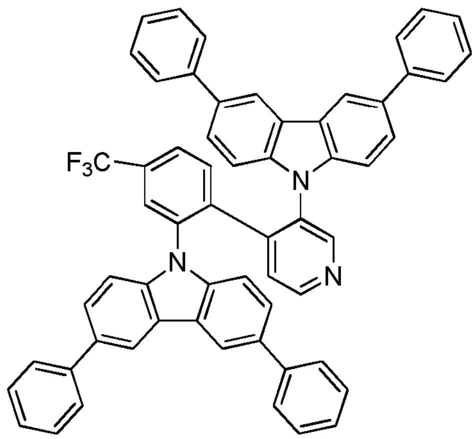

Example **3** was synthesized according to ***AAV2*** and ***AAV5*** (59 % yield).
MS (HPLC-MS):

| Molecular Formula | m/z calculated | m/z found |
|---|---|---|
| $C_{36}H_{22}N_4$ | 857.98 | 857.40 |

Figure 3 depicts the emission spectrum of example 3 (10 % by weight in PMMA). The emission maximum ($\lambda_{max}$) is at 472 nm. The photoluminescence quantum yield (PLQY) is 67 %, the full width at half maximum (FWHM) is 0.43 eV and the emission lifetime is 18 μs. The resulting $CIE_x$ coordinate is determined at 0.17 and the $CIE_y$ coordinate at 0.24. The energy of the highest occupied molecular orbital $E^{HOMO}$ is determined at -5.8 eV.

**Additional Examples of Organic Molecules of the Invention**

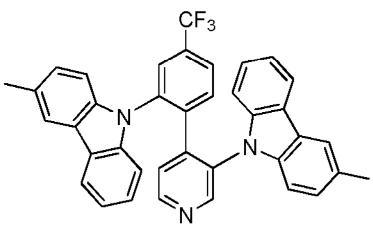

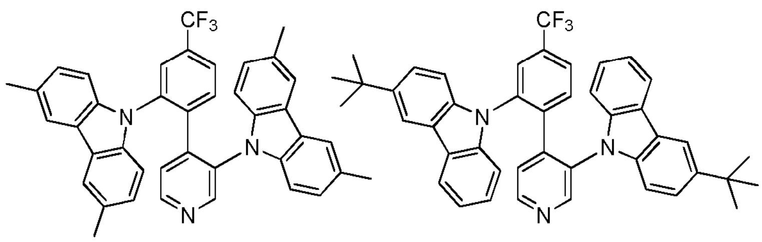

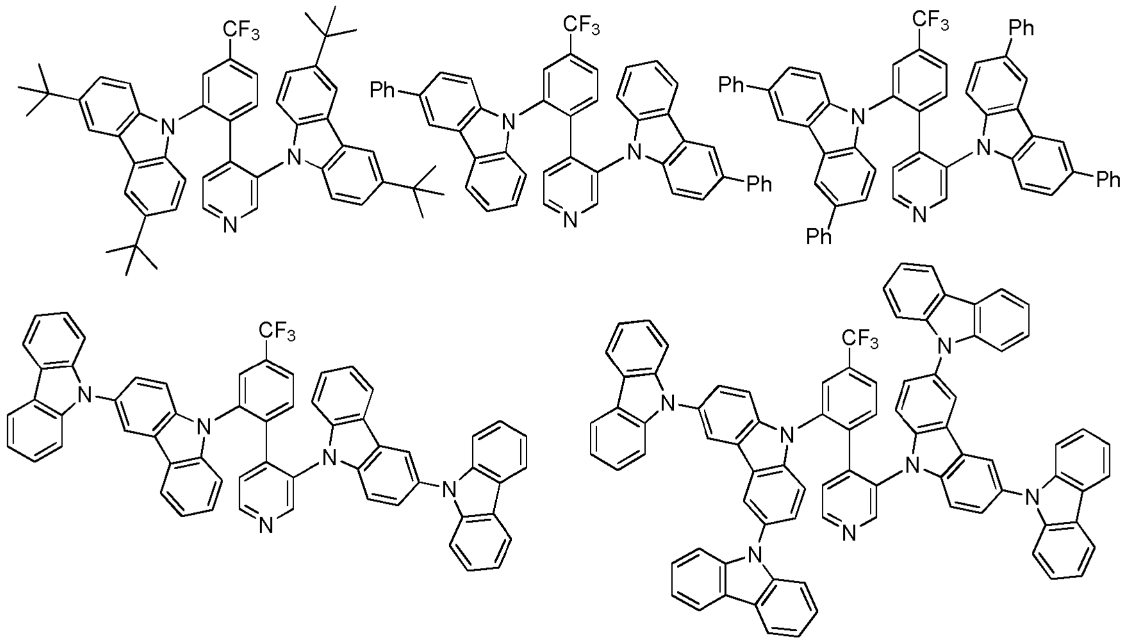

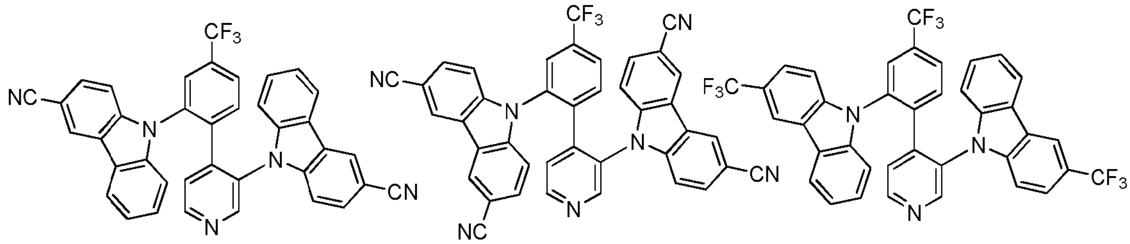

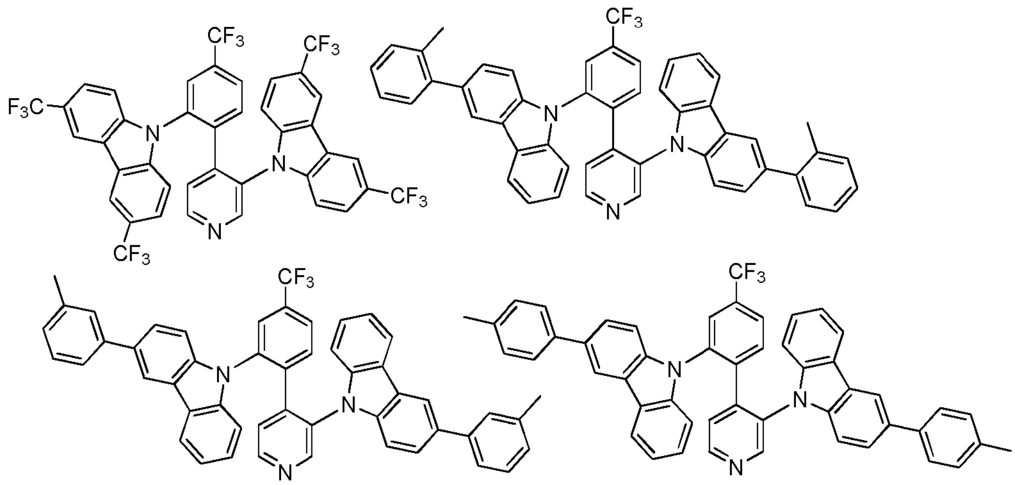

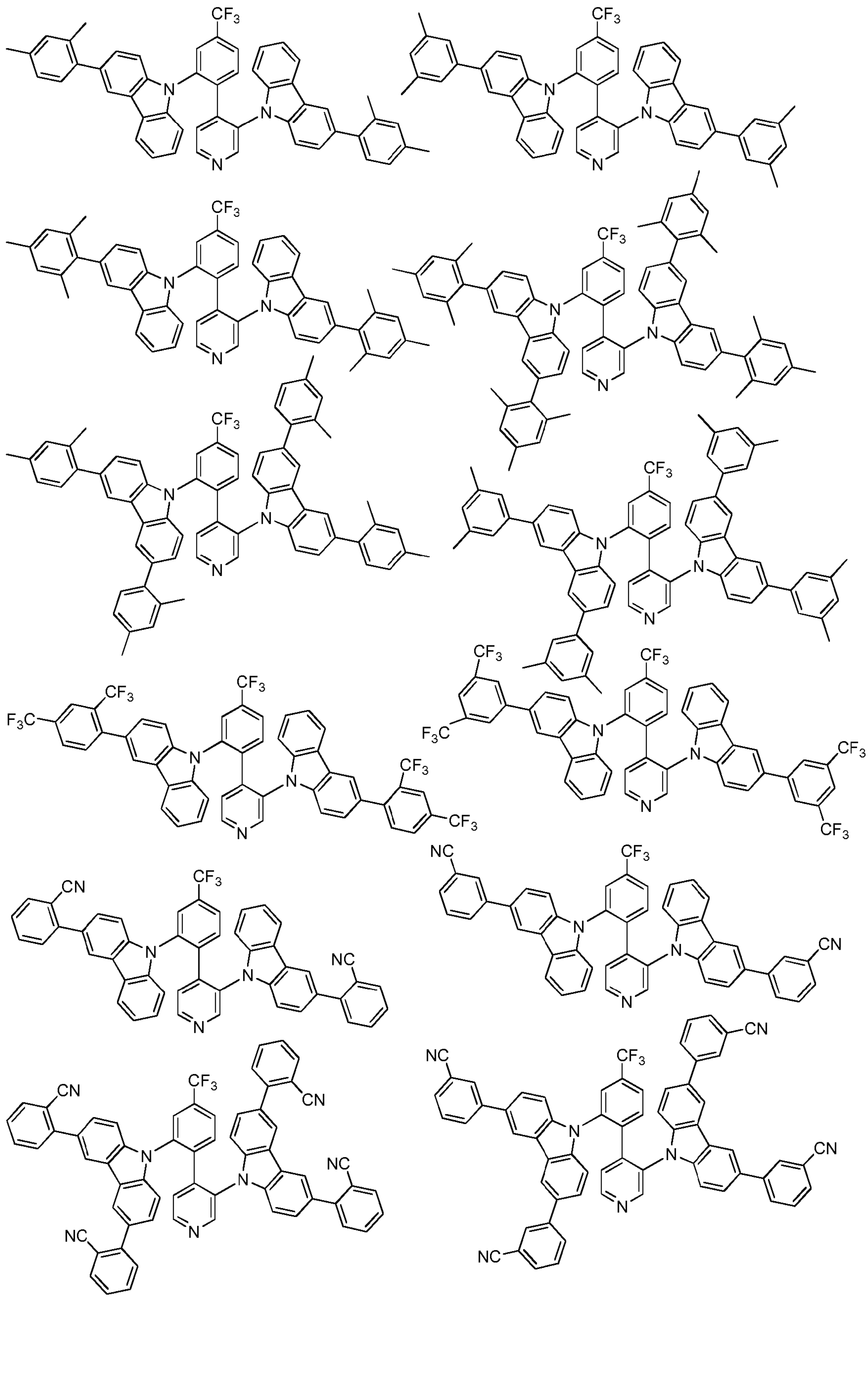

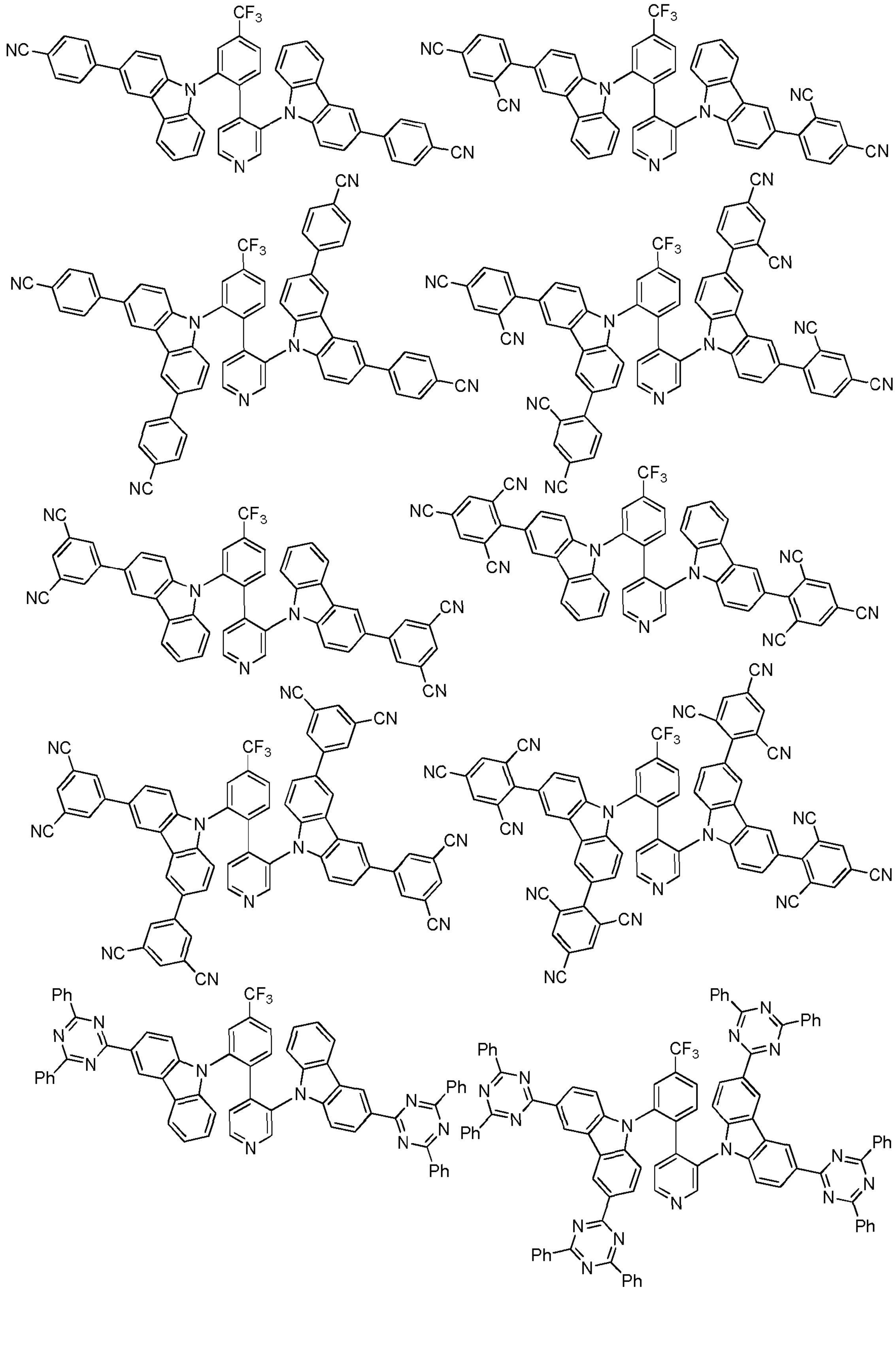

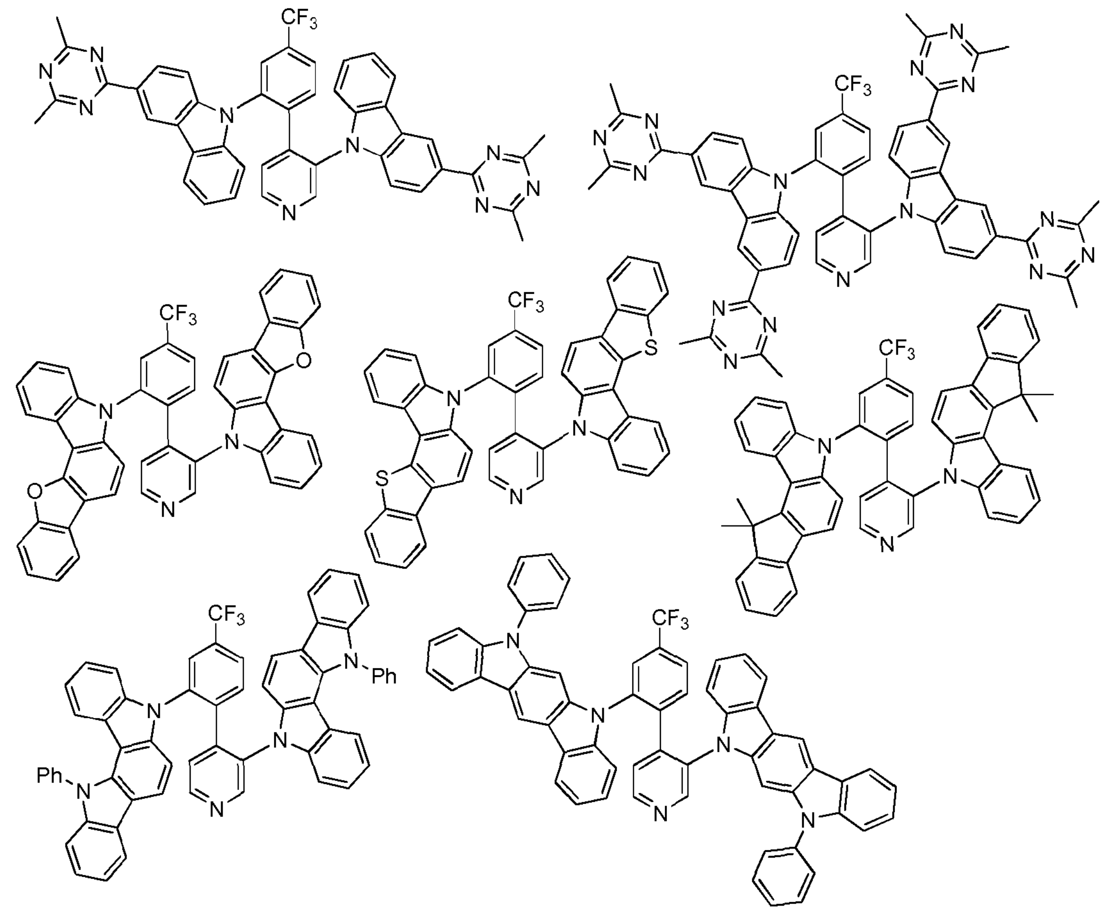

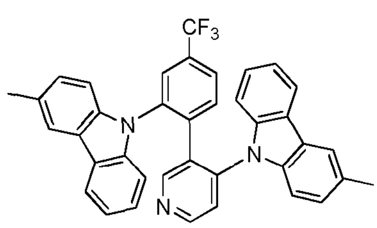

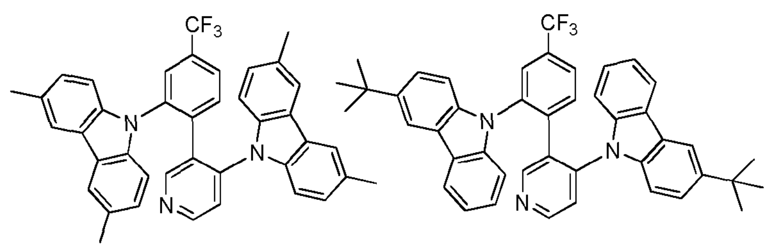

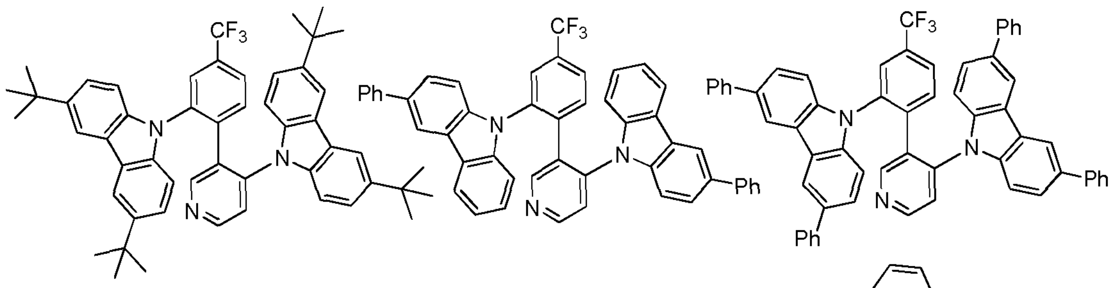

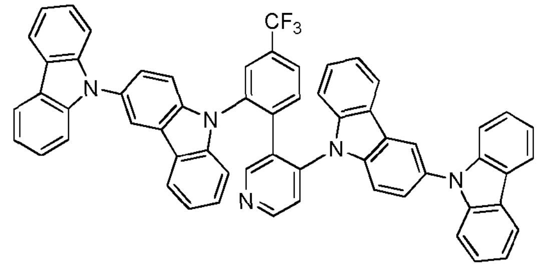

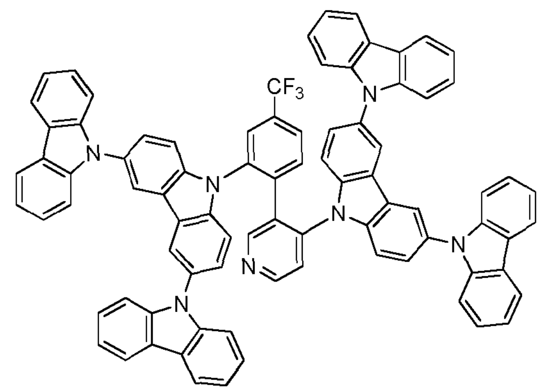

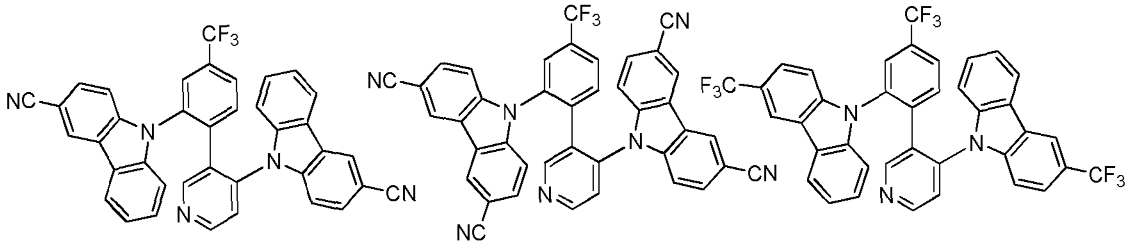

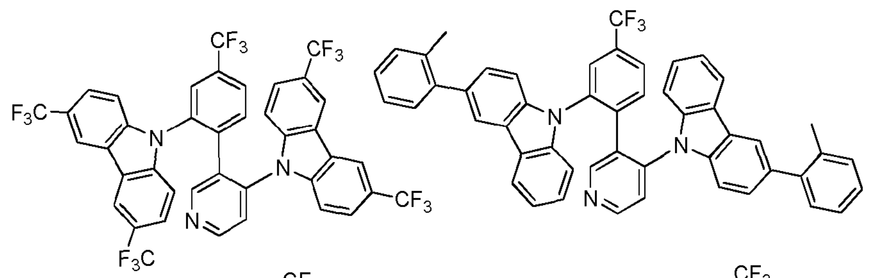

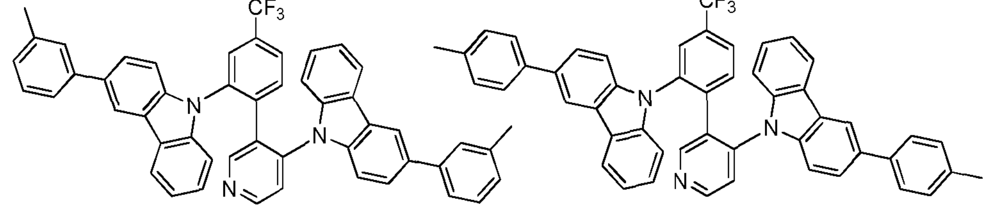

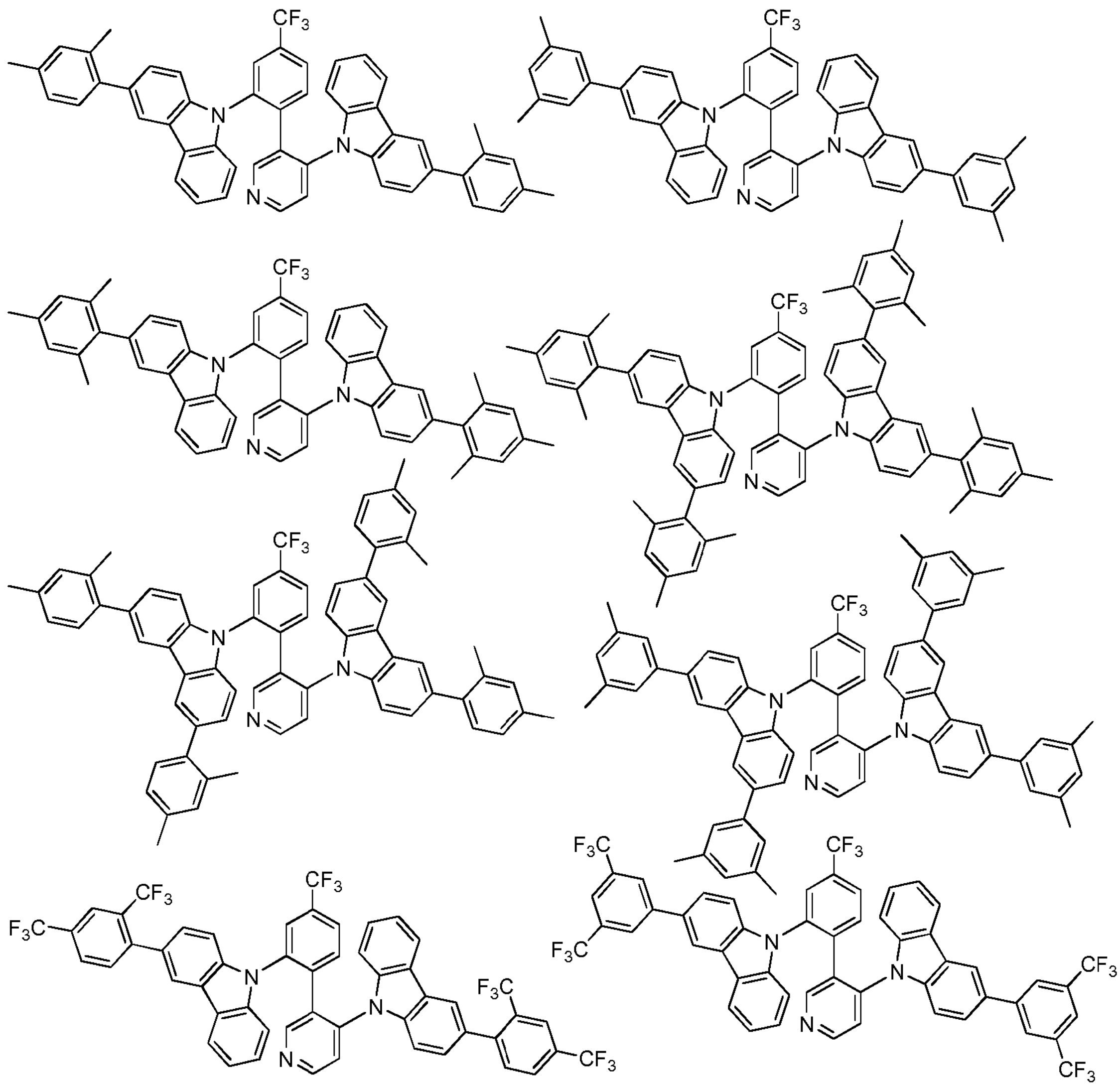

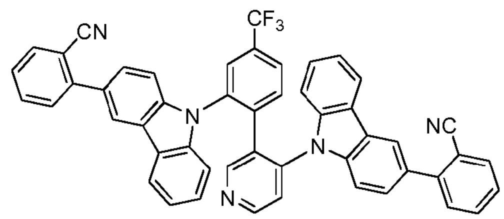

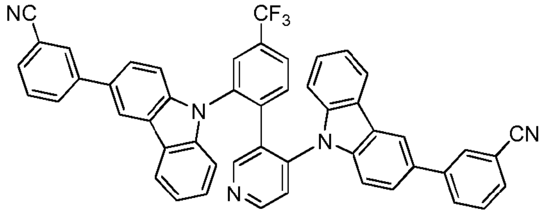

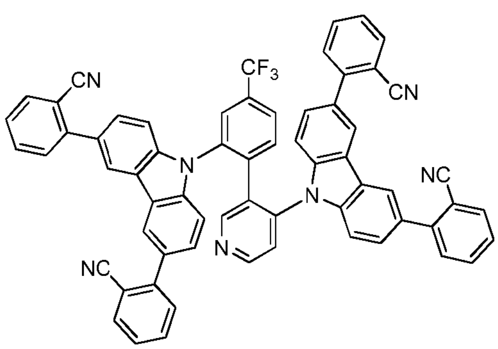

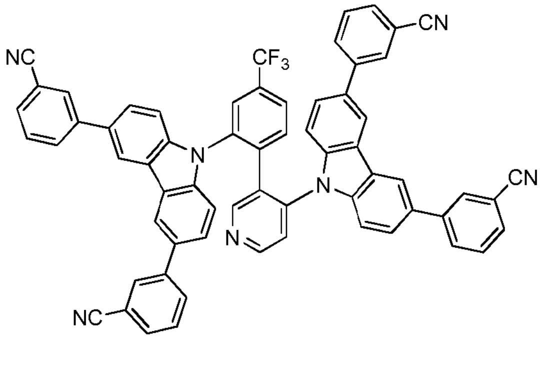

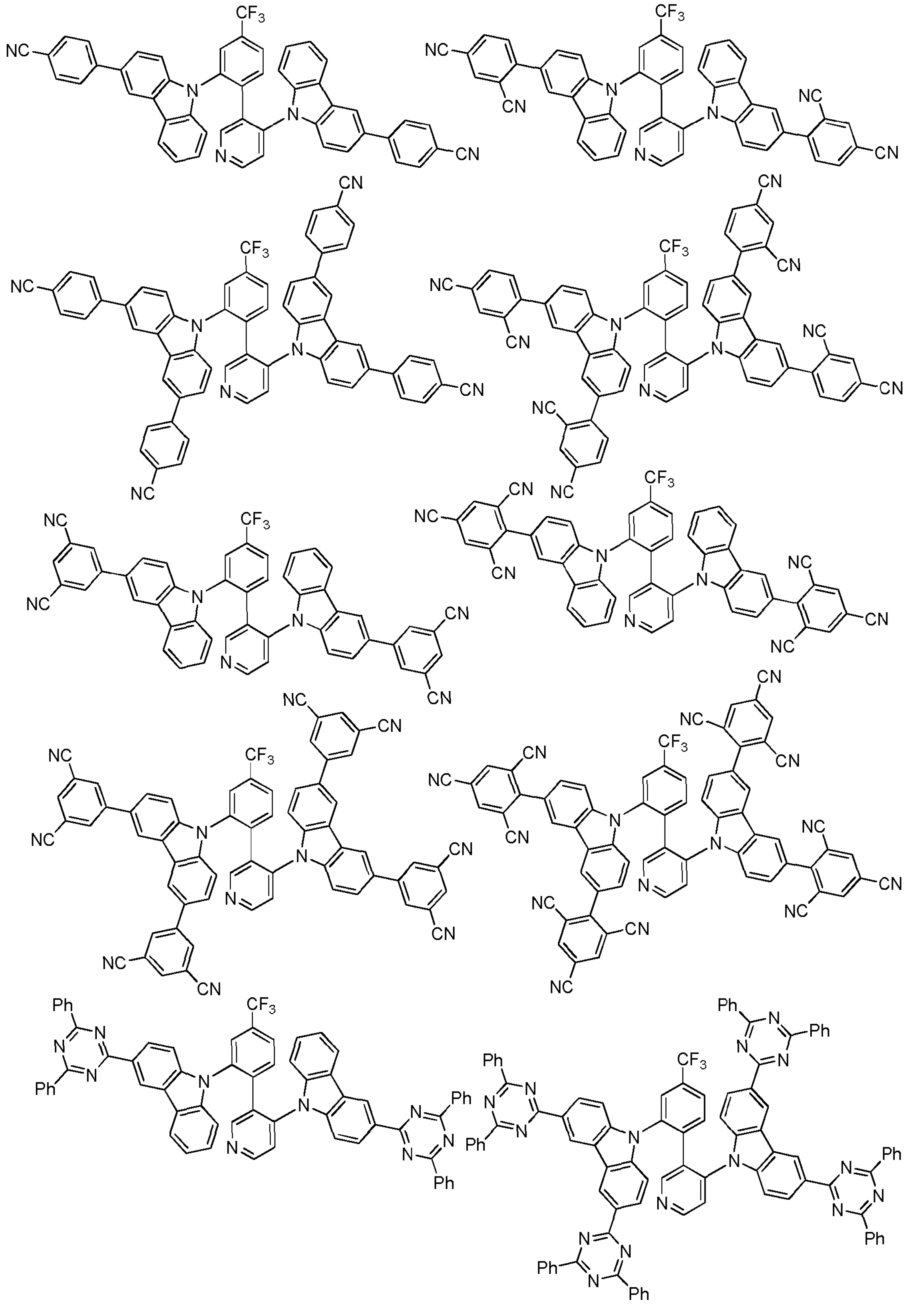

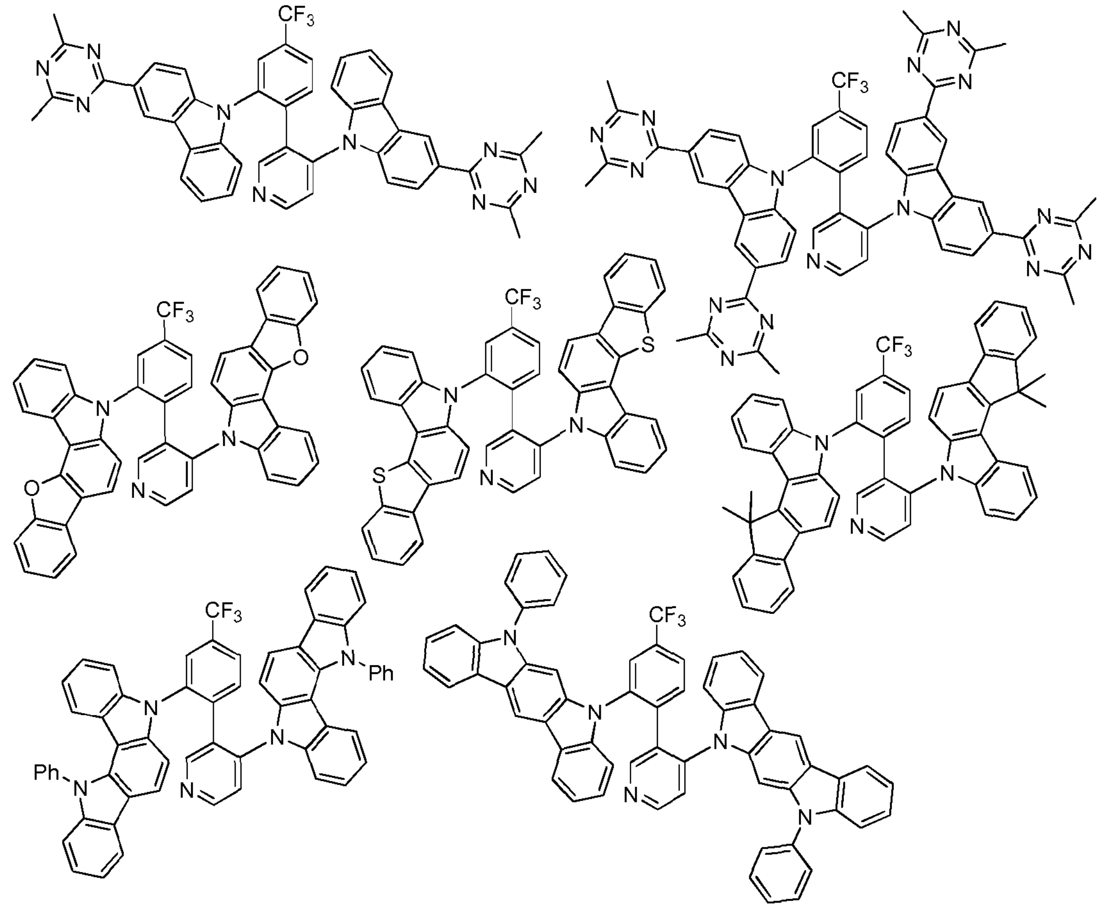

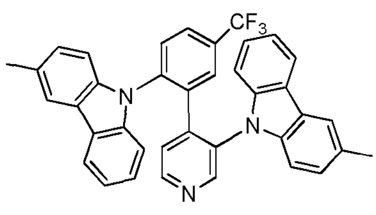

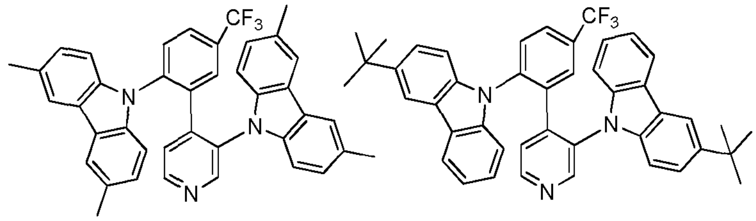

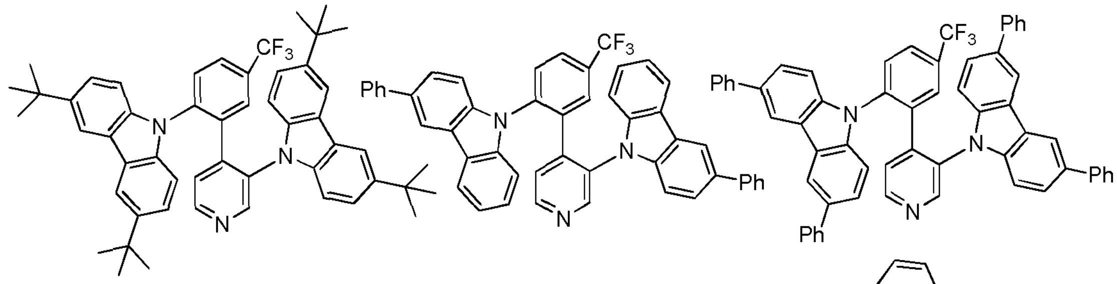

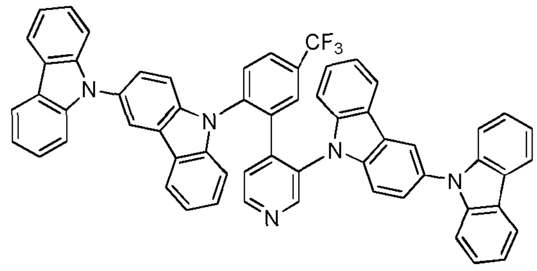

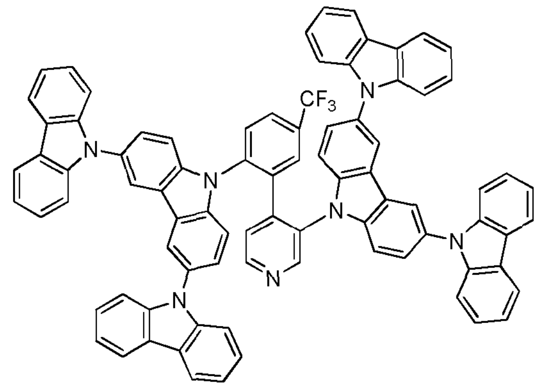

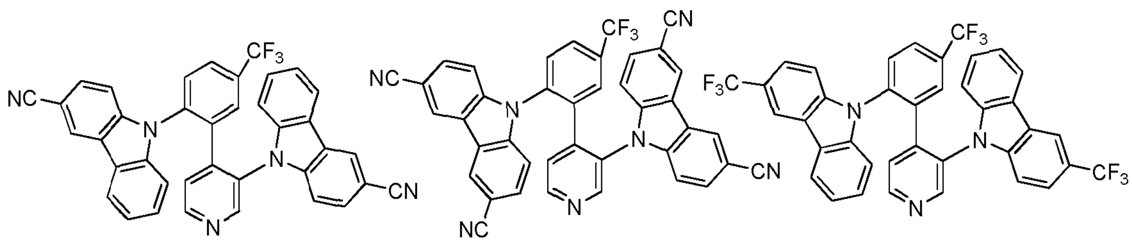

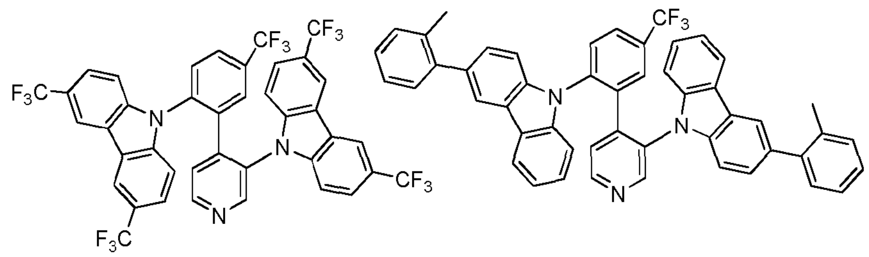

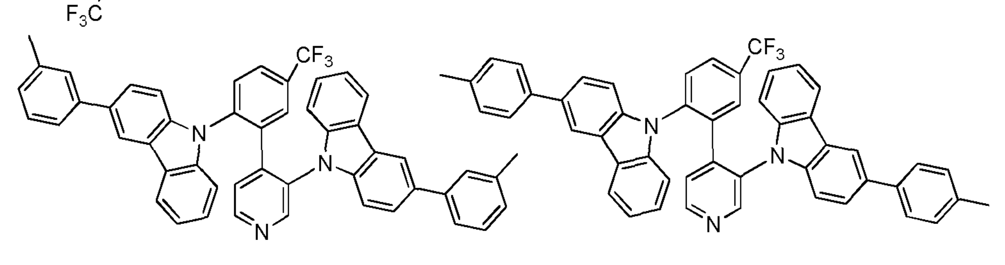

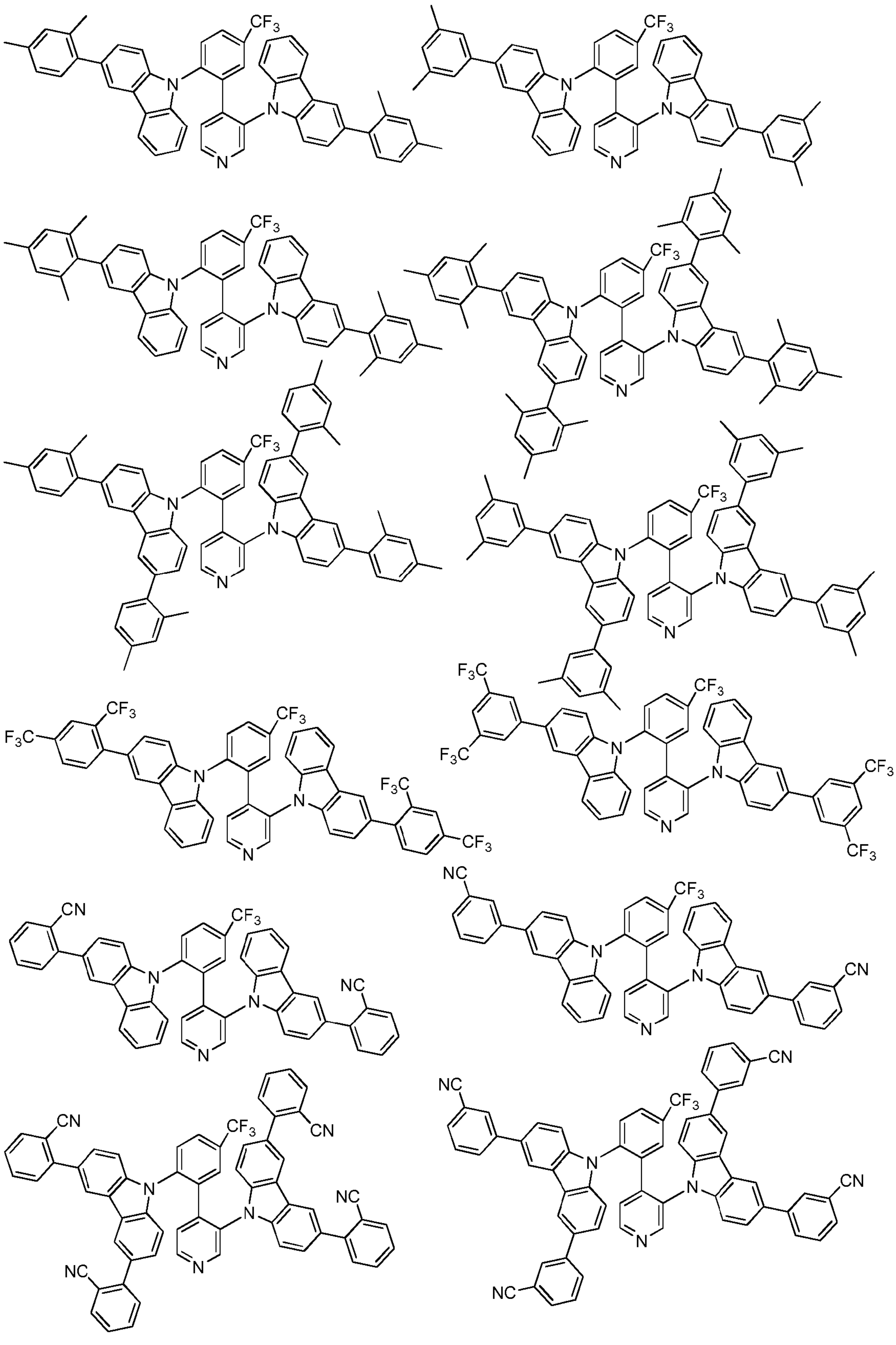

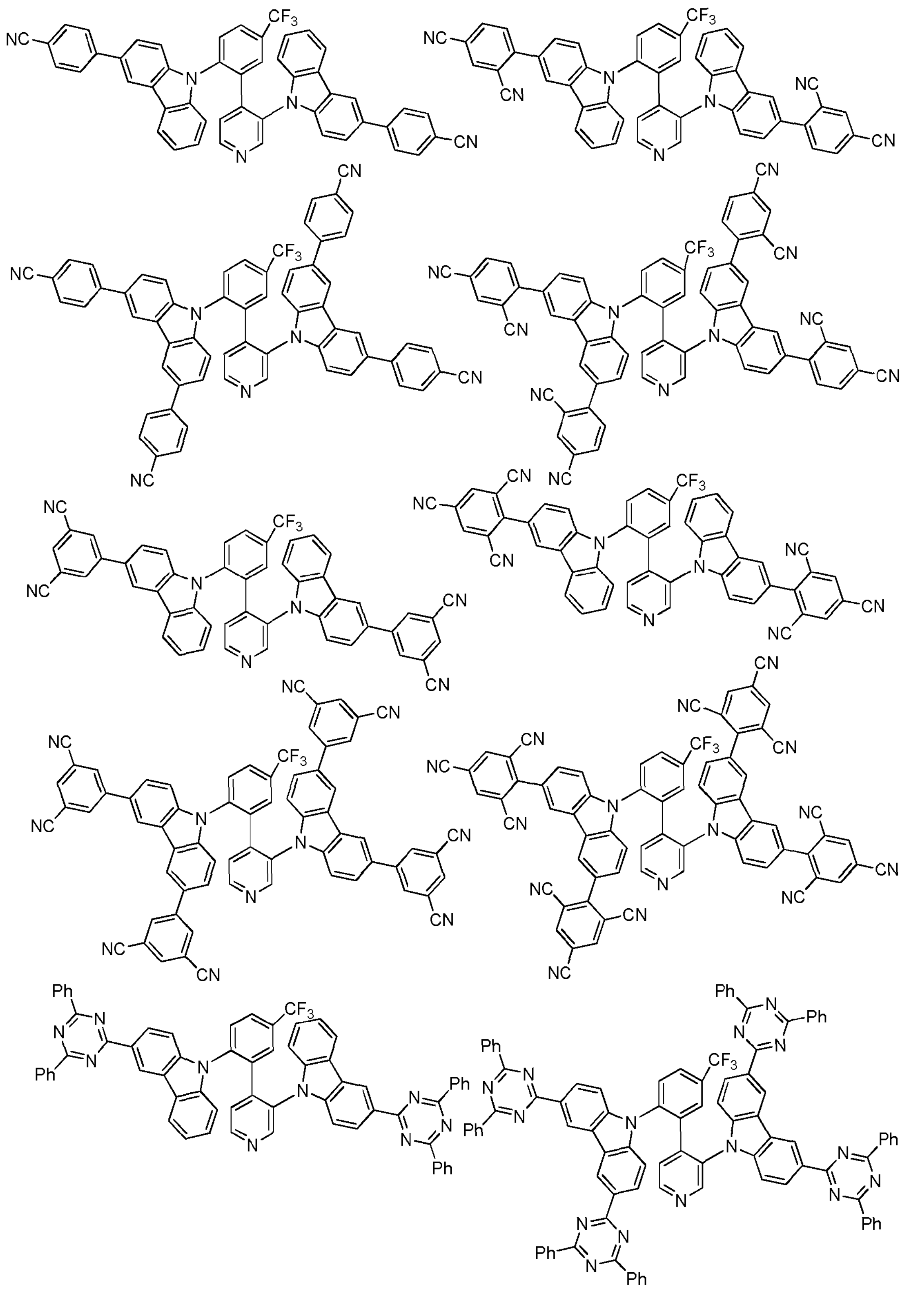

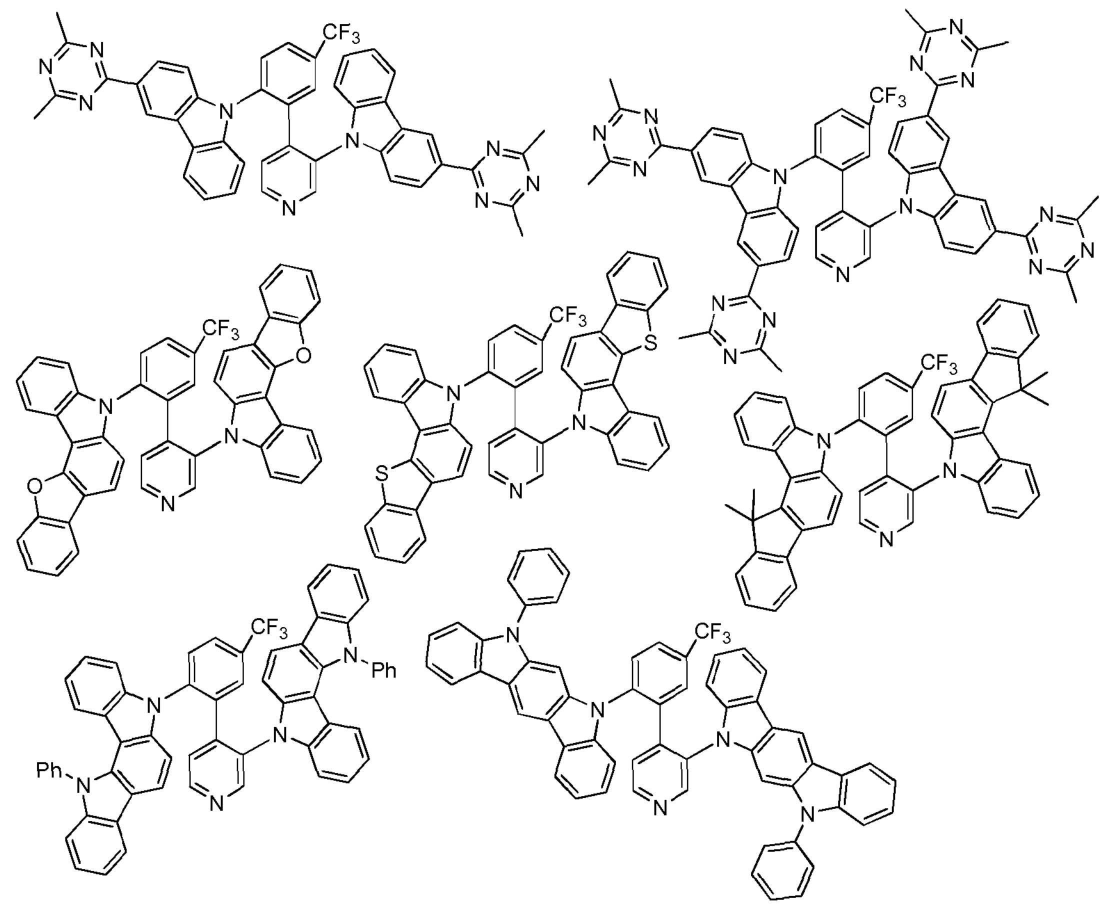

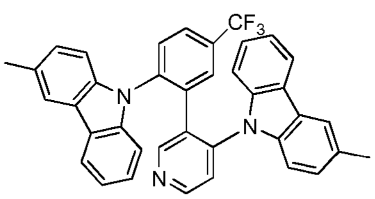

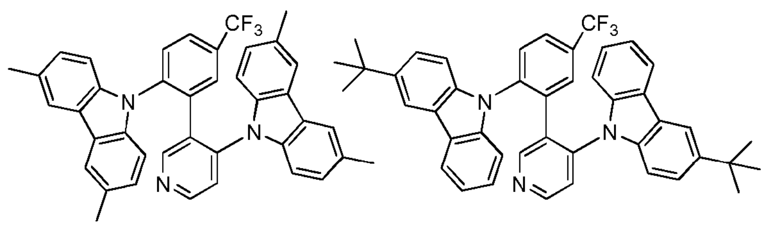

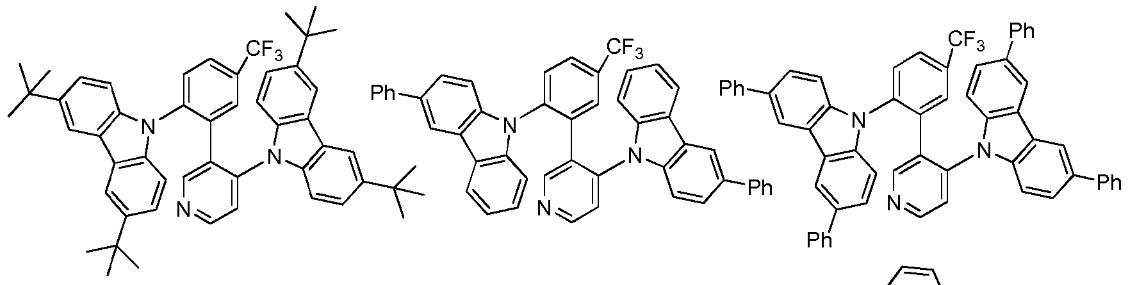

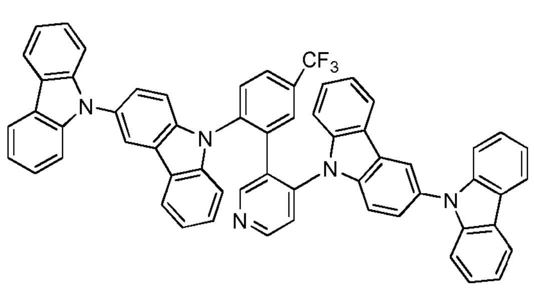

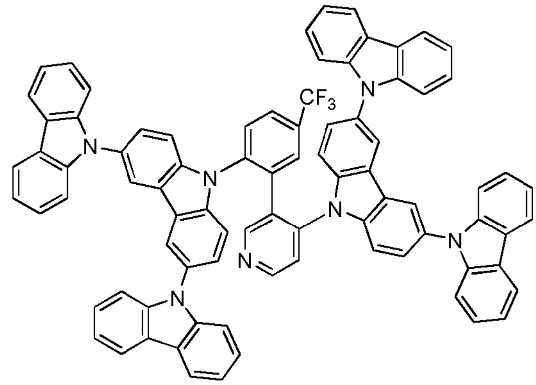

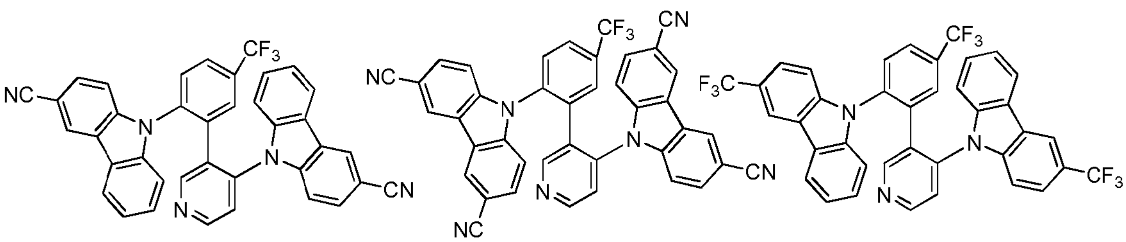

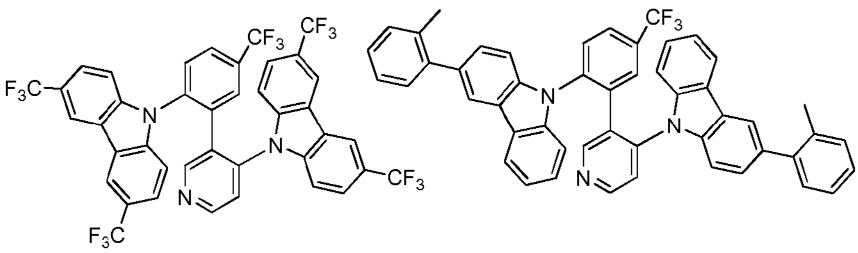

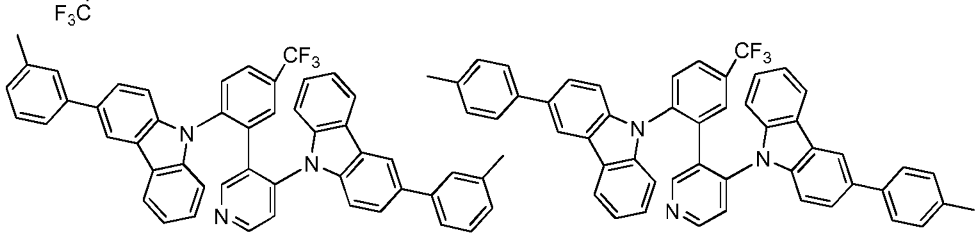

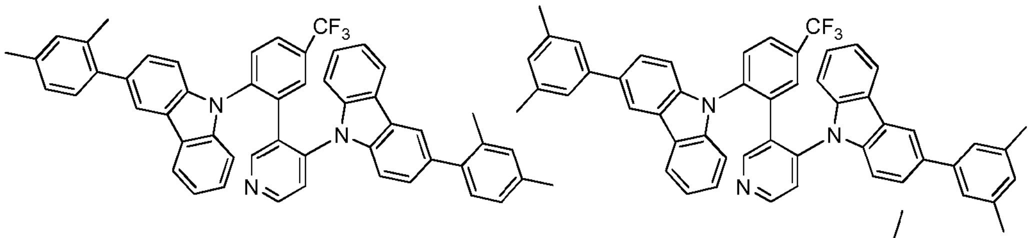

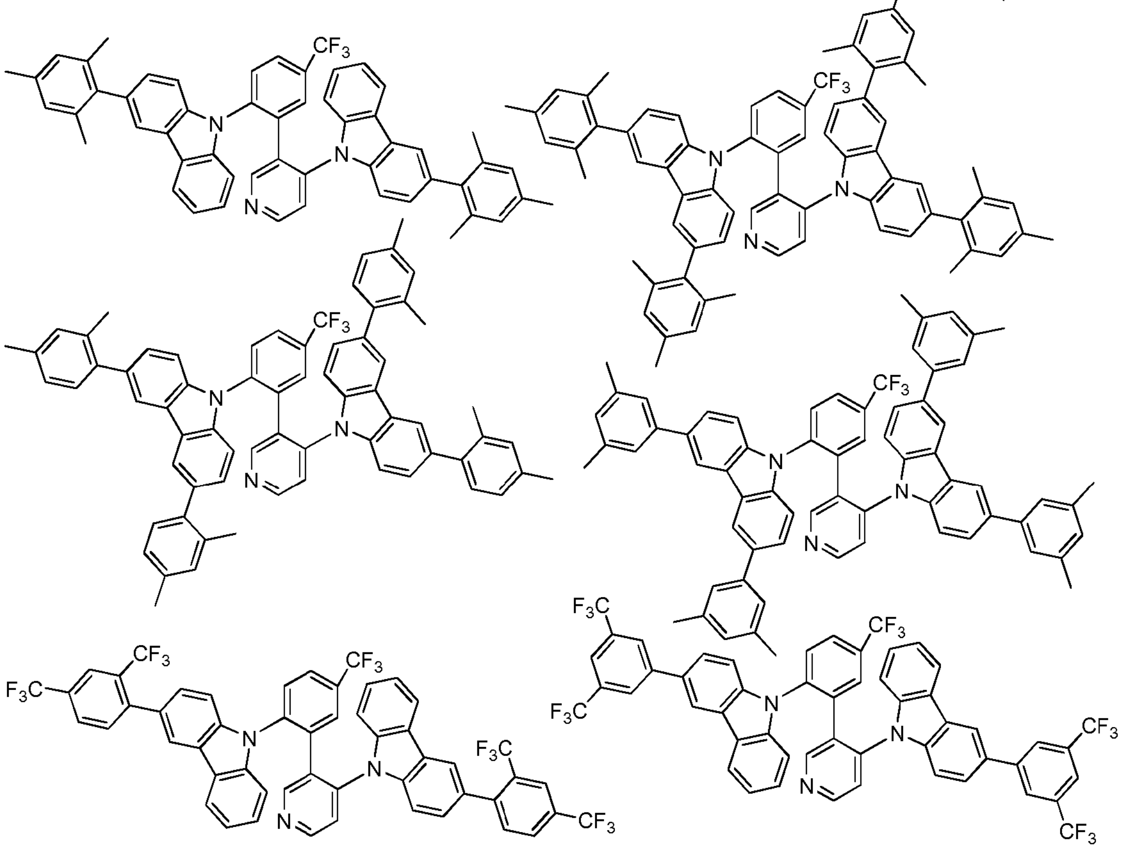

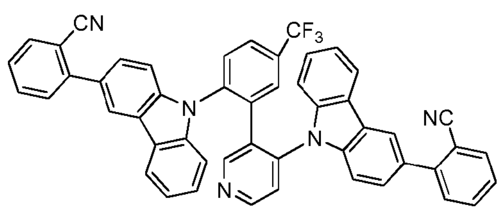

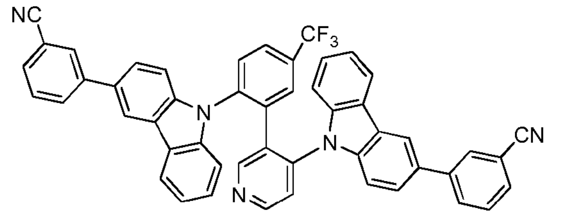

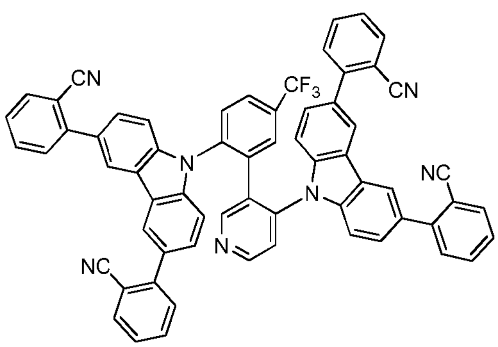

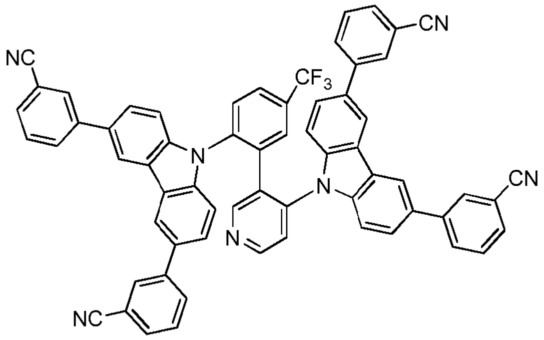

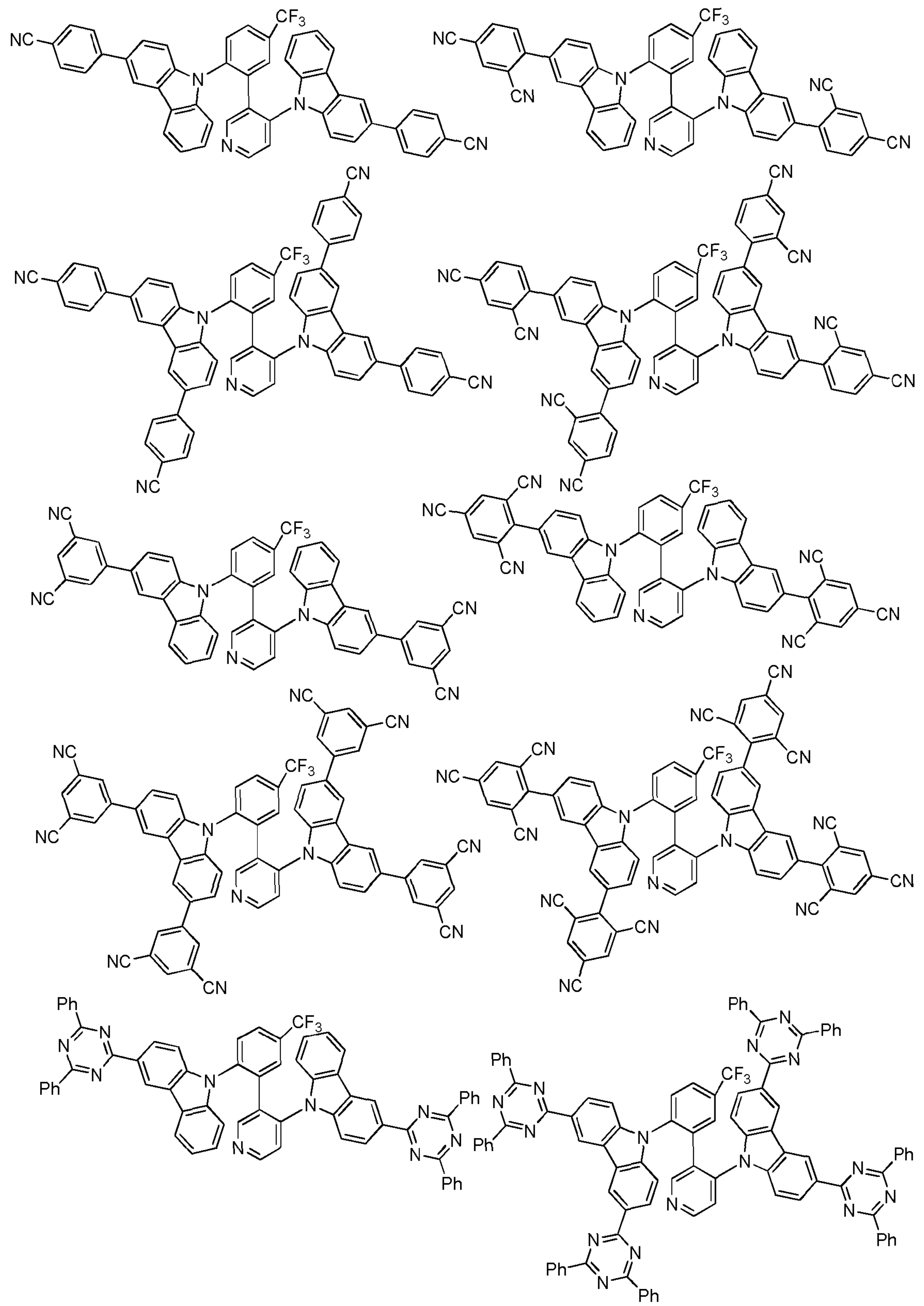

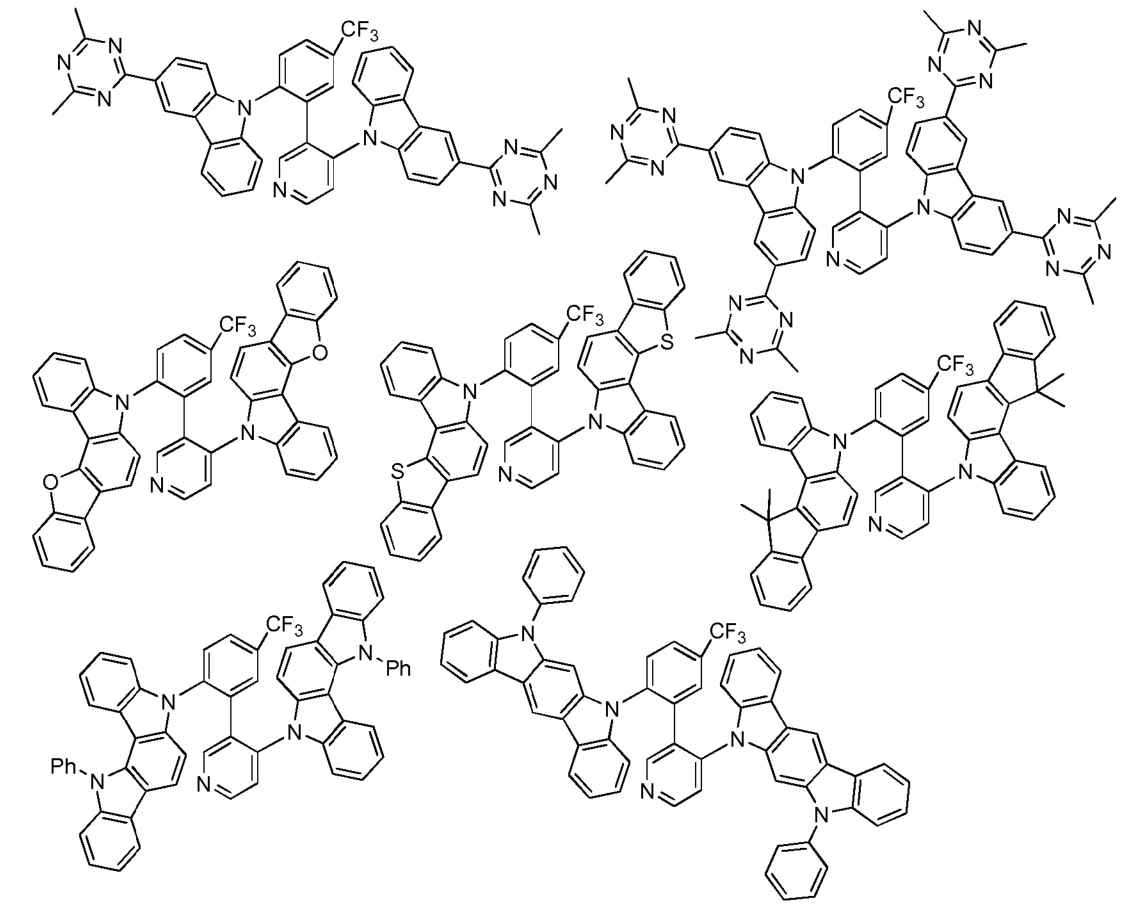

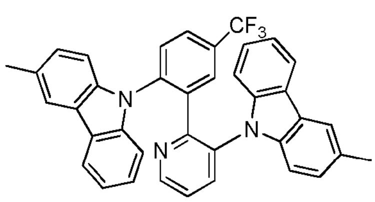

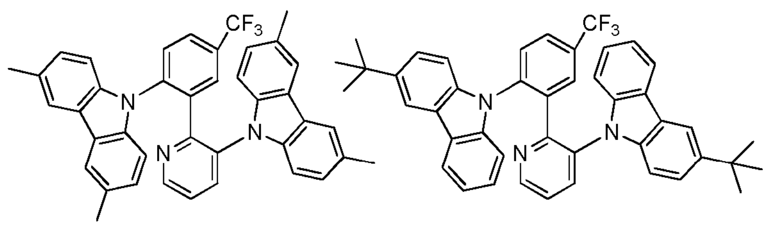

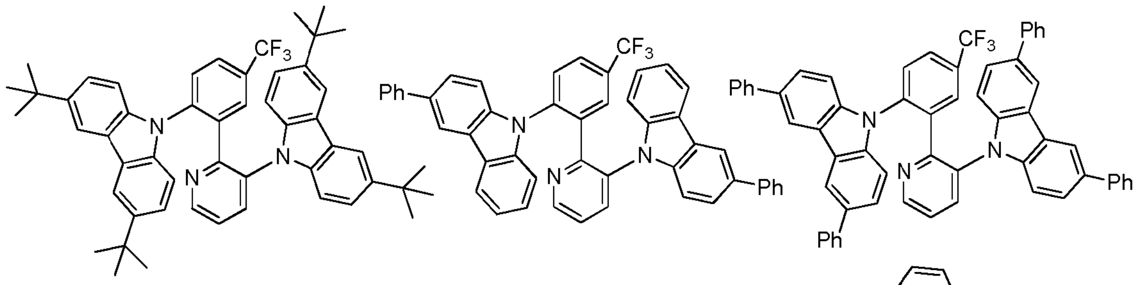

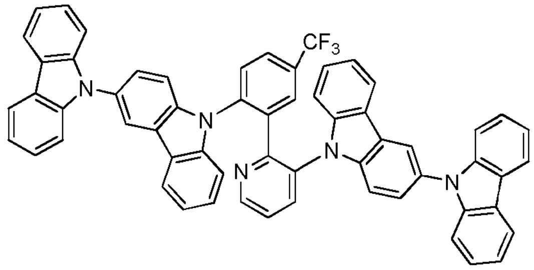

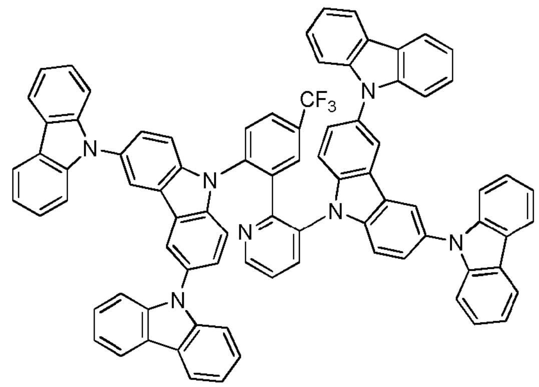

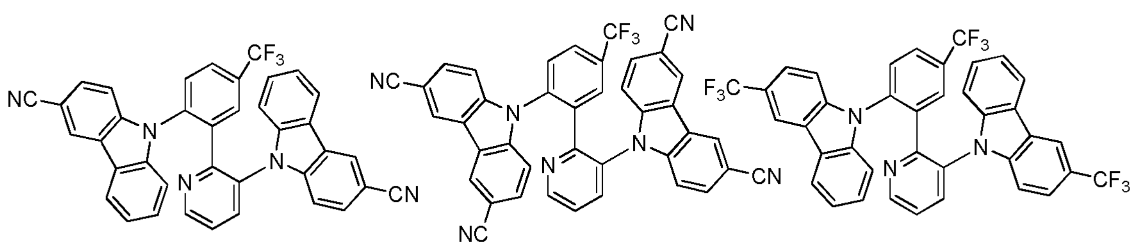

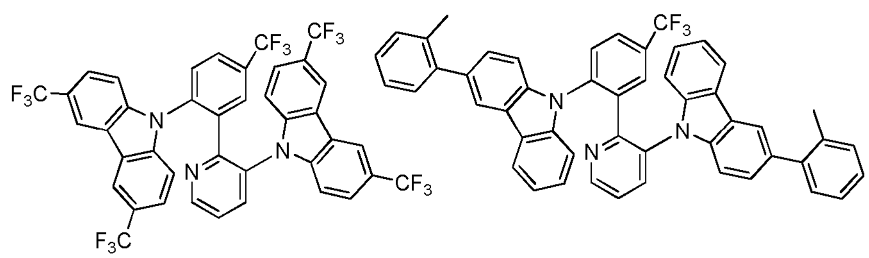

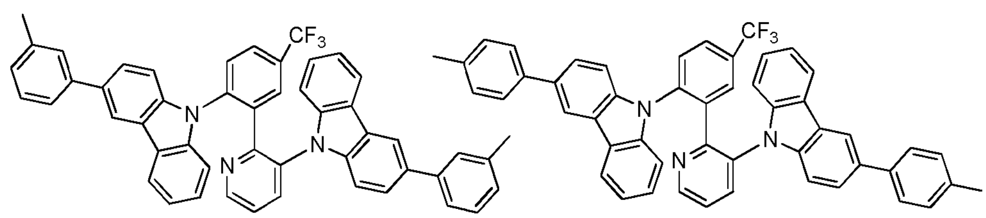

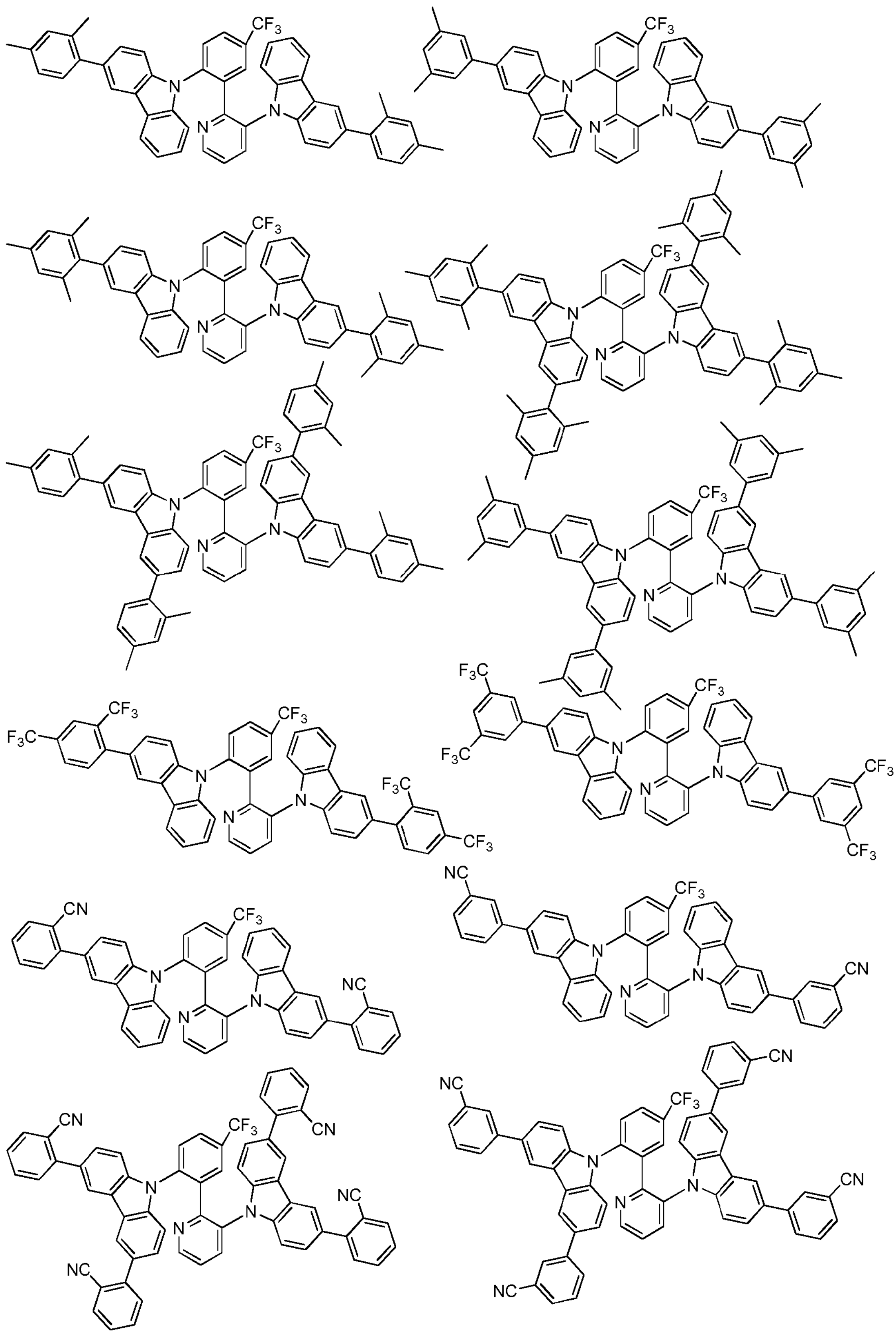

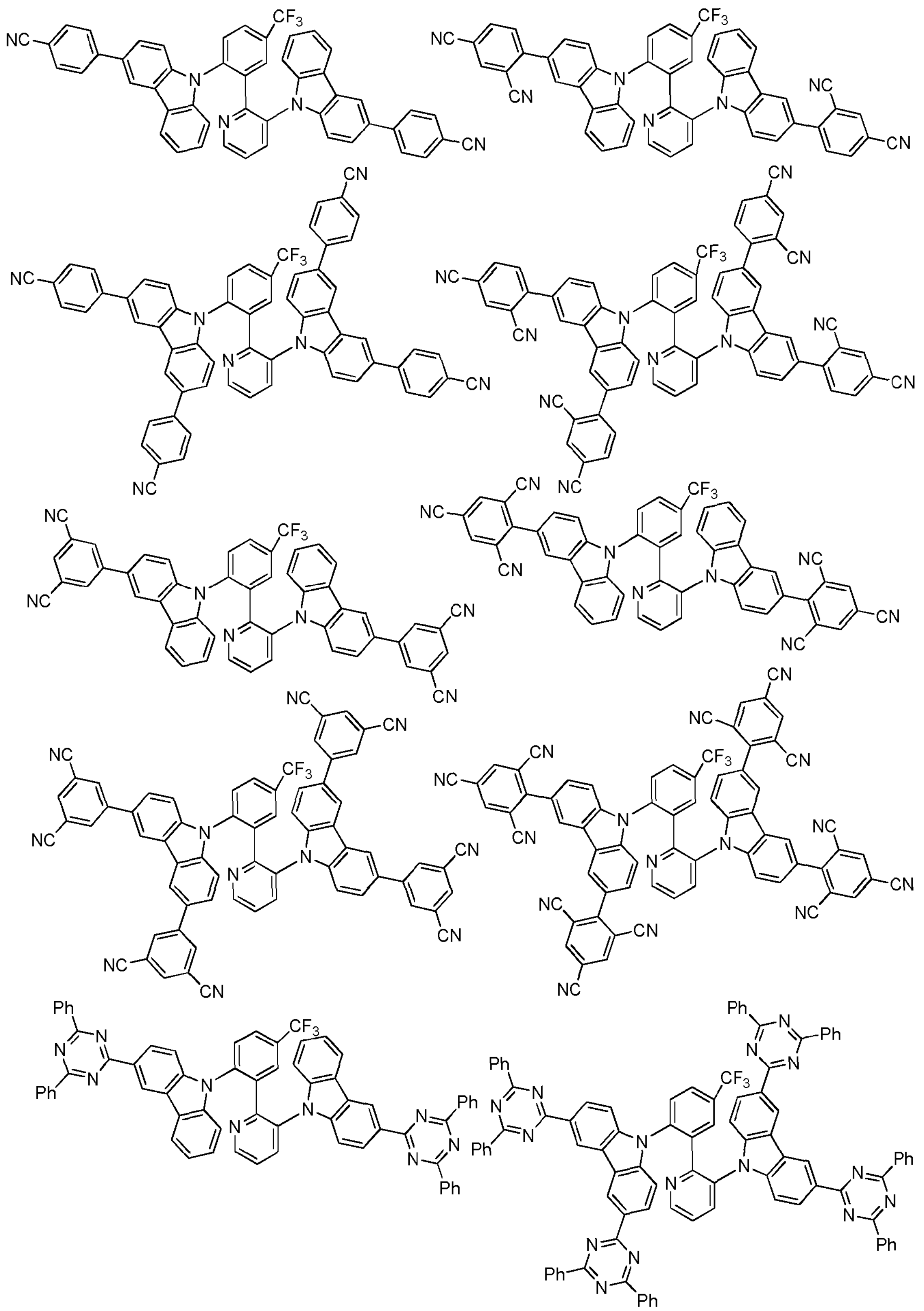

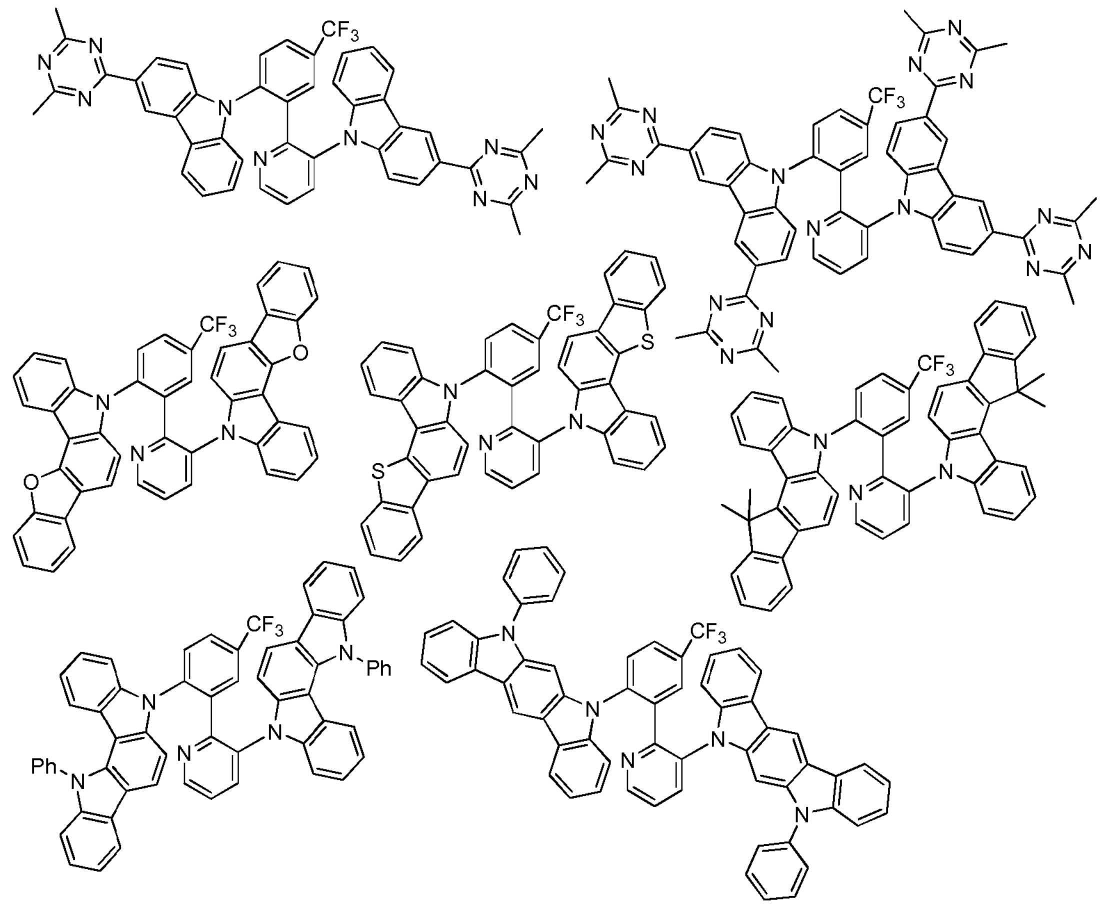

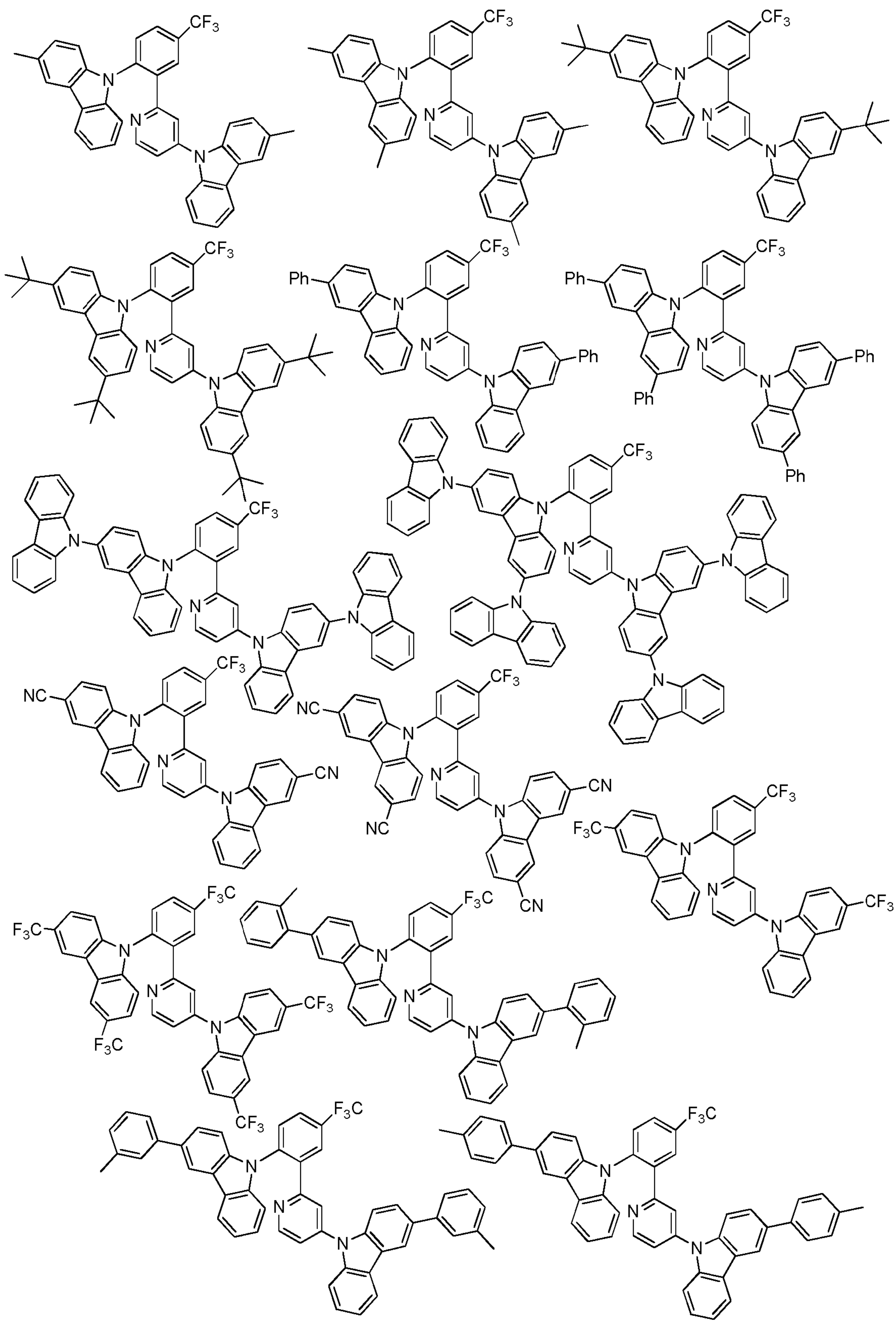

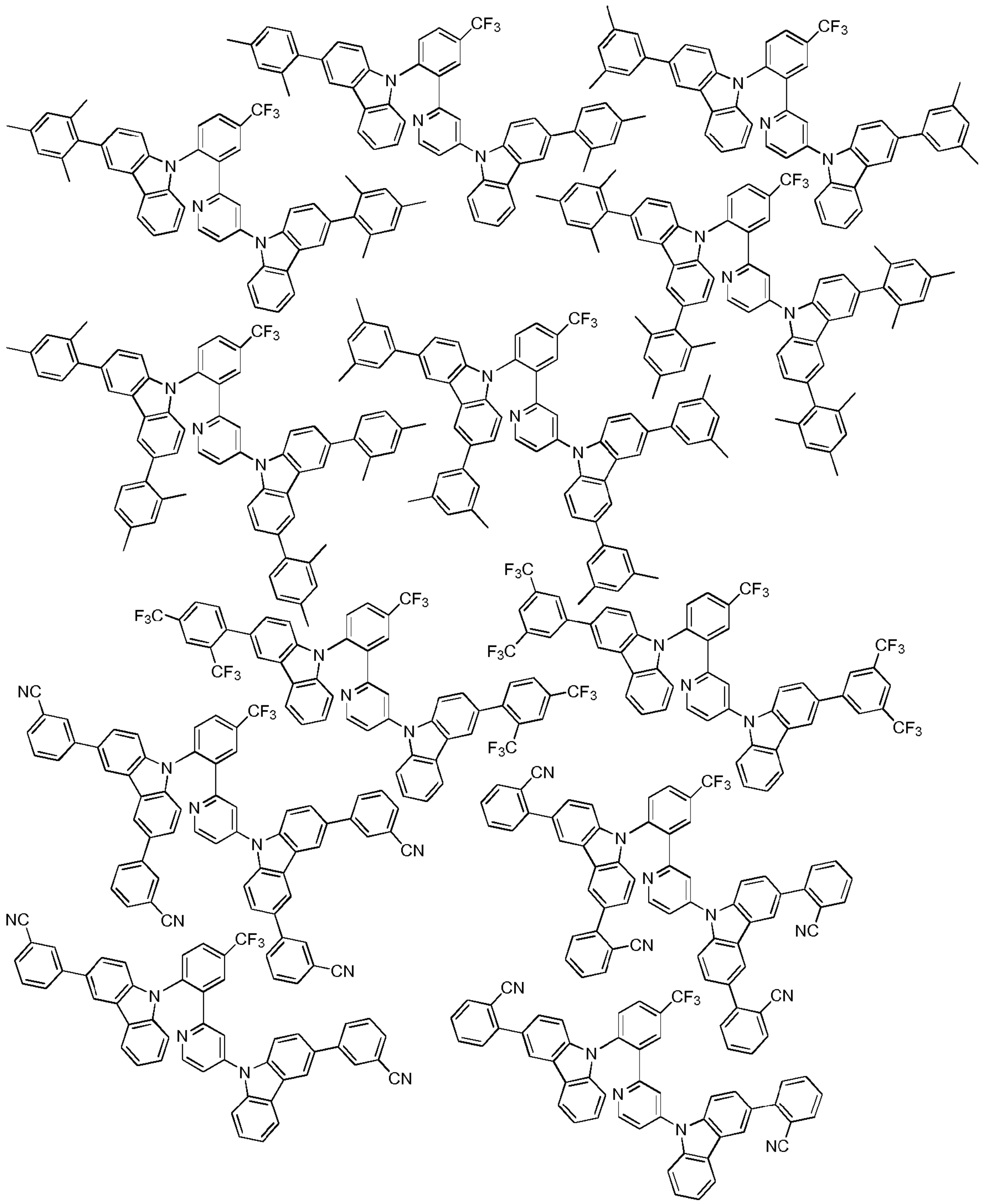

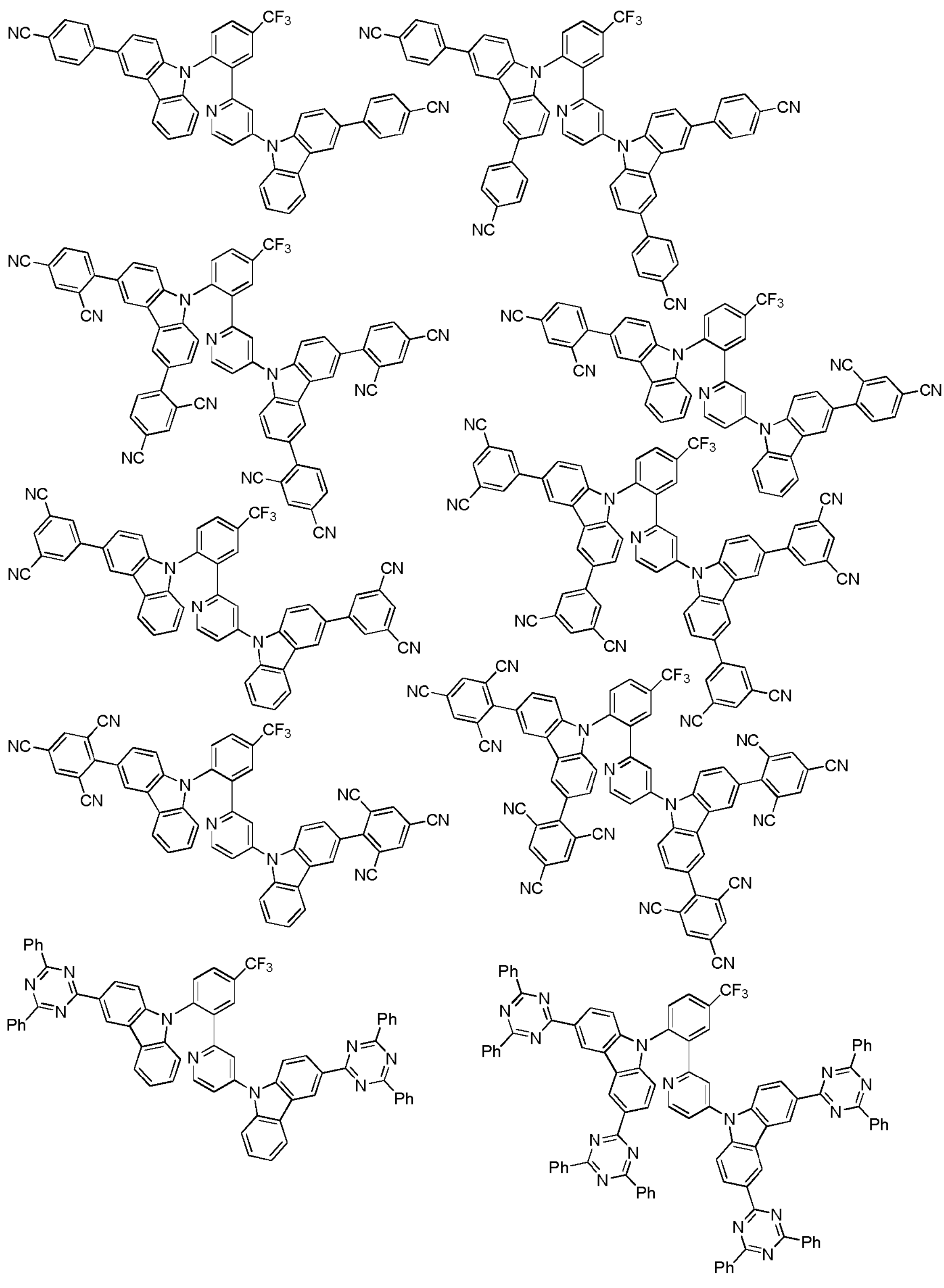

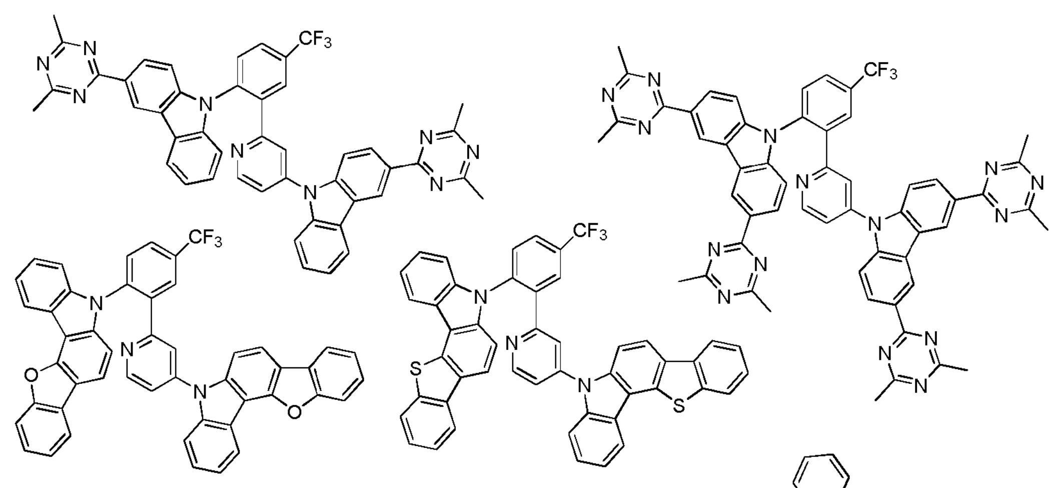

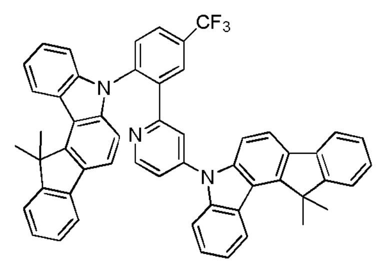

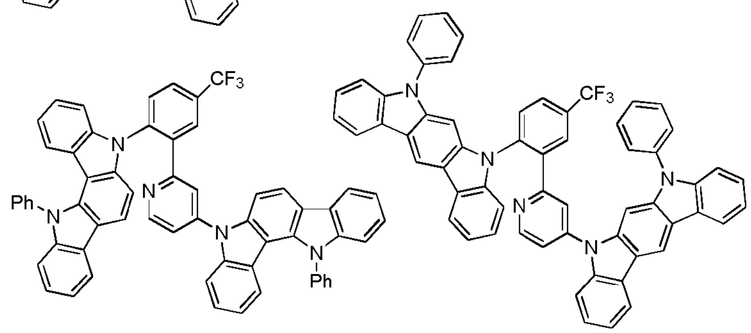

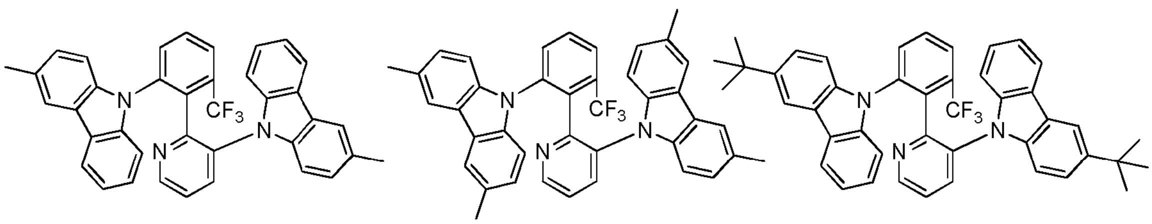

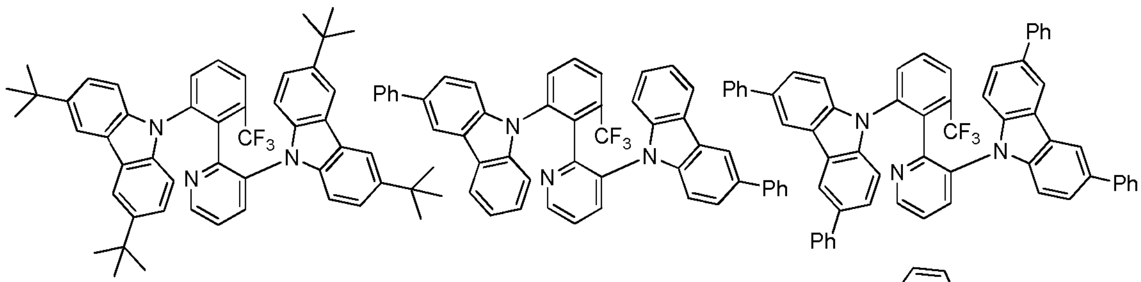

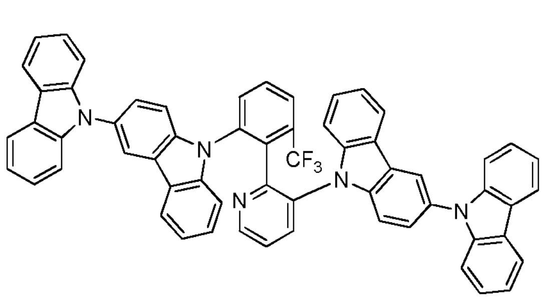

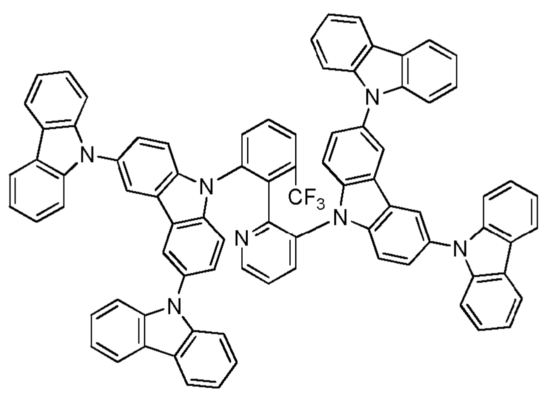

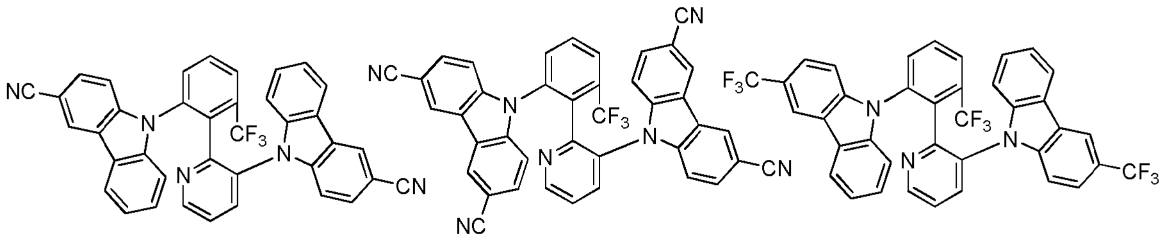

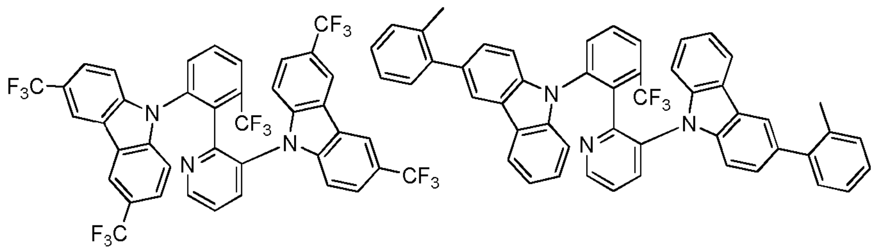

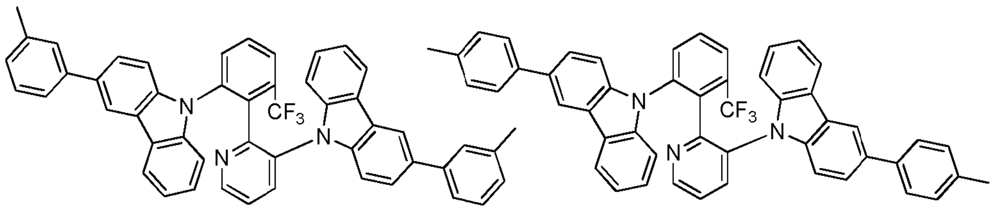

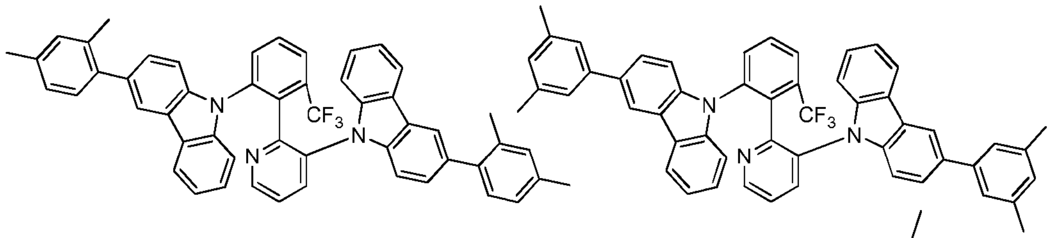

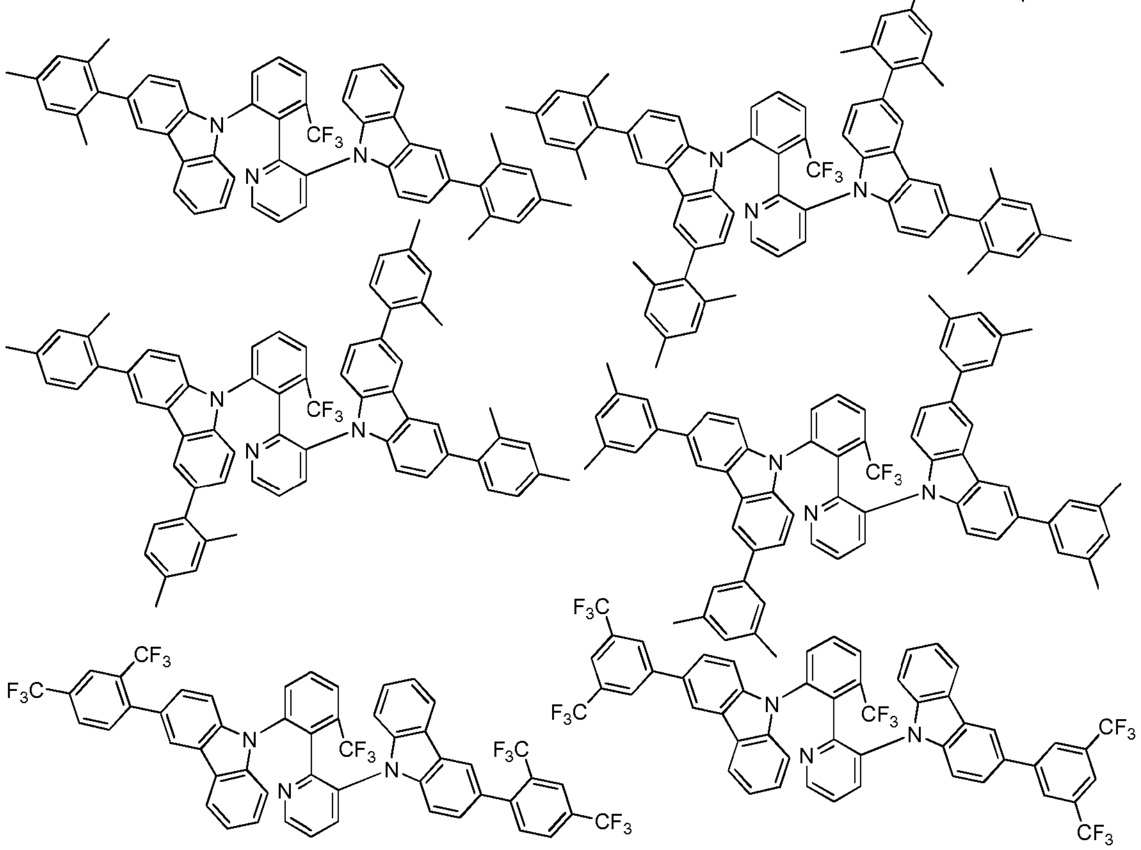

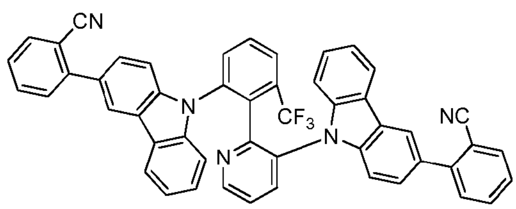

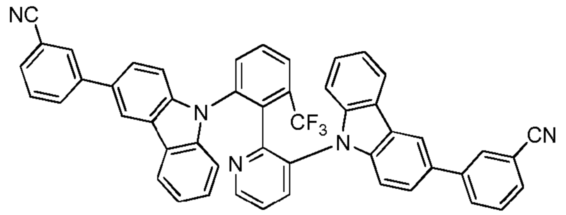

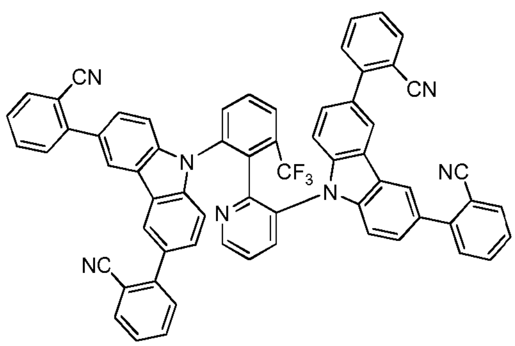

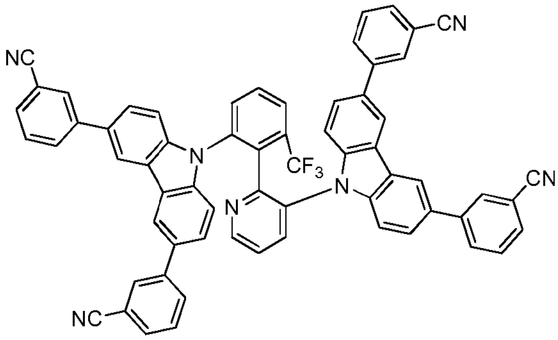

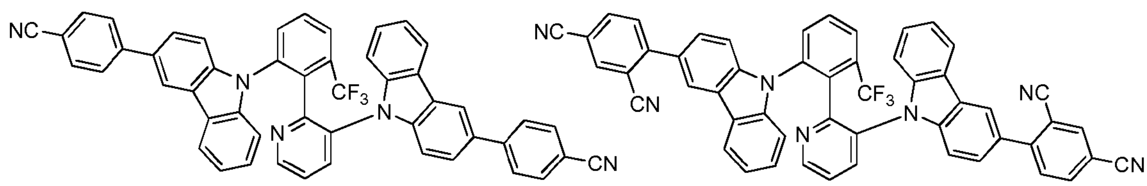

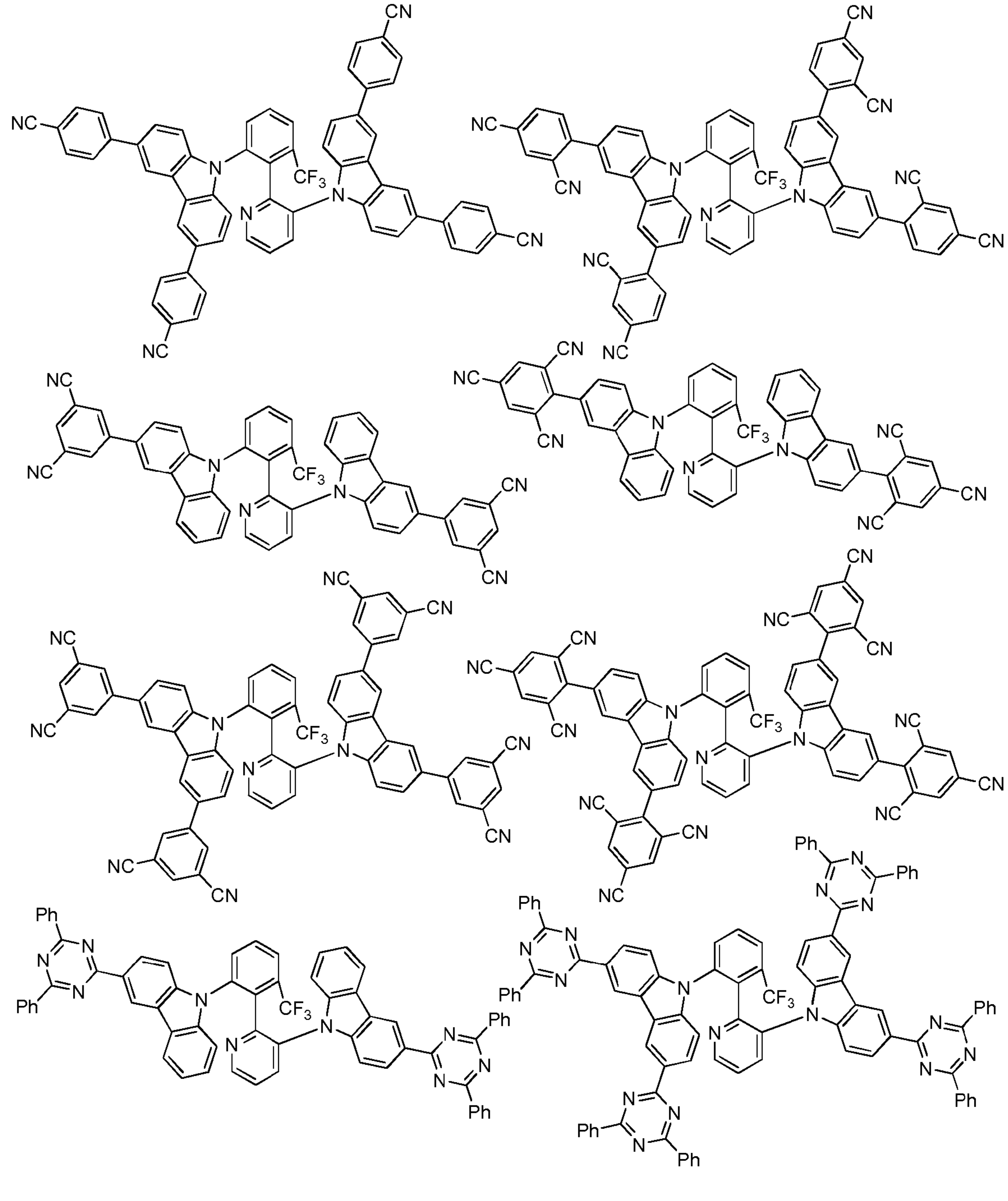

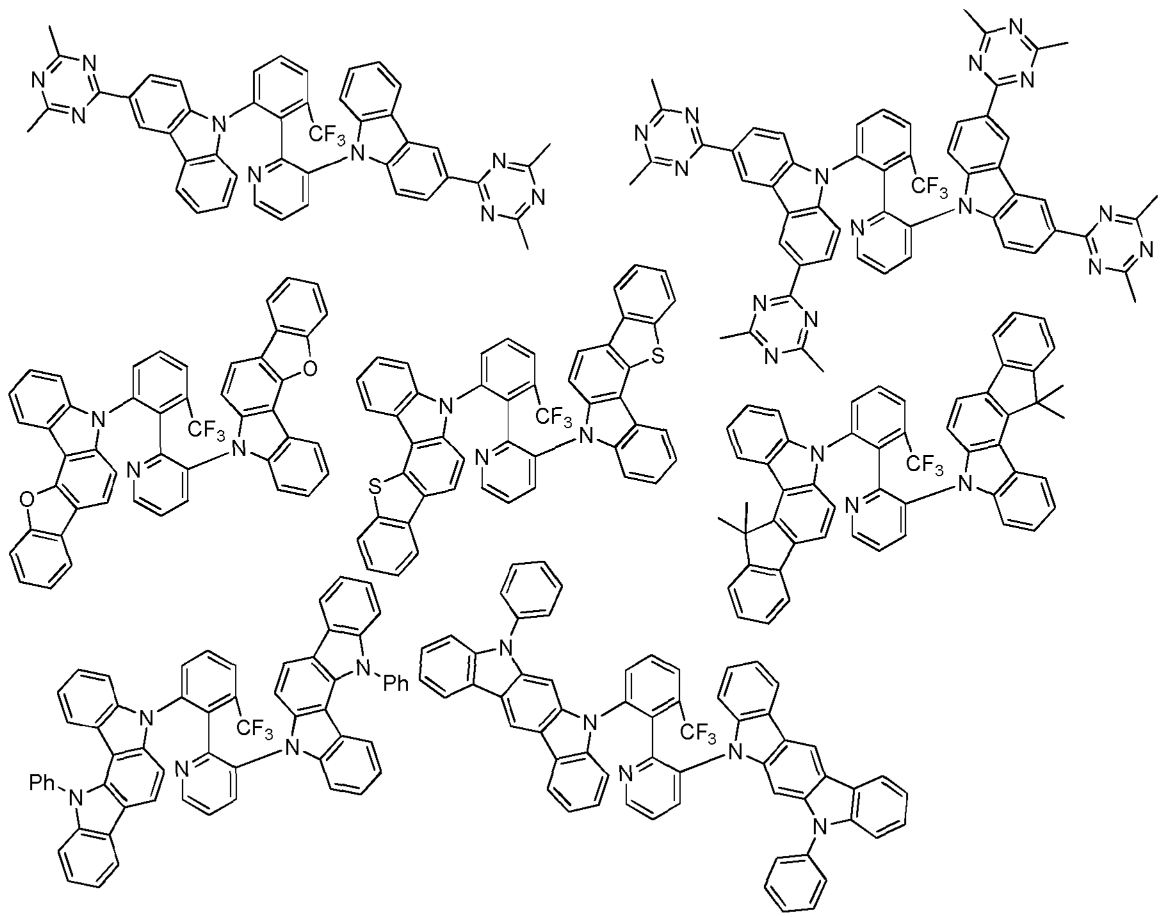

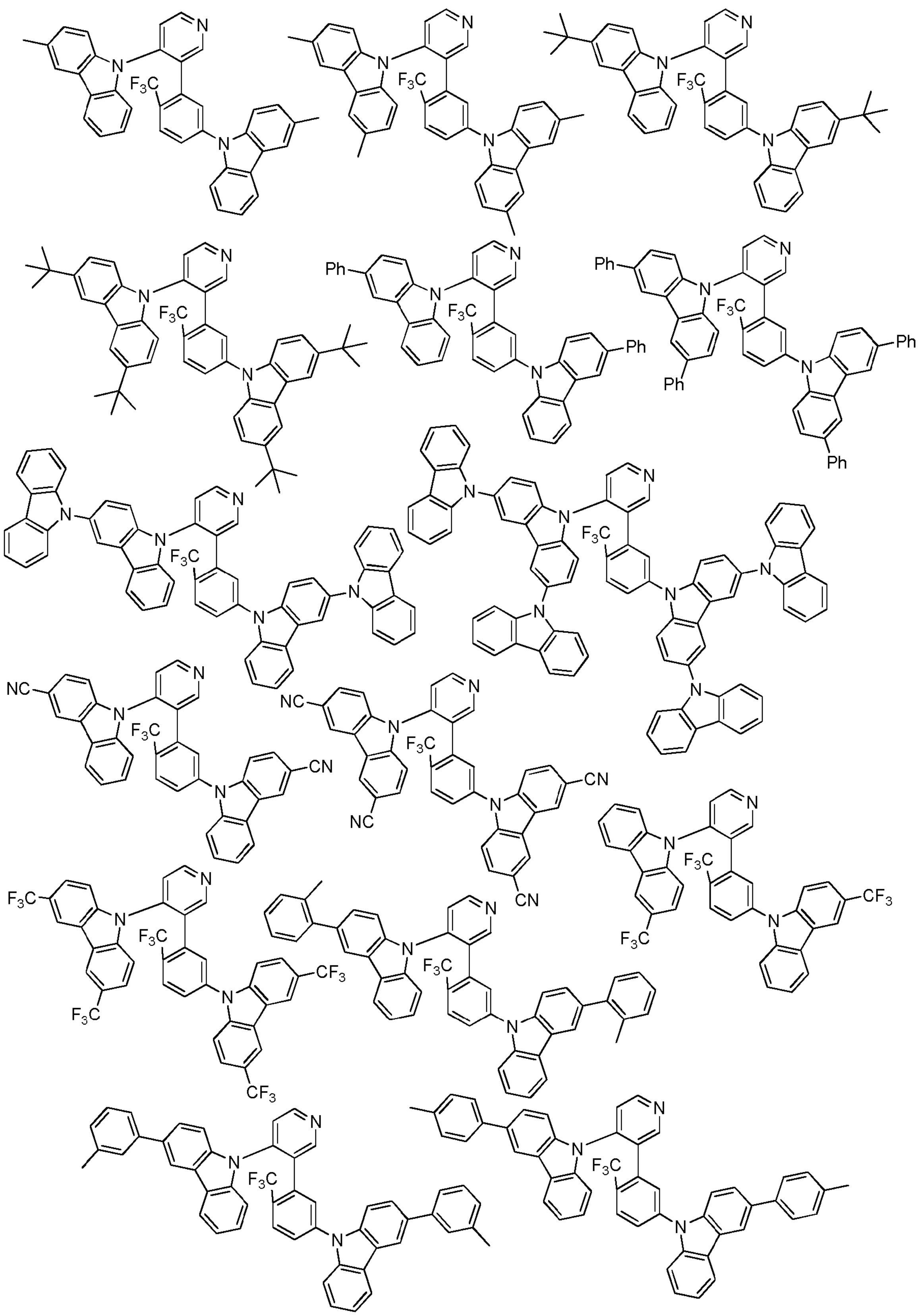

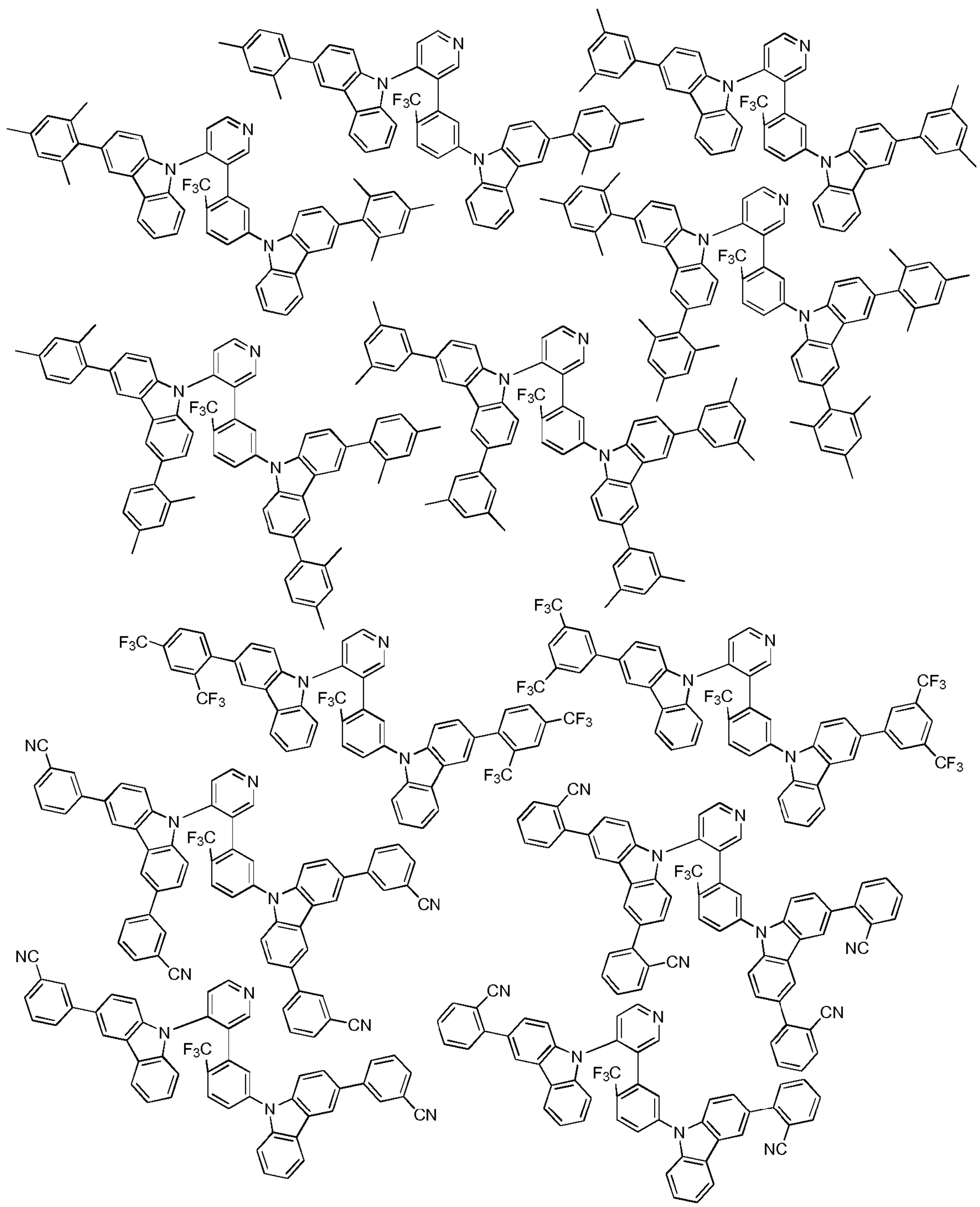

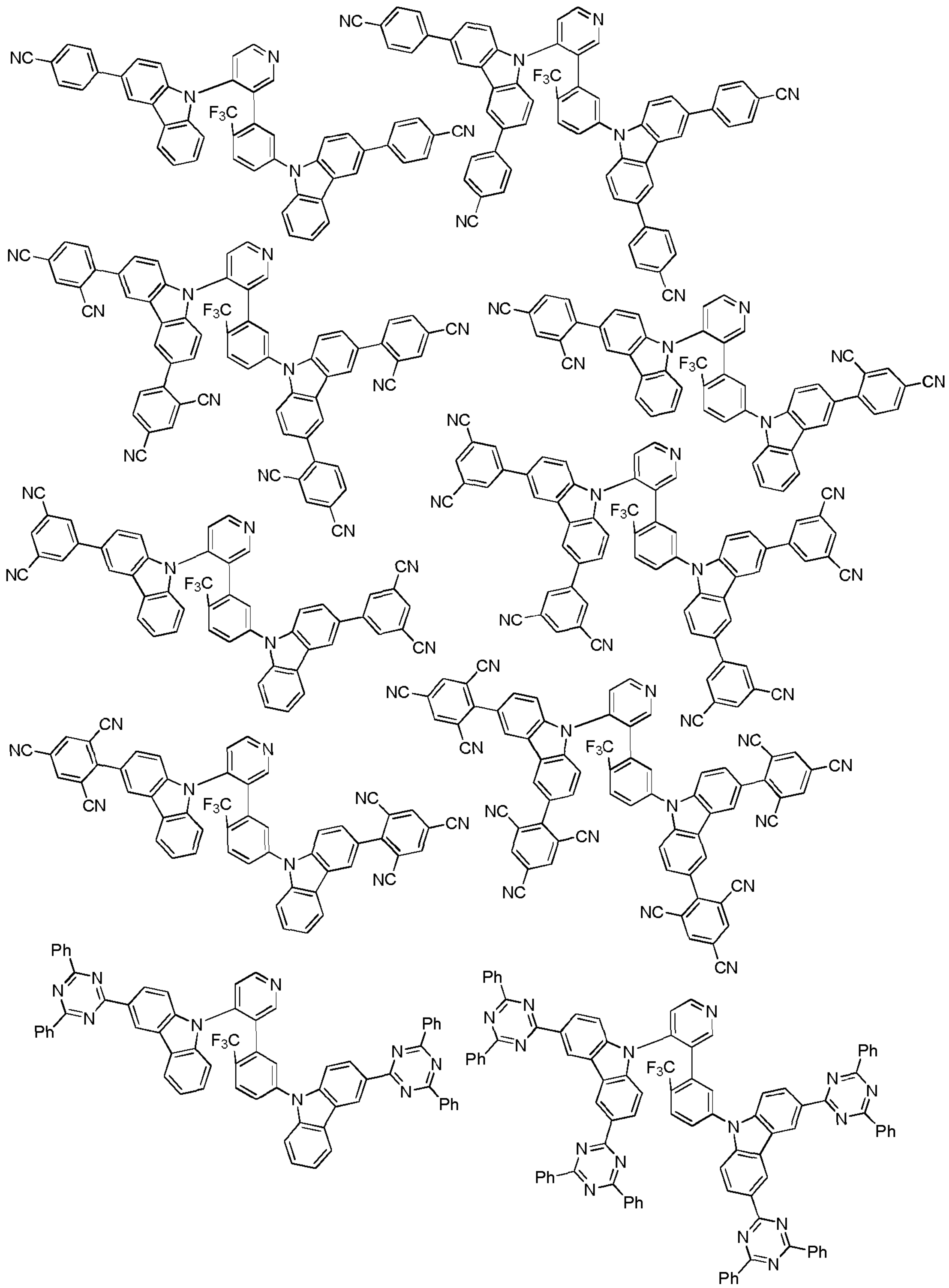

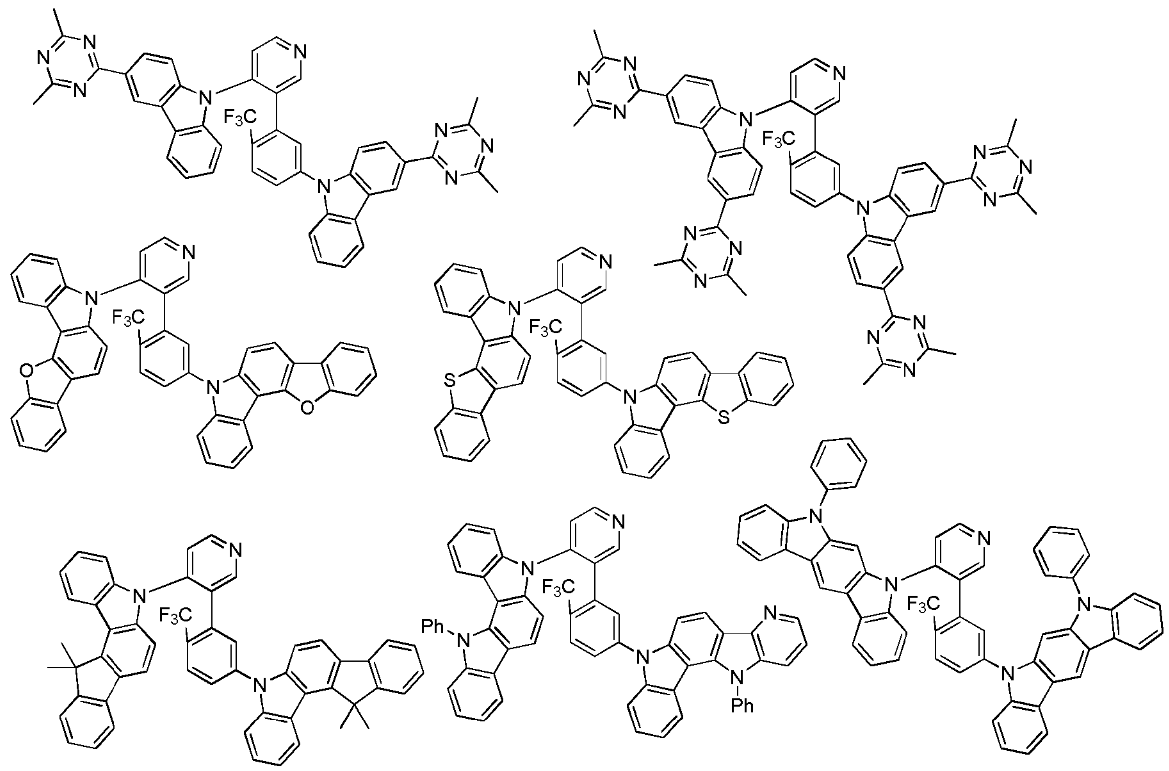

**Figures**

Figure 1 Emission spectrum of example **1** (10% by weight) in PMMA.
Figure 2 Emission spectrum of example **2** (10% by weight) in PMMA.
Figure 3 Emission spectrum of example **3** (10% by weight) in PMMA.

**Claims**

**1.** Organic molecule, comprising

- one first chemical moiety comprising a structure of formula I,

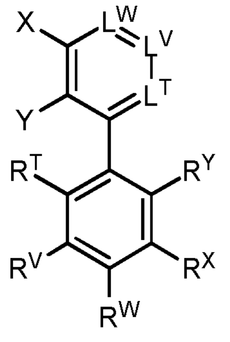

Formula I

and
- two second chemical moieties, each independently from another comprising a structure of formula II,

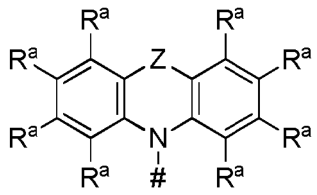

Formula II

wherein the first chemical moiety is linked to each of the two second chemical moieties via a single bond; wherein

$L^T$ is N or C-$R^1$;
$L^V$ is N or C-$R^1$;
$L^W$ is N or C-W;
X is the binding site of a single bond linking the first chemical moiety to one of the two second chemical moieties or is $R^2$;
Y is the binding site of a single bond linking the first chemical moiety to one of the two second chemical moieties or is $R^2$;
W is the binding site of a single bond linking the first chemical moiety to one of the two second chemical moieties or is $R^2$;
$R^T$ is selected from the group consisting of $CF_3$ and $R^I$;
$R^V$ is selected from the group consisting of $CF_3$ and $R^I$;
$R^W$ is the binding site of a single bond linking the first chemical moiety to one of the two second chemical moieties or is selected from the group consisting of $CF_3$ and $R^I$;
$R^X$ is the binding site of a single bond linking the first chemical moiety to one of the two second chemical moieties or is $R^I$;
$R^Y$ is the binding site of a single bond linking the first chemical moiety to one of the two second chemical moieties or is $R^I$;
# represents the binding site of a single bond linking the second chemical moieties to the first chemical moiety;
Z is at each occurrence independently from another selected from the group consisting of a direct bond, $CR^3R^4$, $C=CR^3R^4$, C=O, $C=NR^3$, $NR^3$, O, $SiR^3R^4$, S, S(O) and $S(O)_2$;
$R^1$ is at each occurrence independently from another selected from the group consisting of:

hydrogen, deuterium,
$C_1$-$C_5$-alkyl,
wherein one or more hydrogen atoms are optionally substituted by deuterium;
C2-C8-alkenyl,
wherein one or more hydrogen atoms are optionally substituted by deuterium;
C2-C8-alkynyl,
wherein one or more hydrogen atoms are optionally substituted by deuterium; and
$C_6$-$C_{18}$-aryl,
which is optionally substituted with one or more substituents $R^6$;
$R^2$ is at each occurrence independently from another selected from the group consisting of:

hydrogen, deuterium,
$C_1$-$C_5$-alkyl,
wherein one or more hydrogen atoms are optionally substituted by deuterium;
C2-C8-alkenyl,
wherein one or more hydrogen atoms are optionally substituted by deuterium;
C2-C8-alkynyl,
wherein one or more hydrogen atoms are optionally substituted by deuterium; and
$C_6$-$C_{18}$-aryl,
which is optionally substituted with one or more substituents $R^6$;
$R^I$ is at each occurrence independently from another selected from the group consisting of:

hydrogen, deuterium,
$C_1$-$C_5$-alkyl,

wherein one or more hydrogen atoms are optionally substituted by deuterium;
C2-C8-alkenyl,
wherein one or more hydrogen atoms are optionally substituted by deuterium;
C2-C8-alkynyl,
wherein one or more hydrogen atoms are optionally substituted by deuterium; and
$C_6$-$C_{18}$-aryl,
which is optionally substituted with one or more substituents $R^6$;
$R^a$, $R^3$ and $R^4$ is at each occurrence independently from another selected from the group consisting of: hydrogen, deuterium, $N(R^5)_2$, $OR^5$, $Si(R^5)_3$, $B(OR^5)_2$, $OSO_2R^5$, $CF_3$, CN, F, Br, I, $C_1$-
$C_{40}$-alkyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-alkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-thioalkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkenyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkynyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_6$-$C_{60}$-aryl,
which is optionally substituted with one or more substituents $R^5$; and
$C_3$-$C_{57}$-heteroaryl,
which is optionally substituted with one or more substituents $R^5$;
$R^5$ is at each occurrence independently from another selected from the group consisting of hydrogen, deuterium, $N(R^6)_2$, $OR^6$, $Si(R^6)_3$, $B(OR^6)_2$, $OSO_2R^6$, $CF_3$, CN, F, Br, I, $C_1$-$C_{40}$-alkyl,

which is optionally substituted with one or more substituents $R^6$ and
wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_1$-$C_{40}$-alkoxy,

which is optionally substituted with one or more substituents $R^6$ and
wherein one or more non-adjacent $CH_2$-groups are optionally substituted by $R^6C{=}CR^6$, $C{\equiv}C$, $Si(R^6)_2$, $Ge(R^6)_2$, $Sn(R^6)_2$, C=O, C=S, C=Se, $C{=}NR^6$, $P({=}O)(R^6)$, SO, $SO_2$, $NR^6$, O, S or $CONR^6$;

$C_1$-$C_{40}$-thioalkoxy,

which is optionally substituted with one or more substituents $R^6$ and
wherein one or more non-adjacent $CH_2$-groups are optionally substituted by $R^6C{=}CR^6$, $C{\equiv}C$, $Si(R^6)_2$, $Ge(R^6)_2$, $Sn(R^6)_2$, C=O, C=S, C=Se, $C{=}NR^6$, $P({=}O)(R^6)$, SO, $SO_2$, $NR^6$, O, S or $CONR^6$;

$C_2$-$C_{40}$-alkenyl,

which is optionally substituted with one or more substituents $R^6$ and
wherein one or more non-adjacent $CH_2$-groups are optionally substituted by $R^6C{=}CR^6$, $C{\equiv}C$, $Si(R^6)_2$, $Ge(R^6)_2$, $Sn(R^6)_2$, C=O, C=S, C=Se, $C{=}NR^6$, $P({=}O)(R^6)$, SO, $SO_2$, $NR^6$, O, S or $CONR^6$;

$C_2$-$C_{40}$-alkynyl,

which is optionally substituted with one or more substituents $R^6$ and
wherein one or more non-adjacent $CH_2$-groups are optionally substituted by $R^6C{=}CR^6$, $C{\equiv}C$, $Si(R^6)_2$, $Ge(R^6)_2$, $Sn(R^6)_2$, C=O, C=S, C=Se, $C{=}NR^6$, $P({=}O)(R^6)$, SO, $SO_2$, $NR^6$, O, S or $CONR^6$;

$C_6$-$C_{60}$-aryl,
which is optionally substituted with one or more substituents $R^6$; and
$C_3$-$C_{57}$-heteroaryl,
which is optionally substituted with one or more substituents $R^6$;
$R^6$ is at each occurrence independently from another selected from the group consisting of:

hydrogen, deuterium, OPh, $CF_3$, CN, F,
$C_1$-$C_5$-alkyl,
wherein one or more hydrogen atoms are optionally, independently from each other substituted by deuterium, CN, $CF_3$, or F;
$C_1$-$C_5$-alkoxy,
wherein one or more hydrogen atoms are optionally, independently from each other substituted by deuterium, CN, $CF_3$, or F;
$C_1$-$C_5$-thioalkoxy,
wherein one or more hydrogen atoms are optionally, independently from each other substituted by deuterium, CN, $CF_3$, or F;
C2-C5-alkenyl,
wherein one or more hydrogen atoms are optionally, independently from each other substituted by deuterium, CN, $CF_3$, or F;
C2-C5-alkynyl,
wherein one or more hydrogen atoms are optionally, independently from each other substituted by deuterium, CN, $CF_3$, or F;
$C_6$-$C_{18}$-aryl,
which is optionally substituted with one or more $C_1$-$C_5$-alkyl substituents;
$C_3$-$C_{17}$-heteroaryl,
which is optionally substituted with one or more $C_1$-$C_5$-alkyl substituents;
$N(C_6$-$C_{18}$-aryl$)_2$;
$N(C_3$-$C_{17}$-heteroaryl$)_2$,
and $N(C_3$-$C_{17}$-heteroaryl$)(C_6$-$C_{18}$-aryl$)$;
wherein the substituents $R^a$, $R^3$, $R^4$ or $R^5$ independently from each other optionally form a mono- or polycyclic, aliphatic, aromatic and/or benzo-fused ring system with one or more substituents $R^a$, $R^3$, $R^4$ or $R^5$;

wherein the terms alkyl, alkenyl, alkynyl, alkoxy and thioalkoxy can refer to linear, branched, and cyclic substituents;
wherein
exactly one ring atom selected from the group consisting of $L^T$, $L^V$ and $L^W$ is N,
exactly one substituent selected from the group consisting of $R^T$, $R^V$ and $R^W$ is $CF_3$,
exactly one substituent selected from the group consisting of W, Y and X represents the binding site of a single bond linking the first chemical moiety and one of the two second chemical moieties and
exactly one substituent selected from the group consisting of $R^W$, $R^Y$ and $R^X$ represents the binding site of a single bond linking the first chemical moiety and one of the two second chemical moieties.

2. Organic molecule according to claim 1, wherein the first chemical moiety comprises a structure of formula Ia:

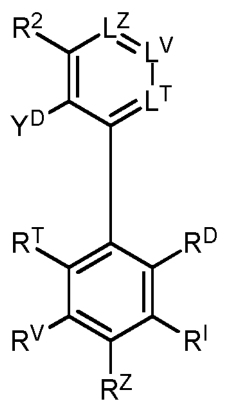

Formula Ia

wherein

$Y^D$ is the binding site of a single bond linking the first chemical moiety to one of the two second chemical moieties;
$R^D$ is the binding site of a single bond linking the first chemical moiety to one of the two second chemical moieties;
$L^Z$ is N or C-$R^2$;
$R^Z$ is $CF_3$ or $R^I$;
wherein exactly one substituent selected from the group consisting of $R^T$, $R^V$ and $R^Z$ is $CF_3$ and exactly one ring atom selected from the group consisting of $L^T$, $L^V$ and $L^Z$ is N; and wherein apart from that the definitions in claim 1 apply.

3. Organic molecule according to claim 1 or 2, wherein $R^1$, $R^2$ and $R^I$ is independently from each other at each occurrence independently from another selected from the group consisting of H, methyl, mesityl, tolyl and phenyl.

4. Organic molecule according to one or more of claims 1 to 3, wherein $R^W$ or $R^Z$ is $CF_3$.

5. Organic molecule according to one or more of claims 1 to 4, wherein the two second chemical moieties, each at each occurrence independently from another comprise a structure of formula IIa:

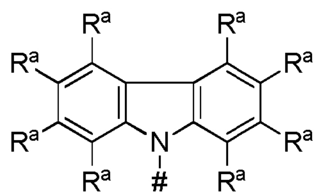

Formula IIa

wherein # and $R^a$ are defined as in claim 1.

6. Organic molecule according to one or more of claims 1 to 5, wherein the two second chemical moieties, each at each occurrence independently from another comprise a structure of formula IIb:

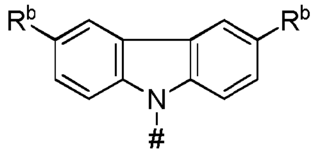

Formula IIb

wherein
$R^b$ is at each occurrence independently from another selected from the group consisting of:

deuterium, $N(R^5)_2$, $OR^5$, $Si(R^5)_3$, $B(OR^5)_2$, $OSO_2R^5$, $CF_3$, CN, F, Br, I,
$C_1$-$C_{40}$-alkyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-alkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-thioalkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkenyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkynyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_6$-$C_{60}$-aryl,
which is optionally substituted with one or more substituents $R^5$; and
$C_3$-$C_{57}$-heteroaryl,
which is optionally substituted with one or more substituents $R^5$;
and wherein apart from that the definitions in claim 1 apply.

7. Organic molecule according to one or more of claims 1 to 5, wherein the two second chemical moieties, each at each occurrence independently from another comprise a structure of formula IIc:

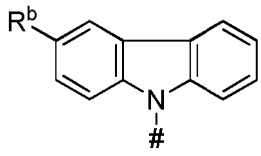

Formula IIc

wherein

$R^b$ is at each occurrence independently from another selected from the group consisting of deuterium, $N(R^5)_2$, $OR^5$, $Si(R^5)_3$, $B(OR^5)_2$, $OSO_2R^5$, $CF_3$, CN, F, Br, I,
$C_1$-$C_{40}$-alkyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-alkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-thioalkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkenyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkynyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_6$-$C_{60}$-aryl,
which is optionally substituted with one or more substituents $R^5$; and
$C_3$-$C_{57}$-heteroaryl,
which is optionally substituted with one or more substituents $R^5$;
and wherein apart from that the definitions in claim 1 apply.

**8.** Organic molecule according to claim 6 or 7, wherein $R^b$ is at each occurrence independently from another selected from the group consisting of

- Me, $^i$Pr, $^t$Bu, CN, $CF_3$,
- Ph, which is optionally substituted with one or more substituents independently from each other selected from the group consisting of Me, $^i$Pr, $^t$Bu, CN, $CF_3$ and Ph;
- pyridinyl, which is optionally substituted with one or more substituents independently from each other selected from the group consisting of Me, $^i$Pr, $^t$Bu, CN, $CF_3$ and Ph;
- pyrimidinyl, which is optionally substituted with one or more substituents independently from each other selected from the group consisting of Me, $^i$Pr, $^t$Bu, CN, $CF_3$ and Ph;

- carbazolyl, which is optionally substituted with one or more substituents independently from each other selected from the group consisting of Me, $^{i}Pr$, $^{t}Bu$, CN, $CF_3$ and Ph;
- triazinyl, which is optionally substituted with one or more substituents independently from each other selected from the group consisting of Me, $^{i}Pr$, $^{t}Bu$, CN, $CF_3$, and Ph;
and
- $N(Ph)_2$.

**9.** Method for preparing an organic molecule according to any of claims 1 to 8, comprising providing a halo-fluorobenzotrifluoride that is substituted with three $R^I$ as a reactant.

**10.** Use of an organic molecule according to one or more of claims 1 to 8 as luminescent emitter and/or host material and/or electron transport material and/or hole injection material and/or hole blocking material in an optoelectronic device.

**11.** Use according to claim 10, wherein the optoelectronic device is selected from the group consisting of:

• organic light-emitting diodes (OLEDS),
• light-emitting electrochemical cells,
• OLED-sensors, in particular in non-hermetically shielded gas and vapor sensors,
• organic diodes,
• organic solar cells,
• organic transistors,
• organic field-effect transistors,
• organic lasers and
• down-conversion elements.

**12.** Composition, comprising:

(a) an organic molecule according to one or more of claims 1 to 8, in particular in the form of an emitter and/or a host, and
(b) an emitter and/or host material, which differs from the organic molecule, and
(c) optionally, a dye and/or a solvent.

**13.** Optoelectronic device, comprising an organic molecule according to one or more of claims 1 to 8 or a composition according to claim 12, in particular in form of a device selected from the group consisting of organic light-emitting diode (OLED), light-emitting electrochemical cell OLED-sensor, in particular in non-hermetically shielded gas and vapor sensors, organic diode, organic solar cell, organic transistor, organic field-effect transistor, organic laser and down-conversion element.

**14.** Optoelectronic device according to claim 13, comprising

- a substrate,
- an anode and
- a cathode, wherein the anode or the cathode are disposed on the substrate and
- at least a light-emitting layer, which is arranged between the anode and the cathode and which comprises the organic molecule or the composition.

**15.** Method for producing an optoelectronic device, wherein an organic molecule according to any one of claims 1 to 8 or a composition according to claim 12 is used, in particular comprising the processing of the organic molecule by vacuum evaporation method or from a solution.

**Patentansprüche**

**1.** Organisches Molekül, aufweisend

- eine erste chemische Einheit, die eine Struktur der Formel I

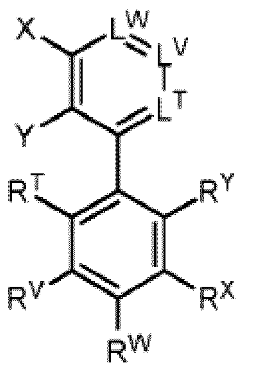

Formel I

aufweist, und

- zwei zweite chemische Einheit, die unabhängig voneinander eine Struktur der Formel II

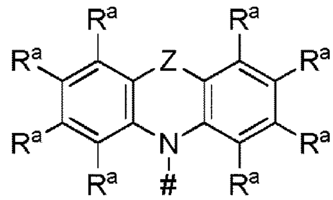

Formel II

aufweisen,

wobei die erste chemische Einheit jeweils über eine Einfachbindung mit einer der zwei zweiten chemischen Einheit verknüpft ist;

wobei

$L^T$ gleich N oder C-$R^1$ ist;

$L^V$ gleich N oder C-$R^1$ ist;

$L^W$ gleich N oder C-W ist;

X für die Bindungsstelle einer Einfachbindung steht, die die erste chemische Einheit mit einer der zwei zweiten chemischen Einheit verknüpft oder $R^2$ ist;

Y für die Bindungsstelle einer Einfachbindung steht, die die erste chemische Einheit mit einer der zwei zweiten chemischen Einheit verknüpft oder $R^2$ ist; W für die Bindungsstelle einer Einfachbindung steht, die die erste chemische Einheit mit einer der zwei zweiten chemischen Einheit verknüpft oder $R^2$ ist;

$R^T$ ausgewählt ist aus der Gruppe bestehend aus $CF_3$ und $R^I$ ;

$R^V$ ausgewählt ist aus der Gruppe bestehend aus $CF_3$ und $R^I$ ;

$R^W$ für die Bindungsstelle einer Einfachbindung steht, die die erste chemische Einheit mit einer der zwei zweiten chemischen Einheit verknüpft oder ausgewählt ist aus der Gruppe bestehend aus $CF_3$ und $R^I$ ;

$R^X$ für die Bindungsstelle einer Einfachbindung steht, die die erste chemische Einheit mit einer der zwei zweiten chemischen Einheit verknüpft oder $R^I$ ist; $R^Y$ für die Bindungsstelle einer Einfachbindung steht, die die erste chemische Einheit mit einer der zwei zweiten chemischen Einheit verknüpft oder $R^I$ ist;

# für die Bindungsstelle einer Einfachbindung steht, die die erste chemische Einheit mit der zweiten chemischen Einheit verknüpft;

Z bei jedem Auftreten unabhängig voneinander aus der Gruppe bestehend aus einer direkten Bindung, $CR^3R^4$, $C=CR^3R^4$, C=O, $C=NR^3$, $NR^3$, O, $SiR^3R^4$, S, S(O) und $S(O)_2$ ausgewählt ist;

$R^1$ bei jedem Auftreten unabhängig voneinander aus der Gruppe bestehend aus

Wasserstoff, Deuterium,

$C_1$-$C_5$-Alkyl,

wobei ein oder mehrere Wasserstoffatome optional durch Deuterium substituiert sind;

$C_2$-$C_8$-Alkenyl,

wobei ein oder mehrere Wasserstoffatome optional durch Deuterium substituiert sind;

$C_2$-$C_8$-Alkinyl,
wobei ein oder mehrere Wasserstoffatome optional durch Deuterium substituiert sind; und
$C_6$-$C_{18}$-Aryl,
das optional durch einen oder mehrere Substituenten $R^6$ substituiert ist;
$R^2$ bei jedem Auftreten unabhängig voneinander aus der Gruppe bestehend aus
Wasserstoff, Deuterium,
$C_1$-$C_5$-Alkyl,
wobei ein oder mehrere Wasserstoffatome optional durch Deuterium substituiert sind;
$C_2$-$C_8$-Alkenyl,
wobei ein oder mehrere Wasserstoffatome optional durch Deuterium substituiert sind;
$C_2$-$C_8$-Alkinyl,
wobei ein oder mehrere Wasserstoffatome optional durch Deuterium substituiert sind; und
$C_6$-$C_{18}$-Aryl,
das optional durch einen oder mehrere Substituenten $R^6$ substituiert ist;
$R^I$ bei jedem Auftreten unabhängig voneinander aus der Gruppe bestehend aus
Wasserstoff, Deuterium,
$C_1$-$C_5$-Alkyl,
wobei ein oder mehrere Wasserstoffatome optional durch Deuterium substituiert sind;
$C_2$-$C_8$-Alkenyl,
wobei ein oder mehrere Wasserstoffatome optional durch Deuterium substituiert sind;
$C_2$-$C_8$-Alkinyl,
wobei ein oder mehrere Wasserstoffatome optional durch Deuterium substituiert sind; und
$C_6$-$C_{18}$-Aryl,
das optional durch einen oder mehrere Substituenten $R^6$ substituiert ist;
$R^a$, $R^3$ und $R^4$ bei jedem Auftreten unabhängig voneinander aus der Gruppe bestehend aus Wasserstoff, Deuterium, $N(R^5)_2$, $OR^5$, $Si(R^5)_3$, $B(OR^5)_2$, $OSO_2R^5$, $CF_3$, CN, F, Br, I,
$C_1$-$C_{40}$-Alkyl,

das optional durch einen oder mehrere Substituenten $R^5$ substituiert ist und
wobei eine oder mehrere nicht benachbarte $CH_2$-Gruppen optional durch $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;

$C_1$-$C_{40}$-Alkoxy,

das optional durch einen oder mehrere Substituenten $R^5$ substituiert ist und
wobei eine oder mehrere nicht benachbarte $CH_2$-Gruppen optional durch $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;

$C_1$-$C_{40}$-Thioalkoxy,

das optional durch einen oder mehrere Substituenten $R^5$ substituiert ist und
wobei eine oder mehrere nicht benachbarte $CH_2$-Gruppen optional durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;

$C_2$-$C_{40}$-Alkenyl,

das optional durch einen oder mehrere Substituenten $R^5$ substituiert ist und
wobei eine oder mehrere nicht benachbarte $CH_2$-Gruppen optional durch $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;

$C_2$-$C_{40}$-Alkinyl,

das optional durch einen oder mehrere Substituenten $R^5$ substituiert ist und
wobei eine oder mehrere nicht benachbarte $CH_2$-Gruppen optional durch $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;

$C_6$-$C_{60}$-Aryl,
das optional durch einen oder mehrere Substituenten $R^5$ substituiert ist; und

$C_3$-$C_{57}$-Heteroaryl,
das optional durch einen oder mehrere Substituenten $R^5$ substituiert ist;
ausgewählt sind;
$R^5$ bei jedem Auftreten unabhängig voneinander aus der Gruppe bestehend aus Wasserstoff, Deuterium, $N(R^6)_2$, $OR^6$, $Si(R^6)_3$, $B(OR^6)_2$, $OSO_2R^6$, $CF_3$, CN, F, Br, I,
$C_1$-$C_{40}$-Alkyl,

das optional durch einen oder mehrere Substituenten $R^6$ substituiert ist und wobei eine oder mehrere nicht benachbarte $CH_2$-Gruppen optional durch $R^6C=CR^6$, $C{\equiv}C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sind;

$C_1$-$C_{40}$-Alkoxy,

das optional durch einen oder mehrere Substituenten $R^6$ substituiert ist und wobei eine oder mehrere nicht benachbarte $CH_2$-Gruppen optional durch $R^6C=CR^6$, $C{\equiv}C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sind;

$C_1$-$C_{40}$-Thioalkoxy,

das optional durch einen oder mehrere Substituenten $R^6$ substituiert ist und wobei eine oder mehrere nicht benachbarte $CH_2$-Gruppen optional durch $R^6C=CR^6$, $C{\equiv}C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sind;

$C_2$-$C_{40}$-Alkenyl,

das optional durch einen oder mehrere Substituenten $R^6$ substituiert ist und wobei eine oder mehrere nicht benachbarte $CH_2$-Gruppen optional durch $R^6C=CR^6$, $C{\equiv}C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sind;

$C_2$-$C_{40}$-Alkinyl,

das optional durch einen oder mehrere Substituenten $R^6$ substituiert ist und wobei eine oder mehrere nicht benachbarte $CH_2$-Gruppen optional durch $R^6C=CR^6$, $C{\equiv}C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sind;

$C_6$-$C_{60}$-Aryl,
das optional durch einen oder mehrere Substituenten $R^6$ substituiert ist; und
$C_3$-$C_{57}$-Heteroaryl,
das optional durch einen oder mehrere Substituenten $R^6$ substituiert ist;
ausgewählt ist;
$R^6$ bei jedem Auftreten unabhängig voneinander aus der Gruppe bestehend aus Wasserstoff, Deuterium, OPh, $CF_3$, CN, F,
$C_1$-$C_5$-Alkyl,
wobei ein oder mehrere Wasserstoffatome optional unabhängig voneinander durch Deuterium, CN, $CF_3$ oder F ersetzt sind;
$C_1$-$C_5$-Alkoxy,
wobei ein oder mehrere Wasserstoffatome optional unabhängig voneinander durch Deuterium, CN, $CF_3$ oder F ersetzt sind;
$C_1$-$C_5$-Thioalkoxy,
wobei ein oder mehrere Wasserstoffatome optional unabhängig voneinander durch Deuterium, CN, $CF_3$ oder F ersetzt sind;
$C_2$-$C_5$-Alkenyl,
wobei ein oder mehrere Wasserstoffatome optional unabhängig voneinander durch Deuterium, CN, $CF_3$ oder F ersetzt sind;
$C_2$-$C_5$-Alkinyl,
wobei ein oder mehrere Wasserstoffatome optional unabhängig voneinander durch Deuterium, CN, $CF_3$ oder F ersetzt sind;
$C_6$-$C_{18}$-Aryl,

das optional durch einen oder mehrere $C_1$-$C_5$-Alkylsubstituenten substituiert ist;
$C_3$-$C_{17}$-Heteroaryl,
das optional durch einen oder mehrere $C_1$-$C_5$-Alkylsubstituenten substituiert ist;
$N(C_6$-$C_{18}$-Aryl$)_2$;
$N(C_3$-$C_{17}$-Heteroaryl$)_2$
und $N(C_3$-$C_{17}$-Heteroaryl$)(C_6$-$C_{18}$-Aryl$)$
ausgewählt ist;
wobei die Substituenten $R^a$, $R^3$, $R^4$ oder $R^5$ unabhängig voneinander optional mit einem oder mehreren Substituenten $R^a$, $R^3$, $R^4$ oder $R^5$ ein mono- oder polycyclisches, aliphatisches, aromatisches und/oder benzoanneliertes Ringsystem bilden;
wobei die Begriffe Alkyl, Alkeny, Alkinyl, Alkoxy und Thioalkoxy lineare, verzweigte oder zyklische Substituenten umfassen;
wobei
genau ein Ringatom ausgewählt aus der Gruppe bestehend aus $L^T$, $L^V$ und $L^W$ gleich N ist;
genau ein Substituent ausgewählt aus der Gruppe bestehend aus $R^T$, $R^V$ und $R^W$ gleich $CF_3$ ist;
genau ein Substituent aus der Gruppe bestehend aus W, Y und X für die Bindungsstelle einer Einfachbindung steht, die die erste chemische Einheit mit einer der zwei zweiten chemischen Einheit verknüpft; und
genau ein Substituent aus der Gruppe bestehend aus $R^W$, $R^Y$ und $R^X$ für die Bindungsstelle einer Einfachbindung steht, die die erste chemische Einheit mit einer der zwei zweiten chemischen Einheit verknüpft.

**2.** Organisches Molekül nach Anspruch 1, wobei die erste chemische Einheit eine Struktur der Formel Ia aufweist:

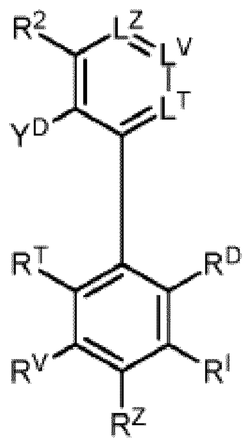

Formel Ia

wobei

$Y^D$ für die Bindungsstelle einer Einfachbindung steht, die die erste chemische Einheit mit einer der zwei zweiten chemischen Einheit verknüpft;
$R^D$ für die Bindungsstelle einer Einfachbindung steht, die die erste chemische Einheit mit einer der zwei zweiten chemischen Einheit verknüpft;
$L^Z$ gleich N oder C-$R^2$ ist;
$R^Z$ gleich $CF_3$ oder $R^I$ ist:
wobei genau ein Substituent ausgewählt aus der Gruppe bestehend aus $R^T$, $R^V$ und $R^Z$ gleich $CF_3$ und genau ein Substituent ausgewählt aus der Gruppe bestehend aus $L^T$, $L^V$ und $L^Z$ gleich N ist; und wobei darüber hinaus die Definitionen des Anspruchs 1 gelten.

**3.** Organisches Molekül nach Anspruch 1 oder 2, wobei $R^1$, $R^2$ und $R^I$ bei jedem Auftreten unabhängig voneinander ausgewählt sind aus der Gruppe bestehend aus H, Methyl, Mesityl, Tolyl und Phenyl.

**4.** Organisches Molekül nach einem oder mehreren der Ansprüche 1 bis 3, wobei $R^W$ oder $R^Z$ gleich $CF_3$ ist.

**5.** Organisches Molekül nach einem oder mehreren der Ansprüche 1 bis 4, wobei die zwei zweiten chemischen Einheiten unabhängig voneinander eine Struktur der Formel IIa aufweisen:

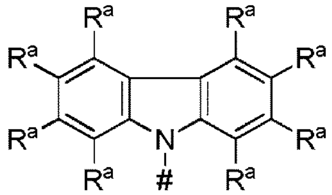

Formel IIa

wobei # und $R^a$ wie in Anspruch 1 definiert sind.

**6.** Organisches Molekül nach einem oder mehreren der Ansprüche 1 bis 5, wobei die zwei zweiten chemischen Einheiten unabhängig voneinander eine Struktur der Formel IIb aufweisen:

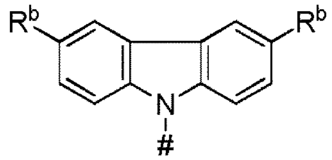

Formel IIb

wobei

$R^b$ bei jedem Auftreten unabhängig voneinander aus der Gruppe bestehend aus Deuterium, $N(R^5)_2$, $OR^5$, $Si(R^5)_3$, $Ge(R^5)_3$, $B(OR^5)_2$, $OSO_2R^5$, $CF_3$, CN, F, Br, I,
$C_1$-$C_{40}$-Alkyl,

das optional durch einen oder mehrere Substituenten $R^5$ substituiert ist und wobei eine oder mehrere nicht benachbarte $CH_2$-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;

$C_1$-$C_{40}$-Alkoxy,

das optional durch einen oder mehrere Substituenten $R^5$ substituiert ist und wobei eine oder mehrere nicht benachbarte $CH_2$-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;

$C_1$-$C_{40}$-Thioalkoxy,

das optional durch einen oder mehrere Substituenten $R^5$ substituiert ist und wobei eine oder mehrere nicht benachbarte $CH_2$-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;

$C_2$-$C_{40}$-Alkenyl,

das optional durch einen oder mehrere Substituenten $R^5$ substituiert ist und wobei eine oder mehrere nicht benachbarte $CH_2$-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;

$C_2$-$C_{40}$-Alkinyl,

das optional durch einen oder mehrere Substituenten $R^5$ substituiert ist und wobei eine oder mehrere nicht benachbarte $CH_2$-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;

$C_6$-$C_{60}$-Aryl,
das optional durch einen oder mehrere Substituenten $R^5$ substituiert ist; und

$C_3$-$C_{57}$-Heteroaryl,
das optional durch einen oder mehrere Substituenten $R^5$ substituiert ist;
ausgewählt ist;
und wobei davon abgesehen die Definitionen in Anspruch 1 gelten.

**7.** Organisches Molekül nach einem oder mehreren der Ansprüche 1 bis 5, wobei die zwei zweiten chemischen Einheiten eine Struktur der Formel IIc aufweisen:

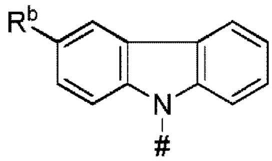

Formel IIc

wobei

$R^b$ bei jedem Auftreten unabhängig voneinander aus der Gruppe bestehend aus Deuterium, $N(R^5)_2$, $OR^5$, $Si(R^5)_3$, $Ge(R^5)_3$, $B(OR^5)_2$, $OSO_2R^5$, $CF_3$, CN, F, Br, I,
$C_1$-$C_{40}$-Alkyl,

das optional durch einen oder mehrere Substituenten $R^5$ substituiert ist und wobei eine oder mehrere nicht benachbarte $CH_2$-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;

$C_1$-$C_{40}$-Alkoxy,

das optional durch einen oder mehrere Substituenten $R^5$ substituiert ist und wobei eine oder mehrere nicht benachbarte $CH_2$-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;

$C_1$-$C_{40}$-Thioalkoxy,

das optional durch einen oder mehrere Substituenten $R^5$ substituiert ist und wobei eine oder mehrere nicht benachbarte $CH_2$-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;

$C_2$-$C_{40}$-Alkenyl,

das optional durch einen oder mehrere Substituenten $R^5$ substituiert ist und wobei eine oder mehrere nicht benachbarte $CH_2$-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;

$C_2$-$C_{40}$-Alkinyl,

das optional durch einen oder mehrere Substituenten $R^5$ substituiert ist und wobei eine oder mehrere nicht benachbarte $CH_2$-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;

$C_6$-$C_{60}$-Aryl,
das optional durch einen oder mehrere Substituenten $R^5$ substituiert ist; und
$C_3$-$C_{57}$-Heteroaryl,
das optional durch einen oder mehrere Substituenten $R^5$ substituiert ist;
ausgewählt ist;
und wobei davon abgesehen die Definitionen in Anspruch 1 gelten.

**8.** Organisches Molekül nach Anspruch 6 oder 7, wobei $R^b$ bei jedem Auftreten unabhängig voneinander aus der

Gruppe bestehend aus

- Me, $^{i}Pr$, $^{t}Bu$, CN, $CF_3$,
- Ph, das optional durch einen oder mehrere Substituenten, die unabhängig voneinander aus der Gruppe bestehend aus Me, $^{i}Pr$, $^{t}Bu$, CN, $CF_3$ und Ph ausgewählt sind, substituiert ist;
- Pyridinyl, das optional durch einen oder mehrere Substituenten, die unabhängig voneinander aus der Gruppe bestehend aus Me, $^{i}Pr$, $^{t}Bu$, CN, $CF_3$ und Ph ausgewählt sind, substituiert ist;
- Pyrimidinyl, das optional durch einen oder mehrere Substituenten, die unabhängig voneinander aus der Gruppe bestehend aus Me, $^{i}Pr$, $^{t}Bu$, CN, $CF_3$ und Ph ausgewählt sind, substituiert ist;
- Carbazolyl, das optional durch einen oder mehrere Substituenten, die unabhängig voneinander aus der Gruppe bestehend aus Me, $^{i}Pr$, $^{t}Bu$, CN, $CF_3$ und Ph ausgewählt sind, substituiert ist;
- Triazinyl, das optional durch einen oder mehrere Substituenten, die unabhängig voneinander aus der Gruppe bestehend aus Me, $^{i}Pr$, $^{t}Bu$, CN, $CF_3$ und Ph ausgewählt sind, substituiert ist;
und
- $N(Ph)_2$

ausgewählt ist.

**9.** Verfahren zur Herstellung eines organischen Moleküls nach einem der Ansprüche 1 bis 8, aufweisend die Bereitstellung eines Halogenfluorobenzotrifluorides als Reaktand, welches mit drei $R^I$ substituiert ist.

**10.** Verwendung eines organischen Moleküls nach einem oder mehreren der Ansprüche 1 bis 8 als lumineszierender Emitter und/oder ein Wirtsmaterial und/oder ein Elektronentransportmaterial und/oder ein Lochinjektionsmaterial und/oder ein Lochblockiermaterial in einer optoelektronischen Vorrichtung.

**11.** Verwendung nach Anspruch 10, wobei die optoelektronische Vorrichtung aus der Gruppe bestehend aus:

- organischen lichtemittierenden Dioden (OLEDs),
- lichtemittierenden elektrochemischen Zellen,
- OLED-Sensoren, insbesondere in nicht-hermetisch abgeschirmten Gas-oder Dampfsensoren,
- organischen Dioden,
- organischen Solarzellen,
- organischen Transistoren,
- organischen Feldeffekttransistoren,
- organischen Lasern und
- Down-Konversions-Elementen

ausgewählt ist.

**12.** Zusammensetzung, aufweisend

(a) mindestens ein organisches Molekül nach einem oder mehreren der Ansprüche 1 bis 8, insbesondere in Form eines Emitters und/oder eines Wirts, und
(b) einen Emitter- und/oder ein Wirtsmaterialien, das von dem organischen Molekül verschieden ist, und
(c) optional einen Farbstoff und/oder ein Lösungsmittel.

**13.** Optoelektronische Vorrichtung, aufweisend ein organisches Molekül nach einem oder mehreren der Ansprüche 1 bis 8 oder eine Zusammensetzung nach Anspruch 12, insbesondere in Form einer Vorrichtung ausgewählt aus der Gruppe bestehend aus einer organischen lichtemittierenden Diode (OLED), einer lichtemittierenden elektrochemischen Zelle, einem OLED-Sensor, insbesondere in nicht-hermetisch abgeschirmten Gas- oder Dampfsensoren, einer organischen Diode, einer organischen Solarzelle, einem organischen Transistor, einem organischen Feldeffekttransistor, einem organischen Laser und einem Down-Konversions-Element.

**14.** Optoelektronische Vorrichtung nach Anspruch 13, aufweisend

- ein Substrat,
- eine Anode und
- eine Kathode, wobei die Anode oder die Kathode auf das Substrat aufgebracht ist, und

- mindestens eine lichtemittierende Schicht, die zwischen der Anode und der Kathode angeordnet ist und die das organische Molekül oder die Zusammensetzung aufweist.

**15.** Verfahren zur Herstellung einer optoelektronischen Vorrichtung, bei dem ein organisches Molekül nach einem der Ansprüche 1 bis 8 oder eine Zusammensetzung nach Anspruch 12 verwendet wird, insbesondere aufweisend das Verarbeiten des organischen Moleküls mittels eines Vakuumverdampfungsverfahrens oder aus einer Lösung.

**Revendications**

**1.** Molécule organique, comprenant

- un premier fragment chimique comprenant une structure de formule I,

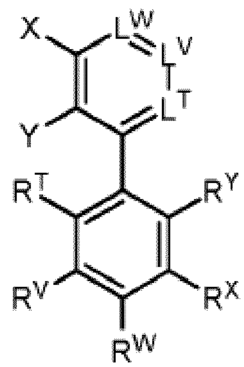

Formule I

et
- deux deuxièmes fragments chimiques, chacun indépendamment l'un de l'autre comprenant une structure de formule II

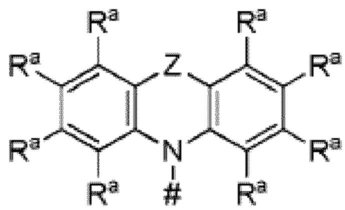

Formule II

le premier fragment chimique étant lié à chacun des deux deuxièmes fragments chimiques via une simple liaison ;

$L^T$ étant N ou C-$R^1$ ;
$L^V$ étant N ou C-$R^1$ ;
$L^W$ étant N ou C-W ;
X étant le site de liaison d'une simple liaison liant le premier fragment chimique à l'un des deux deuxièmes fragments chimiques ou étant $R^2$ ;
Y étant le site de liaison d'une simple liaison liant le premier fragment chimique à l'un des deux deuxièmes fragments chimiques ou étant $R^2$ ;
W étant le site de liaison d'une simple liaison liant le premier fragment chimique à l'un des deux deuxièmes fragments chimiques ou étant $R^2$ ;
$R^T$ étant choisi dans le groupe constitué par $CF_3$ et $R^I$ ;
$R^V$ étant choisi dans le groupe constitué par $CF_3$ et $R^I$ ;
$R^W$ étant le site de liaison d'une simple liaison liant le premier fragment chimique à l'un des deux deuxièmes fragments chimiques ou étant choisi dans le groupe constitué par $CF_3$ et $R^I$ ;

$R^X$ étant le site de liaison d'une simple liaison liant le premier fragment chimique à l'un des deux deuxièmes fragments chimiques ou étant $R^I$ ;
$R^Y$ étant le site de liaison d'une simple liaison liant le premier fragment chimique à l'un des deux deuxièmes fragments chimiques ou étant $R^I$ ;
# représentant le site de liaison d'une simple liaison liant les deuxièmes fragments chimiques au premier fragment chimique ;
Z étant, en chaque occurrence indépendamment l'un de l'autre, choisi dans le groupe constitué par une liaison directe, $CR^3R^4$, $C=CR^3R^4$, C=O, $C=NR^3$, $NR^3$, O, $SiR^3R^4$, S, S(O) et $S(O)_2$ ;
$R^1$ étant, en chaque occurrence indépendamment l'un de l'autre, choisi dans le groupe constitué par :

hydrogène, deutérium,
$C_{1-5}$-alkyle,
un ou plusieurs atomes d'hydrogène étant éventuellement substitués par deutérium ;
$C_{2-8}$-alcényle,
un ou plusieurs atomes d'hydrogène étant éventuellement substitués par deutérium ;
$C_{2-8}$-alcényle,
un ou plusieurs atomes d'hydrogène étant éventuellement substitués par deutérium ; et
$C_{6-18}$-aryle,
qui est éventuellement substitué par un ou plusieurs substituants $R^6$ ;
$R^2$ étant, en chaque occurrence indépendamment l'un de l'autre, choisi dans le groupe constitué par :

hydrogène, deutérium,
$C_{1-5}$-alkyle,
un ou plusieurs atomes d'hydrogène étant éventuellement substitués par deutérium ;
$C_{2-8}$-alcényle,
un ou plusieurs atomes d'hydrogène étant éventuellement substitués par deutérium ;
$C_{2-8}$-alcényle,
un ou plusieurs atomes d'hydrogène étant éventuellement substitués par deutérium ; et
$C_{6-18}$-aryle,
qui est éventuellement substitué par un ou plusieurs substituants $R^6$ ;
$R^I$ étant, en chaque occurrence indépendamment l'un de l'autre, choisi dans le groupe constitué par :

hydrogène, deutérium,
$C_{1-5}$-alkyle,
un ou plusieurs atomes d'hydrogène étant éventuellement substitués par deutérium ;
$C_{2-8}$-alcényle,
un ou plusieurs atomes d'hydrogène étant éventuellement substitués par deutérium ;
$C_{2-8}$-alcényle,
un ou plusieurs atomes d'hydrogène étant éventuellement substitués par deutérium ; et
$C_{6-18}$-aryle,
qui est éventuellement substitué par un ou plusieurs substituants $R^6$ ;
$R^a$, $R^3$ et $R^4$ étant en chaque occurrence choisis indépendamment les uns des autres dans le groupe constitué par hydrogène, deutérium, $N(R^5)_2$, $OR^5$, $Si(R^5)_3$, $B(OR^5)_2$, $OSO_2R^5$, $CF_3$, CN, F, Br, I,
$C_{1-40}$-alkyle,

qui est éventuellement substitué par un ou plusieurs substituants $R^5$ et
un ou plusieurs groupes $CH_2$ non adjacents étant éventuellement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$;

$C_{1-40}$-alcoxy,

qui est éventuellement substitué par un ou plusieurs substituants $R^5$ et
un ou plusieurs groupes $CH_2$ non adjacents étant éventuellement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$;

$C_{1-40}$-thioalcoxy,

qui est éventuellement substitué par un ou plusieurs substituants $R^5$ et un ou plusieurs groupes $CH_2$ non adjacents étant éventuellement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$;

$C_{2-40}$-alcényle,

qui est éventuellement substitué par un ou plusieurs substituants $R^5$ et un ou plusieurs groupes $CH_2$ non adjacents étant éventuellement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$;

$C_{2-40}$-alcynyle,

qui est éventuellement substitué par un ou plusieurs substituants $R^5$ et un ou plusieurs groupes $CH_2$ non adjacents étant éventuellement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$;

$C_{6-60}$-aryle,
qui est éventuellement substitué par un ou plusieurs substituants $R^5$ ; et
$C_{3-57}$-hétéroaryle,
qui est éventuellement substitué par un ou plusieurs substituants $R^5$ ;
$R^5$ étant en chaque occurrence choisi indépendamment l'un de l'autre dans le groupe constitué par hydrogène, deutérium, $N(R^6)_2$, $OR^6$, $Si(R^6)_3$, $B(OR^6)_2$, $OSO_2R^6$, $CF_3$, CN, F, Br, I,
$C_{1-40}$-alkyle,

qui est éventuellement substitué par un ou plusieurs substituants $R^6$ et un ou plusieurs groupes $CH_2$ non adjacents étant éventuellement substitués par $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S, ou $CONR^6$;

$C_{1-40}$-alcoxy,

qui est éventuellement substitué par un ou plusieurs substituants $R^6$ et un ou plusieurs groupes $CH_2$ non adjacents étant éventuellement substitués par $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S, ou $CONR^6$;

$C_{1-40}$-thioalcoxy,

qui est éventuellement substitué par un ou plusieurs substituants $R^6$ et un ou plusieurs groupes $CH_2$ non adjacents étant éventuellement substitués par $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S, ou $CONR^6$;

$C_{2-40}$-alcényle,

qui est éventuellement substitué par un ou plusieurs substituants $R^6$ et un ou plusieurs groupes $CH_2$ non adjacents étant éventuellement substitués par $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S, ou $CONR^6$;

$C_{2-40}$-alcynyle,

qui est éventuellement substitué par un ou plusieurs substituants $R^6$ et

un ou plusieurs groupes $CH_2$ non adjacents étant éventuellement substitués par $R^6C=CR^6$, $C\equiv C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S, ou $CONR^6$;

$C_{6-60}$-aryle,
qui est éventuellement substitué par un ou plusieurs substituants $R^6$ ; et
$C_{3-57}$-hétéroaryle,
qui est éventuellement substitué par un ou plusieurs substituants $R^6$ ;
$R^6$ étant en chaque occurrence choisi indépendamment l'un de l'autre dans le groupe constitué par hydrogène, deutérium, OPh, $CF_3$, CN, F,
$C_{1-5}$-alkyle,
un ou plusieurs atomes d'hydrogène étant éventuellement, indépendamment les uns des autres, substitués par deutérium, CN, $CF_3$, ou F ;
$C_{1-5}$-alcoxy,
un ou plusieurs atomes d'hydrogène étant éventuellement, indépendamment les uns des autres, substitués par deutérium, CN, $CF_3$, ou F ;
$C_{1-5}$-thioalcoxy,
un ou plusieurs atomes d'hydrogène étant éventuellement, indépendamment les uns des autres, substitués par deutérium, CN, $CF_3$, ou F ;
$C_{2-5}$-alcényle,
un ou plusieurs atomes d'hydrogène étant éventuellement, indépendamment les uns des autres, substitués par deutérium, CN, $CF_3$, ou F ;
$C_{2-5}$-alcynyle,
un ou plusieurs atomes d'hydrogène étant éventuellement, indépendamment les uns des autres, substitués par deutérium, CN, $CF_3$, ou F ;
$C_{6-18}$-aryle,
qui est éventuellement substitué par un ou plusieurs substituants $C_{1-5}$-alkyle ;
$C_{3-17}$-hétéroaryle,
qui est éventuellement substitué par un ou plusieurs substituants $C_{1-5}$-alkyle ;
$N(C_{6-18}$-aryle$)_2$ ;
$N(C_{3-17}$-hétéroaryle$)_2$,
et $N(C_{3-17}$-hétéroaryle$)(C_{6-18}$-aryle) ;
les substituants $R^a$, $R^3$, $R^4$ ou $R^5$ formant éventuellement indépendamment les uns des autres un système cyclique monocyclique ou polycyclique, aliphatique, aromatique et/ou benzo-condensé avec un ou plusieurs substituants $R^a$, $R^3$, $R^4$ ou $R^5$;
les termes alkyle, alcényle, alcynyle, alcoxy et thioalcoxy pouvant faire référence à des substituants linéaires, ramifiés, et cycliques ;
exactement un atome de cycle choisi dans le groupe constitué par $L^T$, $L^V$, et $L^W$ étant N ;
exactement un substituant choisi dans le groupe constitué par $R^T$, $R^V$ et $R^W$ étant $CF_3$,
exactement un substituant choisi dans le groupe constitué par W, Y et X représentant le site de liaison d'une simple liaison liant le premier fragment chimique et l'un des deux deuxièmes fragments chimiques et
exactement un substituant choisi dans le groupe constitué par $R^W$, $R^Y$ et $R^X$ représentant le site de liaison d'une simple liaison liant le premier fragment chimique est l'un des deux deuxièmes fragments chimiques.

**2.** Molécule organique selon la revendication 1, le premier fragment chimique comprenant une structure de formule Ia :

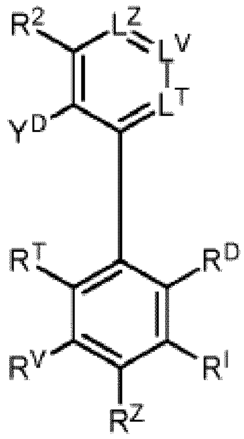

Formule Ia

$Y^D$ étant le site de liaison d'une simple liaison liant le premier fragment chimique à l'un des deux deuxièmes fragments chimiques ;
$R^D$ étant le site de liaison d'une simple liaison liant le premier fragment chimique à l'un des deux deuxièmes fragments chimiques ;
$L^Z$ étant N ou C-$R^2$;
$R^Z$ étant $CF_3$ ou $R^I$ ;
exactement un substituant choisi dans le groupe constitué par $R^T$, $R^V$ et $R^Z$ étant $CF_3$ et exactement un atome de cycle choisi dans le groupe constitué par $L^T$, $L^V$ et $L^Z$ étant N ; et mis à part cela, les définitions de la revendication 1 s'appliquant.

3. Molécule organique selon la revendication 1 ou 2, $R^1$, $R^2$ et $R^I$ étant indépendamment les uns des autres en chaque occurrence indépendamment les uns des autres choisis dans le groupe constitué par H, méthyle, mésityle, tolyle et phényle.

4. Molécule organique selon l'une ou plusieurs des revendications 1 à 3, $R^W$ ou $R^Z$ étant $CF_3$.

5. Molécule organique selon l'une ou plusieurs des revendications 1 à 4, les deux deuxièmes fragments chimiques, chacun en chaque occurrence indépendamment l'un de l'autre, comprenant une structure de formule IIa :

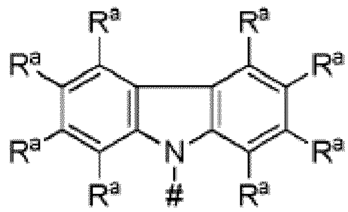

Formule IIa

# et $R^a$ étant définis comme dans la revendication 1.

6. Molécule organique selon l'une ou plusieurs des revendications 1 à 5, les deux deuxièmes fragments chimiques, chacun en chaque occurrence indépendamment l'un de l'autre, comprenant une structure de formule IIb :

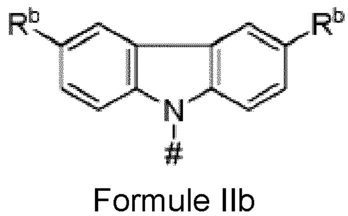

Formule IIb

$R^b$ étant en chaque occurrence indépendamment l'un de l'autre choisi dans le groupe constitué par deutérium, $N(R^5)_2$, $OR^5$, $Si(R^5)_3$, $B(OR^5)_2$, $OSO_2R^5$, $CF_3$, CN, F, Br, I,
$C_{1-40}$-alkyle,
qui est éventuellement substitué par un ou plusieurs substituants $R^5$ et un ou plusieurs groupes $CH_2$ non adjacents étant éventuellement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$;
$C_{1-40}$-alcoxy, qui est éventuellement substitué par un ou plusieurs substituants $R^5$ et un ou plusieurs groupes $CH_2$ non adjacents étant éventuellement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$;
$C_{1-40}$-thioalcoxy, qui est éventuellement substitué par un ou plusieurs substituants $R^5$ et un ou plusieurs groupes $CH_2$ non adjacents étant éventuellement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$;
$C_{2-40}$-alcényle, qui est éventuellement substitué par un ou plusieurs substituants $R^5$ et un ou plusieurs groupes $CH_2$ non adjacents étant éventuellement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$;
$C_{2-40}$-alcynyle, qui est éventuellement substitué par un ou plusieurs substituants $R^5$ et un ou plusieurs groupes $CH_2$ non adjacents étant éventuellement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$;
$C_{6-60}$-aryle, qui est éventuellement substitué par un ou plusieurs substituants $R^5$ ; et
$C_{3-57}$-hétéroaryle,
qui est éventuellement substitué par un ou plusieurs substituants $R^5$ ;
et mis à part cela, les définitions de la revendication 1 s'appliquant.

**7.** Molécule organique selon l'une ou plusieurs des revendications 1 à 5, les deux deuxièmes fragments chimiques, chacun en chaque occurrence indépendamment l'un de l'autre, comprenant une structure de formule IIc :

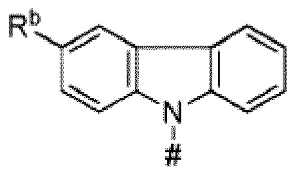

Formule IIc

$R^b$ étant en chaque occurrence indépendamment l'un de l'autre choisi dans le groupe constitué par deutérium, $N(R^5)_2$, $OR^5$, $Si(R^5)_3$, $B(OR^5)_2$, $OSO_2R^5$, $CF_3$, CN, F, Br, I,
$C_{1-40}$-alkyle,
qui est éventuellement substitué par un ou plusieurs substituants $R^5$ et un ou plusieurs groupes $CH_2$ non adjacents étant éventuellement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$;
$C_{1-40}$-alcoxy,
qui est éventuellement substitué par un ou plusieurs substituants $R^5$ et un ou plusieurs groupes $CH_2$ non adjacents étant éventuellement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$;
$C_{1-40}$-thioalcoxy,
qui est éventuellement substitué par un ou plusieurs substituants $R^5$ et un ou plusieurs groupes $CH_2$ non adjacents étant éventuellement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$;
$C_{2-40}$-alcényle,
qui est éventuellement substitué par un ou plusieurs substituants $R^5$ et un ou plusieurs groupes $CH_2$ non adjacents étant éventuellement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$;
$C_{2-40}$-alcynyle,
qui est éventuellement substitué par un ou plusieurs substituants $R^5$ et un ou plusieurs groupes $CH_2$ non adjacents étant éventuellement substitués par $R^5C=CR^5$, C≡C, $Si(R^5)_2$, $Ge(R^5)_2$, $Sn(R^5)_2$, C=O, C=S, C=Se,

$C=NR^5$, $P(=O)(R^5)$, SO, $SO_2$, $NR^5$, O, S, ou $CONR^5$;
$C_{6-60}$-aryle,
qui est éventuellement substitué par un ou plusieurs substituants $R^5$ ; et
$C_{3-57}$-hétéroaryle,
qui est éventuellement substitué par un ou plusieurs substituants $R^5$ ;
et mis à part cela, les définitions de la revendication 1 s'appliquant.

**8.** Molécule organique selon la revendication 6 ou 7, $R^b$ étant en chaque occurrence indépendamment l'un de l'autre choisi dans le groupe constitué par

- Me, $^i$Pr, $^t$Bu, CN, $CF_3$,
- Ph, qui est éventuellement substitué par un ou plusieurs substituants choisis indépendamment les uns des autres dans le groupe constitué par Me, $^i$Pr, $^t$Bu, CN, $CF_3$ et Ph ;
- pyridinyle, qui est éventuellement substitué par un ou plusieurs substituants choisis indépendamment les uns des autres dans le groupe constitué par Me, $^i$Pr, $^t$Bu, CN, $CF_3$ et Ph ;
- pyrimidinyle, qui est éventuellement substitué par un ou plusieurs substituants choisis indépendamment les uns des autres dans le groupe constitué par Me, $^i$Pr, $^t$Bu, CN, $CF_3$ et Ph ;
- carbazolyle, qui est éventuellement substitué par un ou plusieurs substituants choisis indépendamment les uns des autres dans le groupe constitué par Me, $^i$Pr, $^t$Bu, CN, $CF_3$ et Ph ;
- triazinyle, qui est éventuellement substitué par un ou plusieurs substituants choisis indépendamment les uns des autres dans le groupe constitué par Me, $^i$Pr, $^t$Bu, CN, $CF_3$ et Ph ;

et
- $N(Ph)_2$.

**9.** Procédé pour la préparation d'une molécule organique selon l'une quelconque des revendications 1 à 8, comprenant la mise à disposition d'un halogéno-fluorobenzotrifluorure qui est substitué par trois $R^I$ en tant que réactant.

**10.** Utilisation d'une molécule organique selon l'une ou plusieurs des revendications 1 à 8 en tant qu'émetteur luminescent et/ou en tant que matériau hôte et/ou en tant que matériau de transport d'électrons et/ou en tant que matériau d'injection de trous et/ou en tant que matériau de blocage de trous dans un dispositif optoélectronique.

**11.** Utilisation selon la revendication 10, le dispositif optoélectronique étant choisi dans le groupe constitué par :

• des diodes organiques luminescentes (OLED),
• des cellules électrochimiques luminescentes,
• des capteurs OLED, en particulier dans des capteurs de gaz et de vapeur non hermétiquement protégés,
• des diodes organiques,
• des cellules solaires organiques,
• des transistors organiques,
• des transistors organiques à effet de champ,
• des lasers organiques, et
• des éléments de conversion par abaissement.

**12.** Composition, comprenant :

(a) une molécule organique selon l'une ou plusieurs des revendications 1 à 8, en particulier sous forme d'un émetteur et/ou d'un hôte, et
(b) un matériau émetteur et/ou hôte, qui est différent de la molécule organique, et
(c) éventuellement, un colorant et/ou un solvant.

**13.** Dispositif optoélectronique, comprenant une molécule organique selon l'une ou plusieurs des revendications 1 à 8 ou une composition selon la revendication 12, en particulier sous forme d'un dispositif choisi dans le groupe constitué par une diode organique luminescente (OLED), une cellule électrochimique luminescente, un capteur OLED, en particulier dans des capteurs de gaz et de vapeur non hermétiquement protégés, une diode organique, une cellule solaire organique, un transistor organique, un transistor organique à effet de champ, un laser organique, et un élément de conversion par abaissement.

**14.** Dispositif optoélectronique selon la revendication 13, comprenant

- un substrat,
- une anode et
- une cathode, l'anode ou la cathode étant disposée sur le substrat et
- au moins une couche luminescente, qui est agencée entre l'anode et la cathode et qui comprend la molécule organique ou la composition.

**15.** Procédé pour la production d'un dispositif optoélectronique, une molécule organique selon l'une quelconque des revendications 1 à 8 ou une composition selon la revendication 12 étant utilisée, en particulier comprenant le traitement de la molécule organique par un procédé d'évaporation sous vide ou à partir d'une solution.

Figure 1

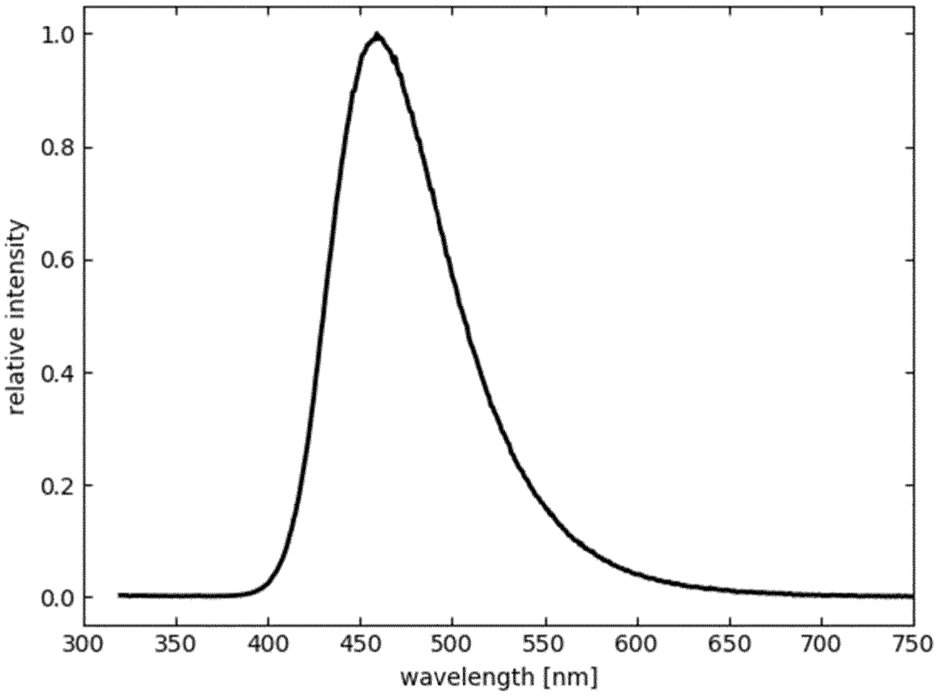


Figure 2

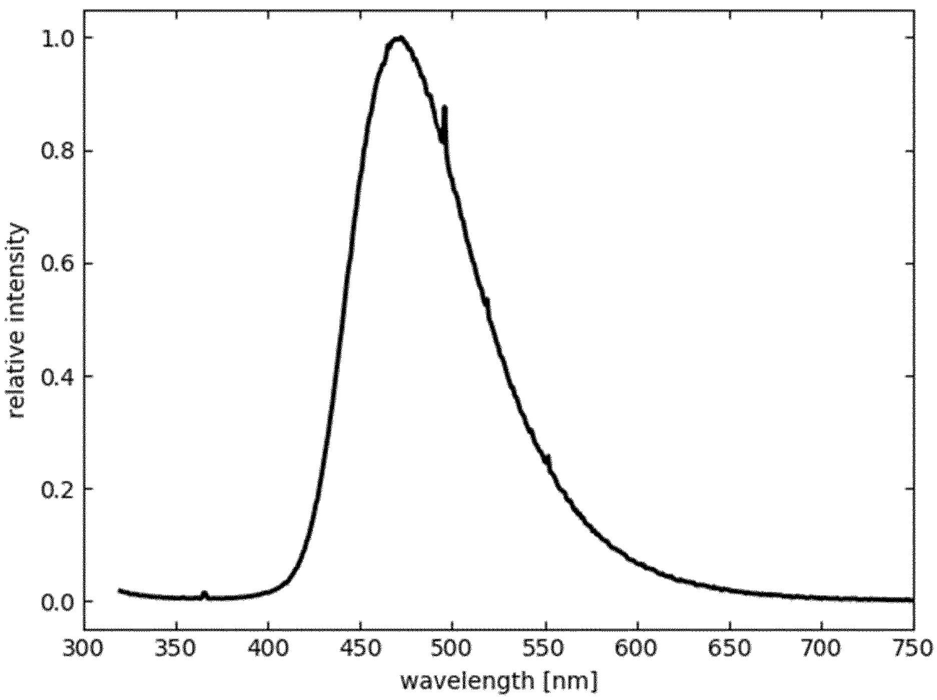

Figure 3

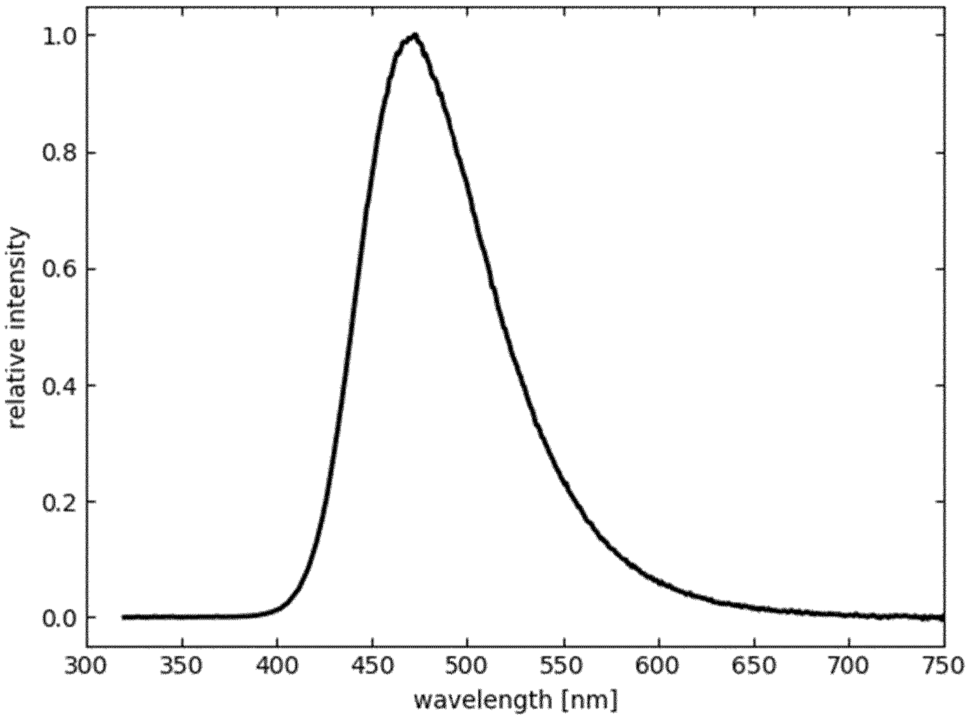
relative intensity
wavelength [nm]
0.0
0.2
0.4
0.6
0.8
1.0
300
350
400
450
500
550
600
650
700
750

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 3287451 A1
- WO 2017005699 A1


### Non-patent literature cited in the description


- *CHEMICAL ABSTRACTS,* 73183-34-3